# EUROPEAN PATENT SPECIFICATION

(11) **EP 1 423 524 B1**
(45) Date of publication and mention of the grant of the patent: **30.08.2006**
(21) Application number: 02767450.6
(22) Date of filing: 28.08.2002
(51) Int. Cl.: C12N 15/82, A01H 5/00

(54) **METHOD OF PROTEIN PRODUCTION IN PLANTS**
VERFAHREN ZUR PROTEINPRODUKTION IN PFLANZEN
PROCEDE DE PRODUCTION DE PROTEINE DANS DES PLANTES

(30) Priority: 04.09.2001 DE 10143205
(43) Date of publication of application: 02.06.2004
(73) Proprietor: Icon Genetics AG, 80539 München (DE)
(72) Inventor: DOROKHOV, Yurii, Moscow, 117321 (RU); SKURAT, Eugene, Moscow, 117421 (RU); KLIMYUK, Victor, 06114 Halle/Saale (DE); GLEBA, Yuri, 06114 Halle/Saale (DE)
(74) Representative: Hartz, Nikolai
(86) International application number: PCT/EP2002/009605
(87) International publication number: WO 2003/020938

(56) References cited:
- EP-A- 0 270 248
- WO-A-00/68391
- US-A- 5 474 925
- US-B1- 6 174 700
- DOMINGO CONCHA ET AL: "Identification of a novel peptide motif that mediates cross-linking of proteins to cell walls." PLANT JOURNAL., vol. 20, no. 5, December 1999 (1999-12), pages 563-570, XP002248474 ISSN: 0960-7412 cited in the application
- UEDA M ET AL: "Genetic immobilization of proteins on the yeast cell surface" BIOTECHNOLOGY ADVANCES, ELSEVIER PUBLISHING, BARKING, GB, vol. 18, no. 2, April 2000 (2000-04), pages 121-140, XP004205315 ISSN: 0734-9750
- SCHREUDER M P ET AL: "TARGETING OF A HETEROLOGOUS PROTEIN TO THE CELL WALL OF SACCHAROMYCES CEREVISIAE" YEAST, CHICHESTER, SUSSEX, GB, vol. 9, no. 4, April 1993 (1993-04), pages 399-409, XP002944788 ISSN: 0749-503X
- CARPIN SABINE ET AL: "Identification of a Ca2+-pectate binding site on an apoplastic peroxidase." PLANT CELL, vol. 13, no. 3, March 2001 (2001-03), pages 511-520, XP002248654 ISSN: 1040-4651 cited in the application
- LEHTIO JANNE ET AL: "Directed immobilization of recombinant staphylococci on cotton fibers by functional display of a fungal cellulose-binding domain." FEMS MICROBIOLOGY LETTERS, vol. 195, no. 2, 2001, pages 197-204, XP002248475 ISSN: 0378-1097

### SEQUENCE LISTING

<110> Icon Genetics AG
   Dorokhov, Yurii
   Skurat, Eugene
   Klimyuk, Victor
   Gleba, Yuri
<120> Method of Protein Production in Plants
<130> PCT-11971
<140> PCT/EP02/09605
   <141> 2002-08-28
<150> DE10143205.4
   <151> 2001-09-04
<160> 41
<170> PatentIn version 3.1
<210> 1
   <211> 2060
   <212> DNA
   <213> Nicotiana tabacum
<400> 1
   agaaatataa ggtgtcaaag atttcttcgc cggaatgttg gattccggca agaacgttaa      60
   tttttccaaa ggaaaaaaga aactattctt gaccgtcgtt gcctcagtcc tactcgttgc     120
   agcagttatc ggagtagttg tcggtgtaaa attccgttca aataactccg acgaccacgc     180
   agacatacag gccattacct ctgcagctca tgccattgta aaatctgcgt gtgaaaacac     240
   cctgcacccc gaattatgtt actctacaat tgcaagtgtt tcagatttct ccaaaaaagt     300
   aactagccaa aaagacgtga ttgagttgtc cttgaatatc acctgcaggg ccgtccaaca     360
   caactttttt aaggtcgaaa aactcatcaa aacaagaaaa ggtctaaagc cacgagaaaa     420
   ggttgcgttg cacgattgcc ttgagacaat agacgagact ctcgacgaac tccacacggc     480
   cataaaagac ctcgagctat atcccaacaa aaagtctctg aaagcccacg cagatgacct    540
   taaaactcta ataagttcag caattactaa ccaagagact tgccttgacg gtttctctca    600
   cgacgacgcc gataaaaagg ttcgcaaagc tttgctgaaa ggtcaaaagc acgtagaaaa     660
   aatgtgcagt aatgctttag ctatgatttg taatatgacc gatacagaca ttgctaatga     720
   gcagaagttg aagggtacca ctactaacag gaagttgaga gaggacaata gtgagtggcc     780
   ggagtggttg tctgctgggg acagaagatt gctgcagtcc tcgacggtga ggcccgacgt     840
   ggttgtggcg gcggacggaa gcggaaattt caagacggtg tcggaggcgg tagcaaaagc     900
   accagagaag agcagcaaga ggtatgtaat aaggataaaa gctggtgttt acagggagaa     960
   tgtagatgtg ccgaagaaga agacaaatat tatgtttatg ggagatggaa gaagcaatac    1020
   tattattaca ggaagtagaa atgtgaaaga tggtagcact actttccact ccgccactgt   1080
   tgctgcggta ggtgaaaaat tcttggcccg agacataact ttccaaaaca ctgcaggggc   1140
   cgcaaaacac caagccgtag cacttcgcgt ggggtctgat ttgtccgctt tctatagatg   1200
   tgacatttta gcctatcaag actctctcta cgttcactcc aatcgtcaat actttgttca   1260
   gtgtttaatt gctggcacag tcgattttat ttttggcaat gctgctgctg ttttacagaa   1320
   ctgtgatatt catgcccgac gtcctggttc gggtcagaag aacatggtca ctgcccaagg   1380
   aagatctgac ccgaaccaga acactggaat tgtgatccag aaatgtagaa ttggtgcaac   1440
   gtctgatttg agaccagttc agaagagttt ccccacgtat cttggacggc catggaaaga   1500
   atattctagg actgtgatta tgcaatcgtc aattactgat gtgattaatt ctgctggatg   1560
   gcatgaatgg aatgggaatt ttgcgcttaa tactttgttt tatggcgagt atcaaaatac   1620
   tggggcagga gctgggacct caggaagagt taaatggaaa gggtttaagg tcattacaag   1680
   tgcaactgag gctcaagctt atactcctgg tagattcatt gctggaggta gttggttgag   1740
   ctccactggc ttcccttttt ctcttggtct ctgaaatcct ttattttaag tagtattttg   1800
   ttttcaagtt tttgtgaagt ttcatgagaa atgtttattt tgagccggag gacactcatg   1860
   catggcaatg gtgtttggct gatggagaaa taacaagatt catcaacgaa taggggttgg   1920
   aaattttctt tatgaacttc tattcgtgct gaggaatttt agtgatatag acctaaatat   1980
   tccctaatgt gtgtaattat cctatctaaa agcgaaaagc caaaagaatg tggaaataaa   2040
   ataagagaat ttcaaagctc                                               2060
<210> 2
   <211> 579
   <212> PRT
   <213> Nicotiana tabacum
<400> 2
   Met Leu Asp Ser Gly Lys Asn Val Asn Phe Ser Lys Gly Lys Lys Lys
   1               5                   10                  15
   Leu Phe Leu Thr Val Val Ala Ser Val Leu Leu Val Ala Ala Val Ile
               20                  25                  30
   Gly Val Val Val Gly Val Lys Phe Arg Ser Asn Asn Ser Asp Asp His
           35                  40                  45
   Ala Asp Ile Gln Ala Ile Thr Ser Ala Ala His Ala Ile Val Lys Ser
       50                  55                  60
   Ala Cys Glu Asn Thr Leu His Pro Glu Leu Cys Tyr Ser Thr Ile Ala
   65                  70                  75                  80
   Ser Val Ser Asp Phe Ser Lys Lys Val Thr Ser Gln Lys Asp Val Ile
   85 90 95
   Glu Leu Ser Leu Asn Ile Thr Cys Arg Ala Val Gln His Asn Phe Phe
   100 105 110
   Lys Val Glu Lys Leu Ile Lys Thr Arg Lys Gly Leu Lys Pro Arg Glu
   115 120 125
   Lys Val Ala Leu His Asp Cys Leu Glu Thr Ile Asp Glu Thr Leu Asp
   130 135 140
   Glu Leu His Thr Ala Ile Lys Asp Leu Glu Leu Tyr Pro Asn Lys Lys
   145 150 155 160
   Ser Leu Lys Ala His Ala Asp Asp Leu Lys Thr Leu Ile Ser Ser Ala
   165 170 175
   Ile Thr Asn Gln Glu Thr Cys Leu Asp Gly Phe Ser His Asp Asp Ala
   180 185 190
   Asp Lys Lys Val Arg Lys Ala Leu Leu Lys Gly Gln Lys His Val Glu
   195 200 205
   Lys Met Cys Ser Asn Ala Leu Ala Met Ile Cys Asn Met Thr Asp Thr
   210 215 220
   Asp Ile Ala Asn Glu Gln Lys Leu Lys Gly Thr Thr Thr Asn Arg Lys
   225 230 235 240
   Leu Arg Glu Asp Asn Ser Glu Trp Pro Glu Trp Leu Ser Ala Gly Asp
   245 250 255
   Arg Arg Leu Leu Gln Ser Ser Thr Val Arg Pro Asp Val Val Val Ala
   260 265 270
   Ala Asp Gly Ser Gly Asn Phe Lys Thr Val Ser Glu Ala Val Ala Lys
   275 280 285
   Ala Pro Glu Lys Ser Ser Lys Arg Tyr Val Ile Arg Ile Lys Ala Gly
   290 295 300
   Val Tyr Arg Glu Asn Val Asp Val Pro Lys Lys Lys Thr Asn Ile Met
   305 310 315 320
   Phe Met Gly Asp Gly Arg Ser Asn Thr Ile Ile Thr Gly Ser Arg Asn
                   325                 330                 335
   Val Lys Asp Gly Ser Thr Thr Phe His Ser Ala Thr Val Ala Ala Val
               340                 345                 350
   Gly Glu Lys Phe Leu Ala Arg Asp Ile Thr Phe Gln Asn Thr Ala Gly
           355                 360                 365
   Ala Ala Lys His Gln Ala Val Ala Leu Arg Val Gly Ser Asp Leu Ser
       370                 375                 380
   Ala Phe Tyr Arg Cys Asp Ile Leu Ala Tyr Gln Asp Ser Leu Tyr Val
   385                 390                 395                 400
   His Ser Asn Arg Gln Tyr Phe Val Gln Cys Leu Ile Ala Gly Thr Val
                   405                 410                 415
   Asp Phe Ile Phe Gly Asn Ala Ala Ala Val Leu Gln Asn Cys Asp Ile
               420                 425                 430
   His Ala Arg Arg Pro Gly Ser Gly Gln Lys Asn Met Val Thr Ala Gln
           435                 440                 445
   Gly Arg Ser Asp Pro Asn Gln Asn Thr Gly Ile Val Ile Gln Lys Cys
       450                 455                 460
   Arg Ile Gly Ala Thr Ser Asp Leu Arg Pro Val Gln Lys Ser Phe Pro
   465                 470                 475                 480
   Thr Tyr Leu Gly Arg Pro Trp Lys Glu Tyr Ser Arg Thr Val Ile Met
                   485                 490                 495
   Gln Ser Ser Ile Thr Asp Val Ile Asn Ser Ala Gly Trp His Glu Trp
               500                 505                 510
   Asn Gly Asn Phe Ala Leu Asn Thr Leu Phe Tyr Gly Glu Tyr Gln Asn
           515                 520                 525
   Thr Gly Ala Gly Ala Gly Thr Ser Gly Arg Val Lys Trp Lys Gly Phe
       530                 535                 540
   Lys Val Ile Thr Ser Ala Thr Glu Ala Gln Ala Tyr Thr Pro Gly Arg
   545                 550                 555                 560
   Phe Ile Ala Gly Gly Ser Trp Leu Ser Ser Thr Gly Phe Pro Phe Ser
                   565                 570                 575
   Leu Gly Leu
<210> 3
   <211> 583
   <212> PRT
   <213> Lycopersicon esculentum
<400> 3
   Met Thr Arg Val Glu Asp Phe Phe Ser Lys Gln Ile Asp Phe Cys Lys
   1               5                   10                  15
   Arg Lys Lys Lys Ile Tyr Leu Ala Ile Val Ala Ser Val Leu Leu Val
               20                  25                  30
   Ala Ala Val Ile Gly Val Val Ala Gly Val Lys Ser His Ser Lys Asn
           35                  40                  45
   Ser Asp Asp His Ala Asp Ile Met Ala Ile Ser Ser Ser Ala His Ala
       50                  55                  60
   Ile Val Lys Ser Ala Cys Ser Asn Thr Leu His Pro Glu Leu Cys Tyr
   65                  70                  75                  80
   Ser Ala Ile Val Asn Val Ser Asp Phe Ser Lys Lys Val Thr Ser Gln
                   85                  90                  95
   Lys Asp Val Ile Glu Leu Ser Leu Asn Ile Thr Val Lys Ala Val Arg
               100                 105                 110
   Arg Asn Tyr Tyr Ala Val Lys Glu Leu Ile Lys Thr Arg Lys Gly Leu
           115                 120                 125
   Thr Pro Arg Glu Lys Val Ala Leu His Asp Cys Leu Glu Thr Met Asp
       130                 135                 140
   Glu Thr Leu Asp Glu Leu His Thr Ala Val Glu Asp Leu Glu Leu Tyr
   145                 150                 155                 160
   Pro Asn Lys Lys Ser Leu Lys Glu His Val Glu Asp Leu Lys Thr Leu
                   165                 170                 175
   Ile Ser Ser Ala Ile Thr Asn Gln Glu Thr Cys Leu Asp Gly Phe Ser
               180                 185                 190
   His Asp Glu Ala Asp Lys Lys Val Arg Lys Val Leu Leu Lys Gly Gln
   195 200 205
   Lys His Val Glu Lys Met Cys Ser Asn Ala Leu Ala Met Ile Cys Asn
   210 215 220
   Met Thr Asp Thr Asp Ile Ala Asn Glu Met Lys Leu Ser Ala Pro Ala
   225 230 235 240
   Asn Asn Arg Lys Leu Val Glu Asp Asn Gly Glu Trp Pro Glu Trp Leu
   245 250 255
   Ser Ala Gly Asp Arg Arg Leu Leu Gln Ser Ser Thr Val Thr Pro Asp
   260 265 270
   Val Val Val Ala Ala Asp Gly Ser Gly Asp Tyr Lys Thr Val Ser Glu
   275 280 285
   Ala Val Arg Lys Ala Pro Glu Lys Ser Ser Lys Arg Tyr Val Ile Arg
   290 295 300
   Ile Lys Ala Gly Val Tyr Arg Glu Asn Val Asp Val Pro Lys Lys Lys
   305 310 315 320
   Thr Asn Ile Met Phe Met Gly Asp Gly Lys Ser Asn Thr Ile Ile Thr
   325 330 335
   Ala Ser Arg Asn Val Gln Asp Gly Ser Thr Thr Phe His Ser Ala Thr
   340 345 350
   Val Val Arg Val Ala Gly Lys Val Leu Ala Arg Asp Ile Thr Phe Gln
   355 360 365
   Asn Thr Ala Gly Ala Ser Lys His Gln Ala Val Ala Leu Cys Val Gly
   370 375 380
   Ser Asp Leu Ser Ala Phe Tyr Arg Cys Asp Met Leu Ala Tyr Gln Asp
   385 390 395 400
   Thr Leu Tyr Val His Ser Asn Arg Gln Phe Phe Val Gln Cys Leu Val
   405 410 415
   Ala Gly Thr Val Asp Phe Ile Phe Gly Asn Gly Ala Ala Val Phe Gln
   420 425 430
   Asp Cys Asp Ile His Ala Arg Arg Pro Gly Ser Gly Gln Lys Asn Met
   435 440 445
   Val Thr Ala Gln Gly Arg Thr Asp Pro Asn Gln Asn Thr Gly Ile Val
       450                 455                 460
   Ile Gln Lys Cys Arg Ile Gly Ala Thr Ser Asp Leu Arg Pro Val Gln
   465                 470                 475                 480
   Lys Ser Phe Pro Thr Tyr Leu Gly Arg Pro Trp Lys Glu Tyr Ser Arg
                   485                 490                 495
   Thr Val Ile Met Gln Ser Ser Ile Thr Asp Val Ile Gln Pro Ala Gly
               500                 505                 510
   Trp His Glu Trp Asn Gly Asn Phe Ala Leu Asp Thr Leu Phe Tyr Gly
           515                 520                 525
   Glu Tyr Ala Asn Thr Gly Ala Gly Ala Pro Thr Ser Gly Arg Val Lys
       530                 535                 540
   Trp Lys Gly His Lys Val Ile Thr Ser Ser Thr Glu Ala Gln Ala Tyr
   545                 550                 555                 560
   Thr Pro Gly Arg Phe Ile Ala Gly Gly Ser Trp Leu Ser Ser Thr Gly
                   565                 570                 575
   Phe Pro Phe Ser Leu Gly Leu
               580
<210> 4
   <211> 584
   <212> PRT
   <213> Citrus sinensis
<400> 4
   Met Thr His Ile Lys Glu Phe Phe Thr Lys Leu Ser Glu Ser Ser Ser
   1 5 10 15
   Asn Gln Asn Ile Ser Asn Ile Pro Lys Lys Lys Lys Lys Leu Phe Leu
   20 25 30
   Ala Leu Phe Ala Thr Leu Leu Val Val Ala Ala Val Ile Gly Ile Val
   35 40 45
   Ala Gly Val Asn Ser Arg Lys Asn Ser Gly Asp Asn Gly Asn Glu Pro
   50 55 60
   His His Ala Ile Leu Lys Ser Ser Cys Ser Ser Thr Arg Tyr Pro Asp
   65 70 75 80
   Leu Cys Phe Ser Ala Ile Ala Ala Val Pro Glu Ala Ser Lys Lys Val
   85 90 95
   Thr Ser Gln Lys Asp Val Ile Glu Met Ser Leu Asn Ile Thr Thr Thr
   100 105 110
   Ala Val Glu His Asn Tyr Phe Gly Ile Gln Lys Leu Leu Lys Arg Thr
   115 120 125
   Asn Leu Thr Lys Arg Glu Lys Val Ala Leu His Asp Cys Leu Glu Thr
   130 135 140
   Ile Asp Glu Thr Leu Asp Glu Leu His Lys Ala Val Glu Asp Leu Glu
   145 150 155 160
   Glu Tyr Pro Asn Lys Lys Ser Leu Ser Gln His Ala Asp Asp Leu Lys
   165 170 175
   Thr Leu Met Ser Ala Ala Met Thr Asn Gln Gly Thr Cys Leu Asp Gly
   180 185 190
   Phe Ser His Asp Asp Ala Asn Lys His Val Arg Asp Ala Leu Ser Asp
   195 200 205
   Gly Gln Val His Val Glu Lys Met Cys Ser Asn Ala Leu Ala Met Ile
   210 215 220
   Lys Asn Met Thr Asp Thr Asp Met Met Ile Met Arg Thr Ser Asn Asn
   225 230 235 240
   Arg Lys Leu Thr Glu Glu Thr Ser Thr Val Asp Gly Trp Pro Ala Trp
   245 250 255
   Leu Ser Pro Gly Asp Arg Arg Leu Leu Gln Ser Ser Ser Val Thr Pro
   260 265 270
   Asn Ala Val Val Ala Ala Asp Gly Ser Gly Asn Phe Lys Thr Val Ala
   275 280 285
   Ala Ala Val Ala Ala Ala Pro Gln Gly Gly Thr Lys Arg Tyr Ile Ile
   290 295 300
   Arg Ile Lys Ala Gly Val Tyr Arg Glu Asn Val Glu Val Thr Lys Lys
   305 310 315 320
   His Lys Asn Ile Met Phe Ile Gly Asp Gly Arg Thr Arg Thr Ile Ile
                   325                 330                 335
   Thr Gly Ser Arg Asn Val Val Asp Gly Ser Thr Thr Phe Lys Ser Ala
               340                 345                 350
   Thr Val Ala Val Val Gly Glu Gly Phe Leu Ala Arg Asp Ile Thr Phe
           355                 360                 365
   Gln Asn Thr Ala Gly Pro Ser Lys His Gln Ala Val Ala Leu Arg Val
       370                 375                 380
   Gly Ala Asp Leu Ser Ala Phe Tyr Asn Cys Asp Met Leu Ala Tyr Gln
   385                 390                 395                 400
   Asp Thr Leu Tyr Val His Ser Asn Arg Gln Phe Phe Val Asn Cys Leu
                   405                 410                 415
   Ile Ala Gly Thr Val Asp Phe Ile Phe Gly Asn Ala Ala Ala Val Leu
               420                 425                 430
   Gln Asn Cys Asp Ile His Ala Arg Lys Pro Asn Ser Gly Gln Lys Asn
           435                 440                 445
   Met Val Thr Ala Gln Gly Arg Thr Asp Pro Asn Gln Asn Thr Gly Ile
       450                 455                 460
   Val Ile Gln Lys Ser Arg Ile Gly Ala Thr Ser Asp Leu Lys Pro Val
   465                 470                 475                 480
   Gln Gly Ser Phe Pro Thr Tyr Leu Gly Arg Pro Trp Lys Glu Tyr Ser
                   485                 490                 495
   Arg Thr Val Ile Met Gln Ser Ser Ile Thr Asp Leu Ile His Pro Ala
               500                 505                 510
   Gly Trp His Glu Trp Asp Gly Asn Phe Ala Leu Asn Thr Leu Phe Tyr
           515                 520                 525
   Gly Glu His Gln Asn Ser Gly Ala Gly Ala Gly Thr Ser Gly Arg Val
       530                 535                 540
   Lys Trp Lys Gly Phe Arg Val Ile Thr Ser Ala Thr Glu Ala Gln Ala
   545                 550                 555                 560
   Phe Thr Pro Gly Ser Phe Ile Ala Gly Ser Ser Trp Leu Gly Ser Thr
                   565                 570                 575
   Gly Phe Pro Phe Ser Leu Gly Leu
   580
<210> 5
   <211> 30
   <212> DNA
   <213> Artificial Sequence
<220>
   <223> PCR primer
<400> 5
   tgacccatgg tggattccgg caagaacgtt 30
<210> 6
   <211> 30
   <212> DNA
   <213> Artificial Sequence
<220>
   <223> PCR primer
<400> 6
   ttttggatcc acggagacca agagaaaaag 30
<210> 7
   <211> 34
   <212> DNA
   <213> Artificial Sequence
<220>
   <223> PCR primer
<400> 7
   tgacggatcc ttggattccg gcaagaacgt taat 34
<210> 8
   <211> 34
   <212> DNA
   <213> Artificial Sequence
<220>
   <223> PCR primer
<400> 8
   cccctctaga tcagagacca agagaaaaag ggaa 34
<210> 9
   <211> 32
   <212> DNA
   <213> Artificial Sequence
<220>
   <223> PCR primer
<400> 9
   ttttccatgg tgaggcccga cgtggttgtg gc 32
<210> 10
   <211> 33
   <212> DNA
   <213> Artificial Sequence
<220>
   <223> PCR primer
<400> 10
   ataaggatcc gtgaggcccg acgtggttgt ggc 33
<210> 11
   <211> 30
   <212> DNA
   <213> Artificial Sequence
<220>
   <223> PCR primer
<400> 11
   ttttccatgg tgagcaaggg cgaggagctg 30
<210> 12
   <211> 49
   <212> DNA
   <213> Artificial Sequence
<220>
   <223> PCR primer
<400> 12
   ttttggatcc tattcttgcg ggccctcgct tgtacagctc gtccatgcc 49
<210> 13
   <211> 50
   <212> DNA
   <213> Artificial Sequence
<220>
   <223> PCR primer
<400> 13
   ttttggatcc ataagaacgg ggcccagagt gagcaagggc gaggagctgt 50
<210> 14
   <211> 30
   <212> DNA
   <213> Artificial Sequence
<220>
   <223> PCR primer
<400> 14
   tttttctaga ttacttgtac agctcgtcca 30
<210> 15
   <211> 34
   <212> DNA
   <213> Artificial Sequence
<220>
   <223> PCR primer
<400> 15
   tgacccatgg gttctaaggc atttctgttt cttg 34
<210> 16
   <211> 34
   <212> DNA
   <213> Artificial Sequence
<220>
   <223> PCR primer
<400> 16
   ttttggatcc ttgtgaaggc tgaacttcag taag 34
<210> 17
   <211> 34
   <212> DNA
   <213> Artificial Sequence
<220>
   <223> PCR primer
<400> 17
   ttttggatcc agatcttcgt aaagactttg accg 34
<210> 18
   <211> 34
   <212> DNA
   <213> Artificial Sequence
<220>
   <223> PCR primer
<400> 18
   ttttggatcc ttgtgaaggc tgaacttcag taag 34
<210> 19
   <211> 31
   <212> DNA
   <213> Artificial Sequence
<220>
   <223> PCR primer
<400> 19
   ttttccatgg tgagcaaggg cgaggagctg t 31
<210> 20
   <211> 49
   <212> DNA
   <213> Artificial Sequence
<220>
   <223> PCR primer
<400> 20
   ttttggatcc tattcttgcg ggccctcgct tgtacagctc gtccatgcc 49
<210> 21
   <211> 125
   <212> DNA
   <213> Artificial Sequence
<220>
   <223> artificial intron
<400> 21
   tatactcaag gttcgtaaag aactttttat tttatcagtg tagtttgagc agttgtgaca 60
   tattgtagtt ctttaatact catgaaattg tttttaattt ttaatatgga gtataggaga 120
   tggga 125
<210> 22
   <211> 57
   <212> DNA
   <213> Artificial Sequence
<220>
   <223> PCR primer
<400> 22
   gcatggtacc ccttaatacg actcactata gttttagttt tattgcaaca acaacaa 57
<210> 23
   <211> 35
   <212> DNA
   <213> Artificial Sequence
<220>
   <223> PCR primer
<400> 23
   gcatgcggcc gctgggcccc tacccggggt taggg 35
<210> 24
   <211> 60
   <212> DNA
   <213> Artificial Sequence
<220>
   <223> PCR primer
<400> 24
   gcatggtacc atttaggtga cactatagaa ctcgttttag ttttattgca acaacaacaa 60
<210> 25
   <211> 35
   <212> DNA
   <213> Artificial Sequence
<220>
   <223> PCR primer
<400> 25
   gcatgcggcc gctgggcccc tacccggggt taggg 35
<210> 26
   <211> 23
   <212> DNA
   <213> Artificial Sequence
<220>
   <223> artificial splicing site
<400> 26
   aacggtcggt aacggtcggt aaa 23
<210> 27
   <211> 28
   <212> DNA
   <213> Artificial Sequence
<220>
   <223> artificial splicing site
<400> 27
   tcgactttac cgaccgttac cgaccgtt 28
<210> 28
   <211> 39
   <212> DNA
   <213> Artificial Sequence
<220>
   <223> artificial splicing site
<400> 28
   agctaaccta gcaggttata tgcaggttat atgcaggtc 39
<210> 29
   <211> 39
   <212> DNA
   <213> Artificial Sequence
<220>
   <223> artificial splicing site
<400> 29
   ttggatcgtc caatatacgt ccaatatacg tccaggtac 39
<210> 30
   <211> 11
   <212> DNA
   <213> Artificial Sequence
<220>
   <223> artificial splicing site
<400> 30
   cgaaaggtaa g 11
<210> 31
   <211> 13
   <212> DNA
   <213> Artificial Sequence
<220>
   <223> artificial splicing site
<400> 31
   tcgacttacc ttt 13
<210> 32
   <211> 21
   <212> DNA
   <213> Artificial Sequence
<220>
   <223> artificial splicing site
<400> 32
   agctaaccta ttgcaggttg c 21
<210> 33
   <211> 21
   <212> DNA
   <213> Artificial Sequence
<220>
   <223> arificial splicing site
<400> 33
   catggcaacc tgcaataggt t 21
<210> 34
   <211> 18
   <212> DNA
   <213> Artificial Sequence
<220>
   <223> PCR primer
<400> 34
   gtggttgacg aattcgtc 18
<210> 35
   <211> 56
   <212> DNA
   <213> Artificial Sequence
<220>
   <223> PCR primer
<400> 35
   ggtctcgagt tatcgattat tcgggtttgt aatgttgtaa gacgttttct tctttc 56
<210> 36
   <211> 48
   <212> DNA
   <213> Artificial Sequence
<220>
   <223> PCR primer
<400> 36
   taactcgaga cctgcagcat gtcttacaac attacaaacc cgaatcag 48
<210> 37
   <211> 48
   <212> DNA
   <213> Artificial Sequence
<220>
   <223> PCR primer
<400> 37
   ctactccatg gtcaagctta agtagcagca gcagtagtcc acggcacc 48
<210> 38
   <211> 41
   <212> DNA
   <213> Artificial Sequence
<220>
   <223> PCR primer
<400> 38
   ggtaattgtt gtaaaaatac gatgggtgaa ggtggagtac g 41
<210> 39
   <211> 30
   <212> DNA
   <213> Artificial Sequence
<220>
   <223> PCR primer
<400> 39
   atcactcgag ctcgaagccg cggtgcgggt 30
<210> 40
   <211> 40
   <212> DNA
   <213> Artificial Sequence
<220>
   <223> PCR primer
<400> 40
   cgtactccac ctcacccatc gtatttttac aacaattacc 40
<210> 41
   <211> 37
   <212> DNA
   <213> Artificial Sequence
<220>
   <223> PCR primer
<400> 41
   catgccatgg gtaattgtaa atagtaattg taatgtt 37

## Description

### FIELD OF THE INVENTION

The present invention relates to a process and vectors for the production of a protein of interest in a plant or in plant cells and proteins and polypeptides obtained thereby. Further, the invention relates to vectors for this process and to plants or plant cells transformed therewith.

### BACKGROUND OF THE INVENTION

Recombinant protein production in plant systems has been very successful for many different products, covering proteins with industrial applications, food and feed additives, animal health products and human pharmaceuticals, such as antigens and immune response proteins.

There are many comprehensive reviews describing the field (Daniell, *et al.,* 2001 *Trends Plant Sci.,* 2001, 6:219-226; Larrick & Thomas, 2001, *Curr. Opin. Biotech.,* 12:411-418; Doran, 2000, *Curr. Opin. Biotech.,* 11:199-204; Hood & Jilka, 1999, *Curr. Opin. Biotech.,* 10:382-386). Plants have been considered as a low-cost production system for proteins, that is significantly cheaper in comparison with bacterial, yeast, insect and mammalian cell-based production systems. However, little consideration was given to the technical challenges associated with downstream protein purification from plant material. Recovery and purification of proteins from biomass is an expensive and technically complex process, and it may account for more than 90% of the total production cost, especially in "inexpensive" production systems such as yeasts or microbial cells. Purification from plant biomass is *a priori* even more difficult, and may represent the most serious bottleneck on the way to industrialization of plant-based production platforms. The available data in this regard confirm the above said. For example, extraction of β-glucuronidase from transgenic corn seeds accounts for 94% of the production cost (Evangelista *et al.,* 1998, *BiotechnoL Prog.,* 14:607-614).

A number of processes have been specifically designed in order to address the problem. Compartmentalization of the recombinant protein within the plant cell followed by its secretion is one pre-requisite of making the product easily purifiable. Rhizosecretion of recombinant proteins was demonstrated for engineered tobacco plants (Borisjuk *et al.,* 1999, *Nat. Biotechnol.,* 17, 466-469; US 6096546). It was shown that targeting a protein to the endoplasmatic reticulum using a fusion with a signal peptide allows to obtain secreted protein that is already partially purified (is in the secretion fluid, along with just a dozen or so of proteins normally secreted by a plant) and in significantly higher amounts, as opposed to isolating it from homogenized plant tissues that are highly complex mixtures of thousands of proteins and other molecules. A similar approach was used with plant viral expression vectors, where the protein was targeted to the apoplast so that purification begins with a highly enriched product (for review see: Yusibov *et al.,* 1999, *Curr Top. Microbial. Immunol.,* 240, 81-94). Another technology, developed by M. Moloney (US 5650554), solves the problem by targeting the protein of interest, as a part of oleosin fusion, to oil bodies, i.e. a cell fraction that is easily removable.

Yet another important strategy is to equip the recombinant protein with an affinity tag, thus facilitating the purification of proteins through affinity chromatography. This method is widely described for *in vitro* purification of proteins using various affinity tags, such as polyhistidine, Flag, protein A, gluthatione-S-transferase, cellulose-binding domains (CBD), etc. (Sassenfeld, 1990, *TIBTECH,* 8, 88-93). In the case of CBD fusion proteins, the protein of interest is fused to a substrate-binding region of a polysaccharidase (cellulases, chitinases and amylases, as well as xylanases and the β-1,4 glycanases). The affinity matrix containing the substrate such as cellulose can be employed to immobilize the polypeptide. The polypeptide or fusion protein can be removed from the matrix using a protease cleavage site. Different variants of this approach using CBDs of plant and bacterial origin are described in several publications and patents (Ong *et al.,* 1991, *Enzyme Microb. Technol.,* 13, 59-65; Linder *et al.,* 1998, *Biotechnol. Bioeng.,* 60, 642-647; US 5137819; US 5202247; US 5670623; US 5719044; US 5962289; US 6060274). This version of purification has never been tested with plants, because of concerns that the material of interest will be attached to the plant cell wall that contains cellulose and will be impossible to purify. A further concern has been that targeting proteins to the plant cell wall interferes with plant growth (WO 00/77174).

The approaches described above focus either on targeting recombinant proteins to a secretory pathway or on employing affinity tags, such as cellulose binding domains for an *in vitro* purification process. There are also inventions describing the subcellular targeting of cellulolytic enzymes for large scale cellulase production and cellulose degradation (WO 9811235), or for generating transgenic plants with altered structure or morphology (US 6184440). None of these patents focuses on improving the protein purification procedure. Rather, they address problems of cellulolytic enzyme toxicity for large-scale production and/or their use for modulation of cell wall morphology. Actually, these technologies are limited to the expression of cell wall degrading or modifying enzymes. Of course, for the production of such enzymes in plants, a subcellular localization is preferred. However, cellulases and cell wall modifying enzymes represent only a small fraction of proteins with commercial value. There is no technology for cheap large scale production and purification of recombinant proteins in general, especially of cytotoxic proteins in plants.

US 5,474,925 discloses a method of targeting a recombinant protein of interest to cotton fibers in cotton plants. The protein of interest is immobilised on said cotton fibers and is used in industrial processes in this immobilised state.

Ziv & Shoseyov (US6331416, WO 00/77174) describe a method of expressing a recombinant protein of interest as a protein fusion with a cellulose binding domain (CBD), whereby binding of said CBD to cell wall cellulose is used during work up for affinity purification of the protein fusion. Since binding of a protein to cell wall cellulose interferes with plant growth and leads to growth arrest, the authors compartmentalise the fusion protein within cells in order to sequester the fusion protein from the cell walls. The protein fusions are brought in contact with cell wall cellulose by homogenising the plant material. This methods has several severe disadvantages which render it practically useless for large large scale or commercial applications like molecular farming:
(i) since the fusion protein is expressed during plant growth and accumulate in cellular compartments, the plant suffers from a substantial burden during growth. Thus, plant growth is slowed down and the maximum plant size is reduced compared to the wild-type plants leading to a loss of biomass and to limited amount of produced recombinant protein. More plants would have to be grown to achieve the same biomass as with wild-type plants meaning consumption of more expensive greenhouse space more expenditure during down-stream processing and protein purification;
(ii) proteins that are toxic to the plant cannot be expressed;
(iii) plants expressing a particular fusion protein have to be raised starting from transgenic seeds or plantlets requiring essentially a whole season for the production of a particular protein of interest. The molecular farmer cannot react to short-term orders for a particular protein. Therefore, this method is extremely unflexible.

It is an object of the invention to provide a process of producing a protein of interest in plants, whereby interference of foreign protein expression with plant growth is not a problem.

It is a further object of the invention to provide a process of producing a protein of interest in plants that allows production of proteins that are toxic to the plant.

It is a further object of the invention to provide a process of producing a protein of interest in plants or plant cells, which is significantly simpler compared to existing technologies and offers an economic way of protein production in plants.

### GENERAL DESCRIPTION OF THE INVENTION

These objects are solved by a process of producing a protein or polypeptide of interest in a plant or in plant cells, comprising:
(I) transforming or transfecting a plant or plant cells with a viral vector, said viral vector containing a nucleotide sequence having a coding region encoding a fusion protein comprising the protein or polypeptide of interest, a signal peptide functional for targeting said fusion protein to the apoplast, and a polypeptide capable of binding the fusion protein to a cell wall component,
(ii) enriching cell wall components having expressed and bound fusion protein, and
(iii) separating the protein or polypeptide of interest or a protein comprising the protein or polypeptide of interest from said cell wall components.

The inventors of the present invention managed to overcome the shortcomings of the prior art by recognising that the life time of a plant used to express a protein of interest can be devided into two conceptually different phases: (i) plant life and growth before transformation or transfection and (ii) plant life and growth after transformation or transfection.

In the process of the invention, a plant used for expressing said fusion protein is preferably first grown to a desired growth state before transformation or transfection. Said desired growth state may be a growth state which is favourable for the expression of a particular fusion protein. Said growth state is preferably close to the maximum growth the plant can achieve, i.e. when maximum or near maximum biomass has accumulated. Transforming or transfecting a plant that has accumulated substantial biomass has the following advantages: (a) many cells and/or a big amount of biomass is available per plant for the expression of the fusion protein; (b) binding of the fusion protein to the cell wall in the apoplast does not interfere with plant growth or such interference does not impede the process of the invention; (c) the nucleotide sequence used for transformation can be selected at a late stage, providing a high degree of flexibility to the molecular farmer.

The approach of the invention has the essential advantage that plant growth is not perturbed by the expression of the fusion protein. Plants, notably wild type plants, can be grown up to a desired growth state without any burden by the expression of said fusion protein. Since the maximum amount protein of interest that can be produced depends on the plant biomass, more protein of interest can be produced per plant than in prior art processes, where plant growth is impeded by the expression of a foreign protein and/or accumulation of a foreign protein in compartments of the cells or in the cell wall. Therefore, the process of the invention is more efficient, more productive, and cheaper than prior art processes.

A plant having expressed said fusion protein may be harvested for carrying out step (ii) at any suitable time. As described above, transforming or transfecting a grown plant is preferred according to the invention, since the time required between transforming or transfecting and harvesting said plants can be reduced enormously. Plants are then preferably harvested soon after said transforming or transfecting. More preferably, plants are harvested between one and seven days, most preferably after two to four days after said transforming or transfecting. In contrast to the prior art, disturbance of cell wall function by binding of the fusion protein is therefore not a problem.

Moreover, the process of the invention provides the molecular farmer with a great degree of flexibility, since the decision of which protein to express (which nucleic acid to transform) can be postponed until a late stage in plant development. If a molecular farmer has grown plants at hand and a number of vectors each encoding a commercial protein, he can react quickly to sudden market requirements and provide big amounts of a protein of interest on short notice, e.g. within one week. The process of the invention therefore fits to modern market needs such as just-in-time production. In contrast, prior art processes require long-term planning of the protein to be produced.

The process of producing a protein or polypeptide of interest according to the invention involves expression of said protein or polypeptide of interest as a fusion protein which is secreted from the cells which express the fusion protein by way of a signal peptide. In the apoplast, the fusion protein will bind to a cell wall component by way of said polypeptide capable of binding the fusion protein to a cell wall component. Thus, the plant cell wall or its components are exploited as affinity matrix for purifying the protein or polypeptide of interest or a protein comprising the protein or polypeptide of interest.

In the first step of the process of the invention, a plant or plant cells are transformed or transfected with a viral vector, said viral vector containing a nucleotide sequence having a coding region encoding said fusion protein. Transformation may produce stably transformed plants or plant cells, e.g. transgenic plants. Alternatively, said plant or plant cells may be transfected for transient expression of said fusion protein. Transient transfection of grown up plants is preferred.

Several transformation or transfection methods for plants or plant cells are known in the art and include *Agrobacterium-*mediated transformation, particle bombardment, PEG-mediated protoplast transformation, viral Infection etc. For the preferred embodiment of transient expression of transfection for transient expression, viral infection or *Agrobacterium*-mediated transformation advantageously employed.

Said nucleotide sequence may be DNA or RNA depending on the transformation or transfection method. In most cases, it will be DNA. In an important embodiment, however, transformation or transfection is performed using RNA virus-based vectors, in which case said nucleotide sequence is RNA.

One very convenient way is to use a DNA vector that is based on a virus. Preferably, the DNA vector is based on an RNA virus, i.e. the DNA vectors contains the cDNA of RNA viral sequences in addition to said nucleotide sequence. Examples of plant DNA or RNA viruses sequences of which may be used for viral vectors according to the invention are given in WO 02/29068 and in PCT/EP02/03476. Such DNA vectors further contain a transcriptional promoter for producing the RNA viral transcript. In these embodiments, transformation or transfection is preferably carried out by viral transfection, more preferably via *Agrobacterium*-mediated transformation.

Said nucleotide sequence comprises a coding region encoding a fusion protein. Said fusion protein comprises the protein or polypeptide of interest (referred to as "protein of interest" in the following). Said protein of interest may be any protein or polypeptide that can be produced and Isolated according to the process of the invention. It may be produced in an unfolded, misfolded or in a natural, functional folding state. The latter possibility is preferred. Pharmaceutical proteins are particularly preferred.

Said fusion protein further comprises a signal peptide functional for targeting said fusion protein to the apoplast. This may be achieved with a signal peptide that targets the fusion protein into the endoplasmatic reticulum (ER) and the secretory pathway. All signal peptides of proteins known to be secreted or targeted to the apoplast may be used for the purposes of the invention. Preferred examples are the signal peptides of tobacco calreticulin or of rice amylase. Further examples are the signal peptides of pectin methylesterase or of *N. tabacco* tyrosine and lysine rich protein (NtTLPR). A further example is the signal peptide of apoplastic isoperoxidase from zucchini (APRX) (Carpin *et al.,* 2001, *The Plant Cell*, 13, 511-520). The signal peptide has to be comprised in said fusion protein such that it is functional for said targeting. This conditions may be fulfilled at any location in the fusion protein. In general, however, the signal peptide is positioned at the N-terminus of the fusion protein for functional targeting of the fusion protein to the apoplast.

Said fusion protein further comprises a polypeptide capable of binding the fusion protein to a cell wall component. By way of said polypeptide, a predominant fraction of the fusion protein secreted to the apoplast is bound or immobilized on the cell wall. Binding to the cell wall may be to any cell wall component. Cell wall components for the purposes of this invention include polysaccharides like cellulose, hemicellulose, β-1,4-glycan, pectin etc. and non-polysaccharides like proteins or lignin. Binding to polysaccharide components of the cell wall is preferred. Most preferred is binding to cellulose.

Pectin is another major cell wall polymer found in all land plants (Willats *et al.,* 2001, *Plant Mol Biol,* 47, 9-27). Pectin is a complex polymeric network of polysaccharides like homogalacturonan and rhamnogalacturonan. The main monomeric component of this network is galacturonic acid and not glucose like in the case of cellulose. Peroxidases are involved in the construction of cell walls and in the control of cell wall plasticity by catalysing the crosslinking of aromatic molecules using hydrogen peroxide as an electron acceptor. Another reaction known to be catalysed by peroxidases is the establishment of covalent bonds between hydroxycinnamate ester moieties or flavonoids associated with pectins or hemicellulose. For several peroxidases a specific interaction between the cell wall polymer homogalacturonan was found which seems to be essential for their activity. In one example of this invention we have used Ca²⁺-pectate binding site of apoplastic isoperoxidase from zucchini (APRX) (Carpin *et al.,* 2001, *The Plant Cell,* 13, 511-520). Said site binds *in vivo* as well *in vitro* to pectin chains through cross-links formed by Ca²⁺-ions (see Examples 6 and 7).

Examples of polypeptides capable of binding the fusion protein to a cell wall component are proteins which use such a cell wall component as a substrate like cellulases or hemicellulases. The substrate binding domain of such enzymes is preferably used as said polypeptide capable of binding. Further examples are polysaccharide binding domains (PBDs) of microbial origin (see below). An example specifically exemplified herein is pectin methylesterase (Examples 1 to 3). In these examples, binding to a cell wall component will in general be non-covalent. Alternatively, binding may involve a covalent bond to a cell wall component (cross-linking). An example is NtTLPR which can form disulfide bonds to a proteinaceous cell wall component.

In step (ii) of the process of the invention, cell wall components having expressed and bound fusion protein are enriched. This step involves the removal of many compounds contained in plants and results in a substantial purification of the protein of interest to be produced. While secretion of the fusion protein out of the expressing cell (protoplast) separates the fusion protein containing the protein of interest spatially from most protoplast compounds, these protoplast compounds may be removed in this step. Cell wall preparation procedures known in the art may be used in step (ii). Step (ii) may e.g. be performed by harvesting the plant or plant cells followed by homogenization of the plant tissue which contains expressed fusion protein of the invention. Liquid and solid phases of the homogenisate should then be separated e.g. by employing centrifugation or filtration. The cell wall components may also be enriched by pressing or squashing e.g. between rolls. The solid residue having the fusion protein bound or immobilized thereto is then preferably washed with a suitable washing buffer in order to remove soluble contaminants, again followed by recovering the insoluble cell wall material. The conditions used in step (ii) are preferably chosen to preserve the folding state of the protein of interest-domain in the fusion protein. The washing buffer is preferably aqueous. However, use of organic solvents may also be advantageous depending on the protein to be produced. In order to fully exploit the advantages of the invention, the cell wall component carrying the fusion protein should stay insoluble, preferably at least in the initial stages of the enriching of step (ii). However, the enriching/purification may be designed in many different ways and may also involve separation of cell wall components.

In the final step (iii) of the invention, the protein of interest or a fusion protein comprising the protein of interest is separated from the cell wall component to which it bound in step (i). This separation may be done in many different ways. In one embodiment, the fusion protein may be separated from the cell wall or respective cell wall component. (It is noted that, in most cases, the fusion protein will not comprise the signal peptide any more due to natural cleaving off during secretion of the fusion protein.) The way this may be done depends on the situation, especially on the polypeptide used for binding the fusion protein to the cell wall component. For many polypeptides which bind to a cell wall component, the conditions under which tight or loose binding occurs are known. Alternatively, these conditions can be determined experimentally. If the binding is non-covalent, binding may e.g. be releaved or weakened by adjusting the ionic strength, the pH, temperature or a buffer component (e.g. a chaotropic agent) (Greenwood et al. (1988) FEBS Lett. 224, 127-131; Shoseyov et al. (1992) Proc. Natl. Acad. Sci. USA 89, 3483-3487). In the case of covalent binding, the type of the covalent bond has to be taken into account. In the case of NtTLRP as a polypeptide capable of binding to a cell wall component, addition of a reducing agent containing free -SH groups like mercaptoethanol or dithiothreitol for cleaving disulfide bonds may be used.

Preferably, the separation of step (iii) involves cleavage of the fusion protein such that the protein of interest is obtained free or essentially free of other fusion protein components. This embodiment involves cleavage of at least one peptide bond. To this end, the fusion protein may further comprise a cleavage sequence allowing cleavage of the fusion protein. The cleavage sequence may be a peptide recognized by a site-specific protease. Addition of the appropriate protease may then cut the protein of interest out of the fusion protein still bound to a cell wall component. Removal of remaining cell wall components then produces the protein of interest free of most plant material. As cleavage sequence/protease systems those generally used for removing affinity tags from affinity-purified proteins may also be used for this invention. Such systems are commercially available e.g. from Amersham Pharmacia Biotech, Uppsala, Sweden. A specific example frequently used for removing a His-tag is the factor Xa system. In another embodiment, ubiquitin may be included in the fusion protein and cleavage may be obtained with a ubiquitin-specific hydrolase. The cleavage sequence may also be autocatalytic, whereby cleavage may be achieved by providing appropriate conditions for cleavage, e.g. intein-mediated cleavage may be used. The protein of interest thus obtained may be concentrated. Depending on the situation, the protein of interest may be subjected to further purification.

Construction of the nucleotide sequence of the invention may be done according to standard procedures of molecular biology. Said nucleotide sequence preferably contains a plant-specific promoter operably linked to said coding sequence and a transcripiton terminator after said coding region. Tissue-specific expression of said fusion protein may be achieved if desired by appropriate selection of the promoter. In a preferred embodiment, virus-based vectors are used for performing this invention. Construction of such vectors is described in the reference examples.

Further information on practical aspects of step (ii) and (iii) of the invention are found in WO 00/77174 references cited therein.

The nucleotide sequence comprises a coding region which codes for said fusion protein. The components of said fusion protein may be arranged in different orders. However, the signal peptide is preferably placed at the N-terminal end of the fusion protein in order to be functional for targeting. The protein of interest is preferably placed at the C-terminus of the fusion protein, which allows its separation from the remainder of the fusion protein by a single peptide bond cleavage. The polypeptide capable of binding will then be located in between the signal peptide and the protein of interest. However, different orders are also comprised by the invention e.g. placing the polypeptide capable of binding to a cell wall component at the C-terminus of the fusion protein. The protein of interest may further be flanked on both sides by a polypeptide capable of binding to a cell wall component, whereby these polypeptides may be the same or different. The protein of interest may then be cleaved out by two proteolytic events. Preferably, these two proteolytic events use the same cleavage sequence. The protein of interest may further be included in an intein for convenient isolation.

### Preferred embodiments of the invention

In a preferred embodiment, this invention provides a process of producing a protein or polypeptide of interest in a plant, comprising:
(ia) growing a plant to a desired growth state;
(ib) transiently transforming or transfecting the plant of step (ia) or cells thereof with a viral vector, said vector containing a nucleotide sequence having a coding region encoding a fusion protein comprising the protein or polypeptide of interest, a signal peptide functional for targeting said fusion protein to the apoplast, and a polypeptide capable of binding the fusion protein to a cell wall component,
(ii) enriching cell wall components having expressed and bound fusion protein, and
(iii) separating the protein or polypeptide of interest or a protein comprising the protein or polypeptide of interest from said cell wall components
whereby said polypeptide capable of binding the fusion protein to a cell wall component binds to cellulose or to pectin; binding to pectin is most preferred.

In another preferred embodiment, this invention provides a process of producing a protein or polypeptide of interest in a plant, comprising:
(i) transforming or transfecting the plant or cells thereof with a viral vector, said viral vector containing nucleotide sequence having a coding region encoding a fusion protein comprising the protein or polypeptide of interest, a signal peptide functional for targeting said fusion protein to the apoplast, and a polypeptide capable of binding the fusion protein to a cell wall component,
(ii) enriching cell wall components having expressed and bound fusion protein, and
(iii) separating the protein or polypeptide of interest or a protein comprising the protein or polypeptide of interest,
whereby said nucleotide sequence is amplified and/or expressed in said plant by a process comprising: providing the plant or cells thereof with at least one precursor vector designed for undergoing processing in cells of said plant, whereby due to said processing said plant cell is endowed with at least one replicon which provides for said amplification and/or expression.

More preferably, the plant or cells thereof is provided with at least two precursor vectors. Alternatively, due to said processing, said plant cell is endowed with at least two replicons which
(a) are structurally related to each other owing to said processing;
(b) are functionally distinct from each other; and
(c) provide for said amplification and/or expression.
The embodiments involving precursor vectors are further described below and are exemplified in example 6 and 7.

### BRIEF DESCRIPTION OF THE FIGURES

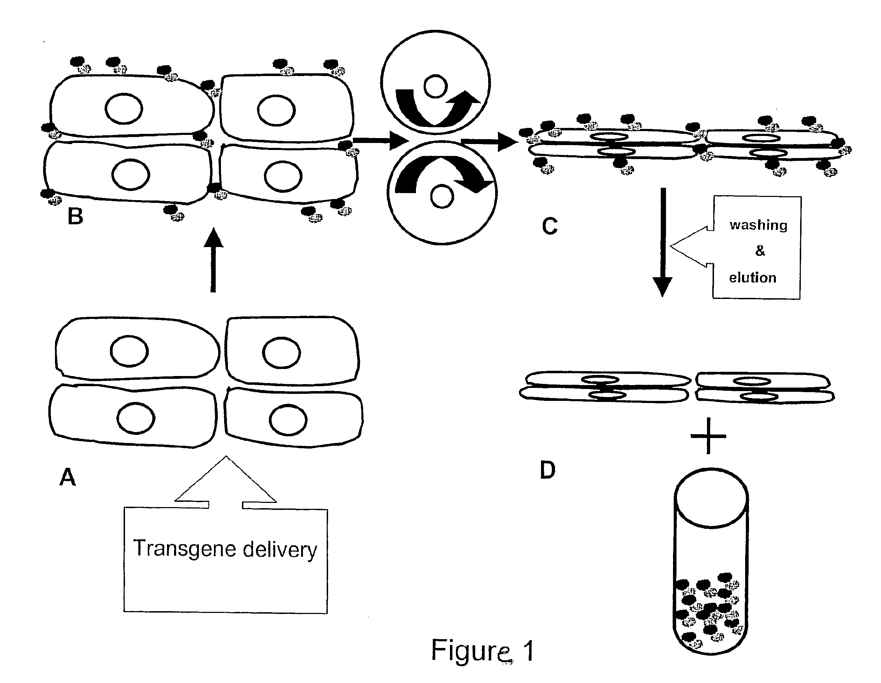

- Fig. 1: shows the general scheme of recombinant protein production in plant cells according to the invention.
A - transgene delivery into the plant cells by different means (viral vectors - RNA, DNA viruses; stable nuclear or plastid transformation; transient *Agrobacterium-*mediated expression - "agroinfiltration");
B - recombinant protein synthesis and attachment to cell wall;
C - enrichment of the plant tissue for recombinant protein by squashing-out cytosol;
D - separation of recombinant protein from the plant tissue.
- Fig. 2: shows the cDNA sequence of the full tobacco pectin methylesterase (PME) gene. Translation start and stop codons are shown in bold.
- Fig. 3: shows an alignment of the deduced amino acid sequences of PME genes from tobacco (accession No. AJ401158), tomato (accession No. U49330) and orange (accession No. U82976). Alignment of deduced amino acid sequences was performed using the Genebee program package. The arrow indicates the N-terminus of a processed PME protein. The transmembran helix (black box) and PME signatures 1 and 2 are boxed.
- Fig. 4: depicts the cloning schemes for different versions of PME-GFP fusions:
(A) cloning scheme for vectors plC2452 (full-length PME-GFP) and plC2462 (mature PME);
(B) cloning scheme of vectors plC2472 (GFP-full-length PME) and plC2482 (GFP - mature PME.
- Fig. 5: shows in vitro RRL (rabbit reticulocyte lysate) translation of four GFP fusions with the PME gene. Lane 1: pIC2482, lane 2: pIC2452, lane 3: pIC2472, and lane 4: pIC2462.
- Fig. 6: depcits the cloning scheme of different GFP-PME fusions in a plant expression cassette for transient expression experiments.
- Fig. 7: shows *N.benthamiana* cells expressing GFP (light areas) targeted into the cell wall after bombardment with a full length PME-GFP fusion under the control of 35S promoter.
- Fig. 8: depicts the T-DNA region of a binary vector carrying the flPME-GFP fusion.
- Fig. 9: depicts a T-DNA region of a binary vector carrying NtTLPR-GFP fusion.
- Fig. 10: depicts a T-DNA region of a binary vector carrying NtTLPRubq-GFP fusion.
- Fig. 11: depicts a crTMV vector carrying NtTLPR-GFP fusion.
- Fig. 12: depicts vectors T7/crTMV and SP6/crTMV.
- Fig. 13: depicts vectors T7/crTMV/IRES_{MP},₇₅^{CR} -GUS, T7/CrTMV/IRES_{MP,75}^{UI}-GUS, T7/crTMV/IRES_{MP,228}^{CR}-GUS, T7/crTMV/IRES_{CP,148}^{CR}-GUS , T7/crTMV/SPACER_{CP,148}^{UI-}GUS and T7/crTMV/PL-GUS.
- Fig. 14: depicts vectors pICH8600-C and pICH9666-C. IRESmp75^{CR} stands for the internal ribosome entry site element of the movement protein of a crucifer-infecting tobamovirus; see WO 02/29068 for details.
- Fig. 15: depicts T-DNA-based vectors plCH4851 that provides the 5'-end of the viral provector system, and plCP1010 that provides the site-specific integrase PhiC31 mediating recombination between attP (plCH4851) and attB (plCH9666-C, Fig 14) sites. Cloning of these vectors is described in PCT/EP02/03476 and in Fig. 17.
- Fig.16: depicts vectors pICH9666-RIA and pICH9666-RI designed to express the restriction endonuclease EcoRI.
- Fig. 17: depicts the structure of a PVX-based precursor vector, its generation by transcription (from plC3242) to give the complete form of the transcript and its splicing product. Expression can occur from the transcript (precursor vector and replicon) and the spliced transcript (replicon).
- Fig. 18: depicts basic constructs and cloning strategy used for cloning of the cDNA of a PVX-based precursor vector.
- Fig. 19: depicts the general scheme of expression of two genes (CP and GFP) using a CrTMV-based spliceable precursor vector (provector).
- Fig. 20: depicts the cloning strategy of intermediate construct plC3342.
- Fig. 21: depicts the cloning strategy of intermediate constructs plC3378 and plC3382.
- Fig. 22: depicts the final stages of cloning plasmid plC3393.
- Fig. 23: depicts the general scheme of expression of several genes via CrTMV-based precursor vectors (a-c and vector shown at the top) and generation of replicons (A-C) by site-specific recombination at LoxP-sites catalyzed by the enzyme Cre recombinase.
- Fig. 24: depicts the cloning scheme of construct pIC3461 - a primary component of a recombination system.
- Fig. 25: depicts the cloning scheme of construct pIC3441 - one of the secondary components of a recombination system
- Fig. 26: depicts the cloning scheme of construct plC3491 - one of the secondary components of a recombination system.
- Fig. 27: depicts the cloning scheme of construct plC3521 - one of the secondary components of a recombination system.
- Fig. 28: depicts the principal scheme of transgene expression via a noncontagious viral vector.
- Fig. 29: depicts the structure of plasmid pIC1593 used to introduce a Cre recombinase gene into the genome of *N. benthamiana.*
- Fig. 30: depicts the general scheme of expression of several genes via CrTMV-based precursor vectors (a-c and vector shown at the top) and generation of replicons (A-C) by using the integrase/att-system for site-specific recombination. The precursor vectors are cloned into binary vectors with T-DNA-borders (LB and RB).
- Fig. 31: depicts the cloning scheme of construct plCH4851- one of the secondary components of the recombination system.
- Fig. 32: depicts the cloning scheme of construct pICH5151- one of the secondary components of the recombination system.
- Fig. 33: depicts the cloning scheme of construct plCH5161- one of the secondary components of the recombination system.
- Fig. 34: depicts the cloning scheme of construct pICH5951- one of the secondary components of the recombination system.
- Fig. 35: depicts the cloning scheme of the constructs pICH6871 and pICH6891- two of the secondary components of the recombination system.
- Fig. 36: depicts the cloning scheme of construct pICH4371- one of the secondary components of the recombination system.
- Fig. 37: depicts the cloning scheme of plasmid plCH4461- one of the secondary components of the recombination system.

### DETAILED DESCRIPTION OF THE INVENTION

The general principle of the invention is shown in Figure 1. A gene encoding a protein fusion with a secretory signal peptide and a cell wall binding domain is delivered into the plant cell preferably using a DNA or an RNA vector. The recombinant protein is expressed, targeted to the intercellular space (apoplast) due to the presence of secretion signal peptide and bound to the cell wall through its cell wall binding domain. The plants with said fusion protein are then subjected to processing (squashing or homogenization) in order to separate the cell wall matrix from the cell content. The cell wall matrix can then be treated in a way that allows separation of the recombinant protein from said matrix.

Various methods can be used to deliver DNA or RNA vector into the plant cell, including direct introduction of said vector into a plant cell by means of microprojectile bombardment, electroporation or PEG-mediated treatment of protoplasts (for review see: Gelvin, S.B., 1998, *Curr. Opin. Biotechnol.,* 9, 227-232; Hansen & Wright, 1999, *Trends Plant Sci.,* 4, 226-231). Plant RNA and DNA viruses also present efficient delivery systems (Hayes *et al*., 1988, *Nature,* 334, 179-182; *Palmer et al., 1999, Arch. Virol.,* 144, 1345-1360; Lindbo *et al.,* 2001, *Curr. Opin. Plant. Biol*., 4, 181-185). Said vectors can deliver a transgene either for stable integration into the genome/plastome of the plant (direct or *Agrobacterium-mediated* DNA integration) or for transient expression of the transgene ("agroinfiltration").

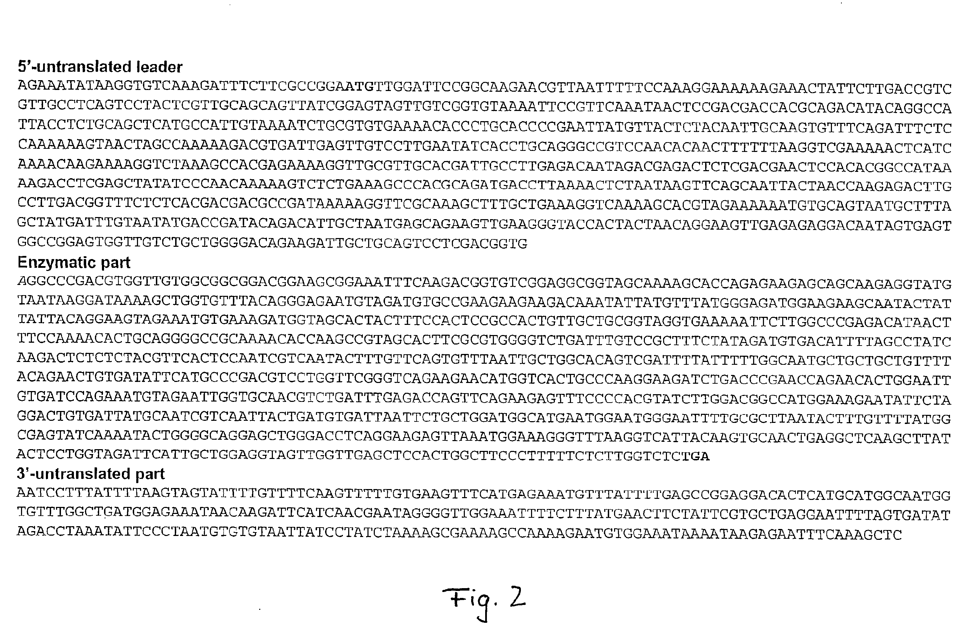

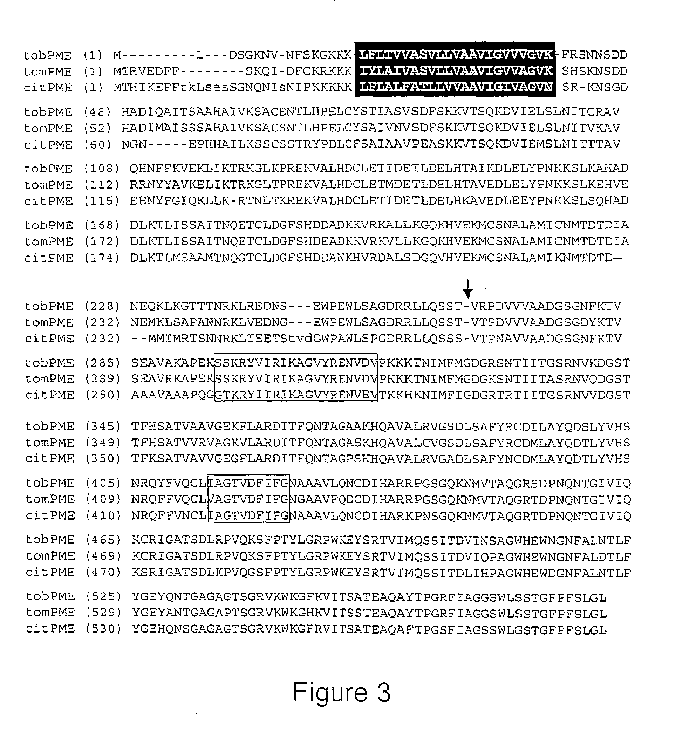

The nucleic acid having the coding region coding for the protein of interest with a secretory signal peptide and a cell wall binding domain can be designed in many different ways. Usually, the signal peptide is localized at the N-terminal end of the fusion protein. The cell wall binding domain(s) can link the signal peptide with the protein of interest or can be located at the C-terminal end of said fusion protein, or two cell wall binding domains may surround the protein of interest. In one embodiment of this invention, the whole tobacco pectin methylesterase (PME cDNA, see Fig. 2; PME protein precursors, see Fig. 3) gene was fused with a green fluorescent protein (GFP) gene as a protein of interest. Pectin methylesterase (PME) (Gaffe *et al.,* 1997, Plant Physiol.; 114, 1547-1556; Dorokhov *et al.,* 1999, *FEBS Lett.,* 461, 223-228) is a secretory protein, which can be detected in the endoplasmatic reticulum (ER) and the cell wall. The members of the PME multigene family that undergo post-translational processing (Gaffe *et al.,* 1997, Plant Physiol., 114, 1547-1556), are involved in cell wall turnover and appear to have a role in plant growth and development (Wen *et al.,* 1999, *Plant Cell,* 11, 1129-1140). PME is known to be a ubiquitous enzyme in the plant kingdom and regulates the cell wall degradation by catalyzing the demethoxylation of pectins. Mature 33 kDa PME is localized in the cell wall, while PME precursor is synthesized as 70 kDa polyprotein. It is obvious that PME processing and trafficking to cell wall is accompanied by the removal of the N-terminal leader sequence, whereas anchoring of PME in the cell wall requires the specific pectin binding domain.

In another embodiment of this invention (see examples 4 and 5) the fusion of a protein of interest was performed with the small (106 amino acid residues) cell wall protein NtTLPR from tobacco, possessing a signal peptide sequence and a cysteine domain (CD). The CD is involved in cross-linking the protein to the cell wall (Domingo et al., 1999, *Plant J.,* 20, 563-570).

The fusion protein of the invention or said protein or polypeptide of interest may further contain an affinity peptide tag. Said affinity peptide tag may be one of the following group: an oleosin protein or part thereof, an intein or part thereof, an additional cell wall binding domain, a starch binding domain, a streptavidin epitope-tag sequence, glutathione-S-transferase (GST) affinity tag, a 6xHis affinity tag, a calmodulin binding peptide (CBP) affinity tag. If said affinity peptide tag is an additional cell wall binding domain, it may be used for increasing the binding affinity and/or likelihood of binding of the fusion protein to the cell wall. If more than one polypeptide capable of binding to a cell wall component is used, they may bind to the same or to different cell wall components. Other affinity peptide tags may be used to provide the fusion protein or the protein of interest with useful properties, e.g. for (affinity) purification or for the use of the protein of interest.

The protein of interest can be fused with a signal sequence and cell wall binding domains from different genes, even from different organisms, for example an ER signal peptide of plant origin (e.g. tobacco calreticulin signal peptide) and a polisaccharide binding domain (PBD) of bacterial origin may be used in said fusion protein. Some plant pathogens (bacteria and fungi) are able to utilize polysaccharides such as cellulose, pectin, xylan and several other carbon sources for their growth and synthesize enzymes containing PBDs. The cellulose binding domains of said enzymes are well characterized and are extensively used for protein purification on a cellulose matrix. The affinity of a particular cellulose or polysaccharide binding domain to a respective cell wall component depends on the particular domain. Elution conditions of a particular fusion protein having a particular CBD or PBD are preferably established experimentally. CBDs usable for this invention are e.g. those listed in US6,174,700.

Considering that recombinant protein production in this invention includes the fusion of said protein of interest with a signaling peptide and cell wall binding domain(s), the precise separation of the protein of interest from the fusion protein can be an important issue. Vectors that include proteolytic sites flanking the coding sequence of interest will allow to cleave the fusion protein and release the protein of interest in a pure form, if the conditions are provided that allow for such proteolytic cleavage. Such cleavage will be of specific advantage, if the cell wall binding domain in the fusion protein shows strong binding and low recovery of fusion protein from the cell wall matrix. It is observed with cell wall binding domain of the NtTLPR protein. Such cleavage is of particular advantage, if the cell wall binding domain in the fusion protein shows strong binding, which may result in a low recovery of the fusion protein from the cell wall matrix. This is e.g. observed with the cell wall binding domain of NtTLPR protein

Cleavage of a translational fusion protein can be achieved via a peptide sequence recognized by a viral site-specific protease or via a catalytic peptide (Dolja *et al.,* 1992, *Proc. Natl. Acad. Sci. USA,* 89, 10208-10212; Gopinath *et al.,* 2000, *Virology,* 267, 159-173; US5162601; US5766885; US5491076). Other examples of site-specific proteases applicable to this invention are mammalian enterokinases, for example, human enterokinase light chain, which recognizes the sequence DDDK-I (Kitamoto et al., 1994, Prtoc. Natl. Acad. Sci., 91, 7588-7592), and specifically cleaves Lys-IIe bonds; viral proteases, like Hc-Pro (Carrington JC & Herndon KL,1992, Virology,187,308-315) which catalyses proteolysis between the Gly-Gly dipeptide but requires 4 amino acids for the recognition of the cleavage site; site-specific protease of Semliki Forest Virus (Vasiljeva et al., 2001, J. Biol. Chem., 276, 30786-30793); and proteases involved in polyubiquitin processing, ubiquitin-carboxy-terminal hydrolases (Osava et al., 2001, Biochem Biophys Res Commun., 283, 627-633).

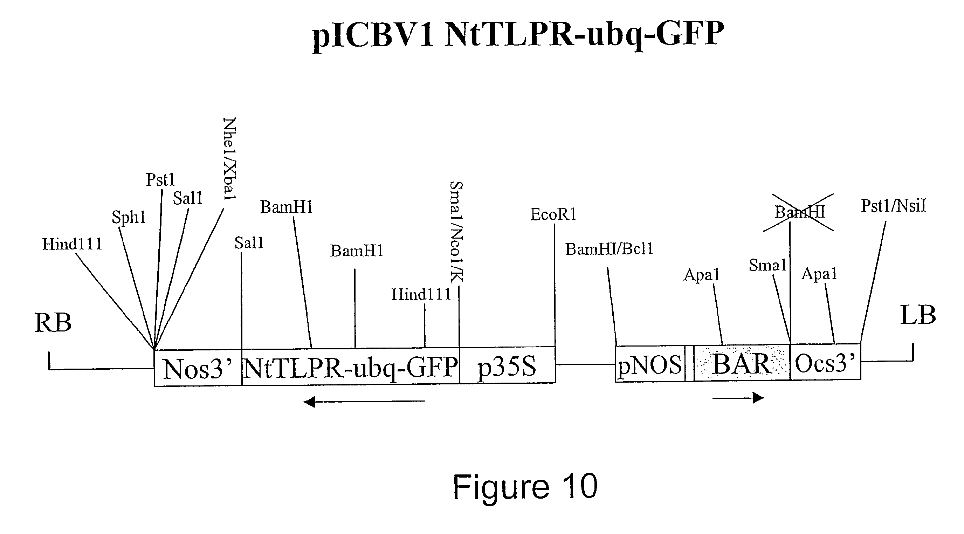

Ubiquitin-specific proteases open another opportunity. Expressing proteins and polypeptides as fusions to ubiquitin offers the advantage of an often-dramatic increase in expression level, and the ability to produce any desired amino-terminal residue upon ubiquitin cleavage. The recent availability of cloned ubiquitin-cleaving enzymes has enhanced this technique for both bacterial and eukaryotic host systems (for review see Baker, R.T., 1996, *Curr. Opin. Biotech.,* 7, 541-546). Ubiquitin fusion expression systems are already described for plants (US 5773705), yeasts (US 6068994) and bacteria (US 545905). In one embodiment of this invention, the ubiquitin polypeptide is inserted between the N-terminal NtTLPR protein and the GFP gene (Fig. 10, Example 4). This allows to have a naturally ocuring N-terminal end of any protein of interest after cleavage of the ubiquitin molecule by a ubiquitin-specific protease. The preciseness of such cleavage does not depend on the N-terminal amino acid sequence of the protein of interest.

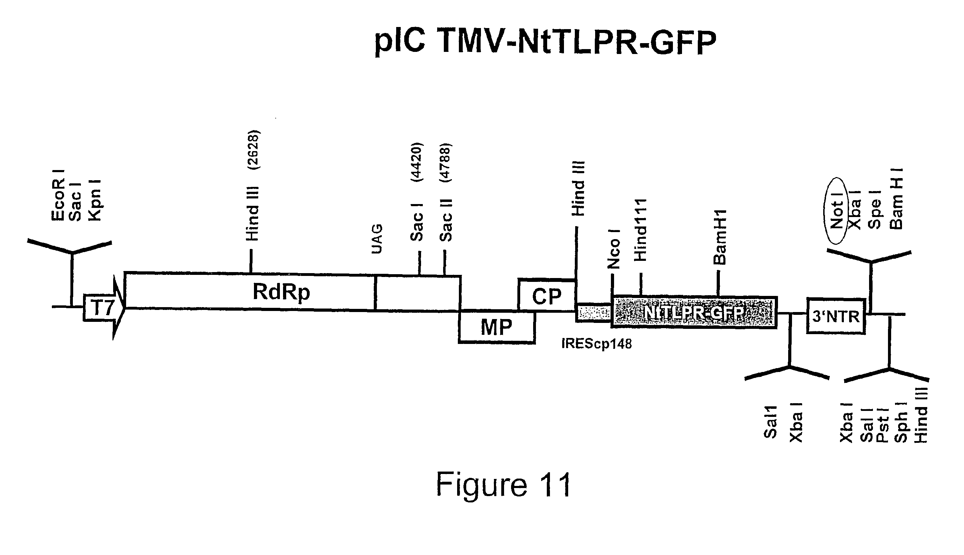

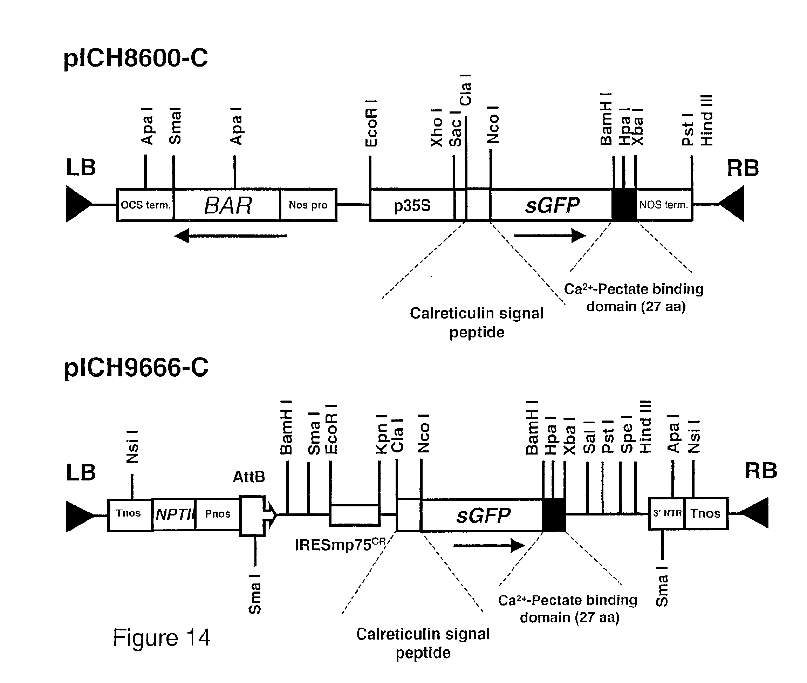

The gene of interest can be delivered into plants or plant cells using viral vectors. Such an approach has an obvious advantage over the constitutive expression of a transgene in stably transformed plants due to the significantly higher expression level and independence from cytotoxic effect, which might be caused by high level of transgenic product. The viral delivery system for this invention is described in Example 5 (Fig. 11) and 6 (Figures 14 to 16). The NtTLPR-GFP fusion is inserted into a crTMV expression vector. The apoplast targeted EcoRl endonuclease- Ca²⁺-pectate binding site fusion is inserted into the 3' end of a TMV-based provector (Example 6). The use of these systems in plants is described in the reference examples 1 to 3 and in WO 02/29068.

The genes of interest that can be expressed and isolated using our invention include, but are not limited to: starch modifying enzymes (starch synthase, starch phosphorylation enzyme, debranching enzyme, starch branching enzyme, starch branching enzyme II, granule bound starch synthase), sucrose phosphate synthase, sucrose phosphorylase, polygalacturonase, polyfructan sucrase, ADP glucose pyrophosphorylase, cyclodextrin glycosyltransferase, fructosyl transferase, glycogen synthase, pectin esterase, aprotinin, avidin, bacterial levansucrase, *E.coli* glgA protein, MAPK4 and orthologues, nitrogen assimilation/methabolism enzyme, glutamine synthase, plant osmotin, 2S albumin, thaumatin, site-specific recombinase/integrase (FLP, Cre, R recombinase, Int, SSVI Integrase R, Integrase phiC31, or an active fragment or variant thereof), isopentenyl transferase, Sca M5 (soybean calmodulin), coleopteran type toxin or an insecticidally active fragment, ubiquitin conjugating enzyme (E2) fusion proteins, enzymes that metabolise lipids, amino acids, sugars, nucleic acids and polysaccharides, superoxide dismutase, inactive proenzyme form of a protease, plant protein toxins, traits altering fiber in fiber producing plants, Coleopteran active toxin from *Bacillus thuringiensis* (Bt2 toxin, insecticidal crystal protein (ICP), CrylC toxin, delta endotoxin, polyopeptide toxin, protoxin etc.), insect specific toxin AalT, cellulose degrading enzymes, E1 cellulase from *Acidothermus celluloticus,* lignin modifying enzymes, cinnamoyl alcohol dehydrogenase, trehalose-6-phosphate synthase, enzymes of cytokinin metabolic pathway, HMG-CoA reductase, *E*. *coli* inorganic pyrophosphatase, seed storage protein, *Erwinia herbicola* lycopen synthase, ACC oxidase, pTOM36 encoded protein, phytase, ketohydrolase, acetoacetyl CoA reductase, PHB (polyhydroxybutanoate) synthase, acyl carrier protein, napin, EA9, non-higher plant phytoene synthase, pTOM5 encoded protein, ETR (ethylene receptor), plastidic pyruvate phosphate dikinase, nematode-inducible transmembrane pore protein, trait enhancing photosynthetic or plastid function of the plant cell, stilbene synthase, an enzyme capable of hydroxylating phenols, catechol dioxygenase, catechol 2,3-dioxygenase, chloromuconate cycloisomerase, anthranilate synthase, *Brassica* AGL15 protein, fructose 1,6-biphosphatase (FBPase), AMV RNA3, PVY replicase, PLRV replicase, potyvirus coat protein, CMV coat protein, TMV coat protein, luteovirus replicase, MDMV messenger RNA, mutant geminiviral replicase, *Umbellularia californica* C12:0 preferring acyl-ACP thioesterase, plant C10 or C12:0 preferring acyl-ACP thioesterase, C14:0 preferring acyl-ACP thioesterase (luxD), plant synthase factor A, plant synthase factor B, Δ6-desaturase, protein having an enzymatic activity in the peroxysomal β-oxidation of fatty acids in plant cells, acyl-CoA oxidase, 3-ketoacyl-CoA thiolase, lipase, maize acetyl-CoA-carboxylase, 5-enolpyruvylshikimate-3-phosphate synthase (EPSP), phosphinothricin acetyl transferase (BAR, PAT), CP4 protein, ACC deaminase, protein having posttranslational cleavage site, DHPS gene conferring sulfonamide resistance, bacterial nitrilase, 2,4-D monooxygenase, acetolactate synthase or acetohydroxyacid synthase (ALS, AHAS), polygalacturonase, Taq polymerase, bacterial nitrilase, many other enzymes of bacterial or phage including restriction endonucleases, methylases, DNA and RNA ligases, DNA and RNA polymerases, reverse trascryptases, nucleases (Dnases and RNAses), phosphatases, transferases etc.

Our invention also can be used for the purpose of molecular farming and purification of commercially valuable and pharmaceutically important proteins including industrial enzymes (cellulases, lipases, proteases, phytases etc.). Any human or animal protein can be expressed and purified using described in our invention approach. Examples of such proteins of interest include inter alia immune response proteins (monoclonal antibodies, single chain antibodies, T cell receptors etc.), antigens, colony stimulating factors, relaxins, polypeptide hormones including somatotropin (HGH) and proinsulin, cytokines and their receptors, interferons, growth factors and coagulation factors, enzymatically active lysosomal enzyme, fibrinolytic polypeptides, blood clotting factors, trypsinogen, α1-antitrypsin (AAT), human serum albumin, native cholera toxin B as well as function-conservative proteins like fusions, mutant versions and synthetic derivatives of the above proteins.

Host plants for practicing this invention may be monocotyledonous or dicotyledonous plants. The most preferred plants include *Nicotiana* species including *N. tabacum, Brassicacea* species including canola, potato, maize, wheat, oat, rice, sugar beet, soybean, Pennisetum, cotton, alfa-alfa, sunflower, hemp, pea, carrot, cucumber, tomato, etc. Practically, any plant species with established transformation and/or transfection protocol can be used in this invention.

### Producing a protein or polypeptide of interest using precursor vectors (Pro-vector system)

The present invention can easily be combined with other techniques of plant engineering. A preferred example of such a technique is the "Pro-vector system" WO 02/088369). Examples 6 and 7 demonstrate the combination of the present invention with precursor vectors (pro-vectors). Processes of causing amplification and/or expression of one or more nucleic acid sequences of interest in a plant using precursor vector(s), which may be combined with the present invention, are described in the following.

A process of causing amplification and/or expression of one or more nucleic acid sequences of interest in a plant, plant tissue, plant cell or cell culture, characterized in that a plant cell is provided with at least one precursor vector designed for undergoing processing in said cell, whereby due to said processing said plant cell is endowed with at least one replicon which provides for said amplification and/or expression, whereby said at least one replicon is preferably structurally related to each of said at least one precursor vectors owing to said processing.

A process of causing amplification and/or expression of one or more nucleic acid sequences of interest in a plant, plant tissue, plant cell or cell culture, characterized in that a plant cell is provided with at least one precursor vector designed for undergoing processing in said cell, whereby due to said processing said plant cell is endowed with at least two replicons which
(a) are structurally related to each other owing to said processing;
(b) are functionally distinct from each other; and
(c) provide for said amplification and/or expression.

A process of causing amplification and/or expression of one or more nucleic acid sequences of interest in a plant, plant tissue, plant cell or cell culture, characterized in that a plant cell is provided with at least two precursor vectors designed for undergoing processing in said cell preferably by site-specific recombination, whereby due to said processing said plant cell is endowed with at least one replicon which provides for said amplification and/or expression. Preferably, said at least one replicon is structurally related to each of said at least two precursor vectors owing to said processing.

Further a process is described for the production of a biochemical product, a process for gene function determination, and a process for artificial or directed evolution, whereby each of these processes comprises one of the above processes of causing amplification and/or expression.

Moreover, vectors or precursor vectors and viral material for this process are provided and viral material, replicons and plant material obtained or obtainable by performing this process. Viral material comprises nucleic acids capable of replicating in a plant cell. It comprises infectious DNA or RNA. Viral material may be naked or coated with a coat protein.

Further, a kit-of-parts comprising (i) plant cells, seeds or plants and (ii) the above vectors, precursor vectors, viral material, or replicons are provided. A further kit-of-parts is provided comprising (i) plant cells, seeds or plants and (ii) *Agrobacterium* cells containing the above vectors, precursor vectors, viral material, or replicons.

The pro-vector process causes amplification and/or expression of one or more nucleic acid sequences of interest in a plant cell. Amplification refers to the production of DNA or RNA (e.g. for anti-sense technology). Expression refers to the formation of a polypeptide or protein. In both cases, the ultimate goal may be a biochemical product the production of which may require amplification of said DNA or RNA and/or expression of a polypeptide or protein.

The pro-vector process may be carried out in a plant, plant tissue, plant cell or cell culture. It is preferred to carry out said process in plant cells. Most preferably, said process is carried out in a plant.

Providing a plant cell with a precursor vector may comprise viral transfection, *Agrobacterium*-mediated delivery, non-biological delivery, or conversion of a replicon pre-precursor DNA that was pre-integrated into a plant nuclear DNA to form a precursor vector or vectors. However, said providing a plant cell with a precursor vector may further comprise, in addition to transfection or transformation, cellular action, e.g. in the case of RNA virus-based precursor vectors, a primary transformed or transfected DNA may require transcription in order to produce the RNA precursor vector in the cell. In the case of *Agrobacterium-*mediated delivery, the precursor vector may have to be excised or transcribed from T-DNA delivered by *Agrobacterium.*

A replicon is a DNA or RNA molecule capable of autonomous replication in a cell of said plant. Examples include: bacterial and yeast plasmids, plastids and mitochondrial DNA, DNA and RNA viruses, viroids, phages. A replicon has to have an origin of replication. The origin of replication may be recognized by a DNA or RNA polymerase of the host plant cell, depending on whether the replicon is a DNA or an RNA replicon, or by a heterologous polymerase e.g. of viral origin. In this case, the plant cell has to be provided with said heterologous polymerase e.g. by one of said replicons. The autonomous replication of the replicons in the plant cell has presumably the effect that their concentration is increased, which may increase the expression level of the sequences of interest and support spread from cell-to-cell and throughout the plant. Preferably, replicons have an increased infectivity and ability to spread compared to precursor vectors.

Said at least one or said at least two replicons may retain additional viral capabilities such as viral particle assembly, infectivity, suppression of gene silencing, reverse transcription, integration into the host chromosome, cell to cell movement, or long distance movement. One of said replicons may essentially be a helper type virus which provides in trans functions necessary for replication of another replicon or replicons. Further, one of said replicons may essentially be a wild-type retrovirus or retrotransposon which provides in trans functions necessary for replication, reverse transcription, integration into a host chromosome of another replicon or replicons.

Said at least one or said at least two replicons provide, preferably together, for the amplification and/or expression of said nucleic acid sequences of interest. If one sequence of interest is amplified or expressed from one of said replicons another replicon may be required e.g. for a function necessary for the replication of said replicons or for spreading of at least one replicon to neighboring cells if said process is performed in cell culture or in a plant. In this case, the replicons cooperate functionally with each other.

If more than one sequence of interest is to be amplified or expressed, each sequence may preferably be expressed from one replicon, whereby the replicons provide (together) for said amplification or expression. Also in this case, the replicons preferably cooperate functionally with each other. As an example, a function for replication or spreading of said replicons may be expressed from one or some of said replicons which amplify or express said sequences of interest or from an additional replicon. Without functional cooperation, the amplification or expression level would be much lower or be limited to the cell(s) provided with said precursor vector(s), if amplification or expression is desired in plant cells or a plant.

Said at least one or said at least two replicons are functionally distinct from each other in that they provide different functions for amplification and/or expression of said sequence(s) of interest. Examples for such functions include, in addition to coding of said nucleic acid sequence(s) of interest, the expression of products necessary for replicating the replicons, expression of factors or products (preferably enzymes) which facilitate the processing of the precursor vector(s) to give said replicons, expression of products necessary for cell to cell or long distance or plant to plant movement of said replicons (e.g. movement protein or coat protein) etc. These products may function in trans or in cis. Functional distinctness does not include random mutations in the replicons which may occur in the course of the processing in the plant cell.

Owing to said processing, said at least replicons are structurally related to each other in that they share sequence portions with each other. The type of the relatedness depends on the type of processing (modification process). If one replicon is produced in said process, said replicon is structurally related to said at least one or to each of said at least two precursor vectors owing to said processing.

The precursor vectors undergo processing in the plant cell by one of the following DNA or RNA modification processes, which endows the plant cell with said replicon or replicons. The processing in the plant cell may involve DNA modification such as DNA recombination, insertion or excision etc. Alternatively, it may involve RNA modification such as RNA splicing, ligation or recombination etc. Said processing does not include transcription. The precursor vector may itself be a DNA or RNA molecule capable of autonomous replication in a cell of said plant.

The precursor vector(s) are preferably of plant viral origin, more preferably of an RNA virus or DNA virus origin. Examples of specific viruses are given below. Precursor vectors may be capable of autonomous replication in the plant cell as are replicons.

The plant cell may be provided with one or more precursor vectors. If the cell is provided with only one precursor vector, this precursor endows the plant cell with at least two replicons. If the cell is provided with two or more precursor vectors, the cell is preferably endowed with said at least one or said at least two replicons by a processing which involves interaction between said precursor vectors, e.g. recombination. Providing the plant cell with two or more precursor vectors greatly increases the possibilities for generating said replicon or replicons. Several different DNA or RNA modifications may occur in the plant cell depending on the design of the precursor vectors.

This process is based on the use of a wild-type plant cell(s) or on a plant cell(s) that is (are) genetically engineered so as to provide functions necessary for said processing, or to provide in trans one or more functions necessary for infectivity, replicon replication, virus particle assembly, suppression of gene silencing by the host, integration into a host chromosome, reverse transcription, cell to cell or long distance movement of said resultant replicons. Said genetic engineering of said plant cell is done by transient expression, virus- or *Agrobacterium*-mediated transfection, stable integration into genomes of nuclei or organelles or into autonomously replicating plasmids of said plant cell. The plant cells and plants for the process described here are preferably higher plants or cells thereof. Crop plants are particularly preferred.

The process of causing amplification or expression features several important advantages over the prior art. The size of the nucleic acid sequence(s) of interest to be amplified or expressed is far less limited than in the prior art, since functions necessary for amplification or expression are shared by at least two replicons, whereby the replicons are smaller than a prior art vector would be. Consequently, amplification or expression is more efficient.

Furthermore, this process allows the amplification and/or expression of more than one nucleic acid sequence of interest. Examples are provided for the expression of two genes of interest. However, the expression of three, four or even more nucleic acid sequence of interest is also feasible. This allows the expression of a whole biochemical pathway or cascade or of a multi-subunit protein. Each sequence of interest may preferably be expressed from one replicon. Additional function(s) for efficient performance of the process like those listed below may be located on an additional replicon. Alternatively, a replicon may encode more than one of these functions.

A third important advantage is that there are several possibilities of improving the biological safety over prior art processes. In embodiments wherein more than one precursor vector is employed, the process is only functional when all precursors get into the plant cell. Alternatively, the processing in the cell to generate said at least one or said at least two replicons may be dependent on an additional component e.g. an enzyme which catalyses said processing. Then the system is only functional if the additional component is delivered into the cell or if a transgenic cell expressing said component is used. In one embodiment, the replicon which expresses a sequence of interest can spread throughout the plant from the primary infected cell, but spreading to other plants is not possible.

Examples for nucleic acid sequences of interest include coding sequences or parts thereof, or any genetic element. Herein, a genetic element is any DNA or RNA element that has a distinct genetic function other than coding for a structural part of a gene. Examples include: transcriptional enhancer, promoters, translational enhancers, recombination sites, transcriptional termination sequences, internal ribosome entry sites (IRESes), restriction sites. A preferred genetic element is a tobamoviral IRESₘₚ75 used as translational enhancer operably linked to a heterologous nucleic acid sequence encoding a protein of interst.

In a preferred embodiment, said plant cell is endowed with at least one (type of) replicon. Said one replicon is preferably formed by site-specific recombination between at least two precursor vectors. Said site-specific recombination preferably takes place on DNA level. Said one replicon may therefore be DNA. Alternatively, said one replicon may be RNA formed by transcription following said site-specific recombination. In this embodiment, said precursor vectors are preferably not capable of autonomous replication in said plant cell. This embodiment is particularly preferred from the point of view of biological safety.

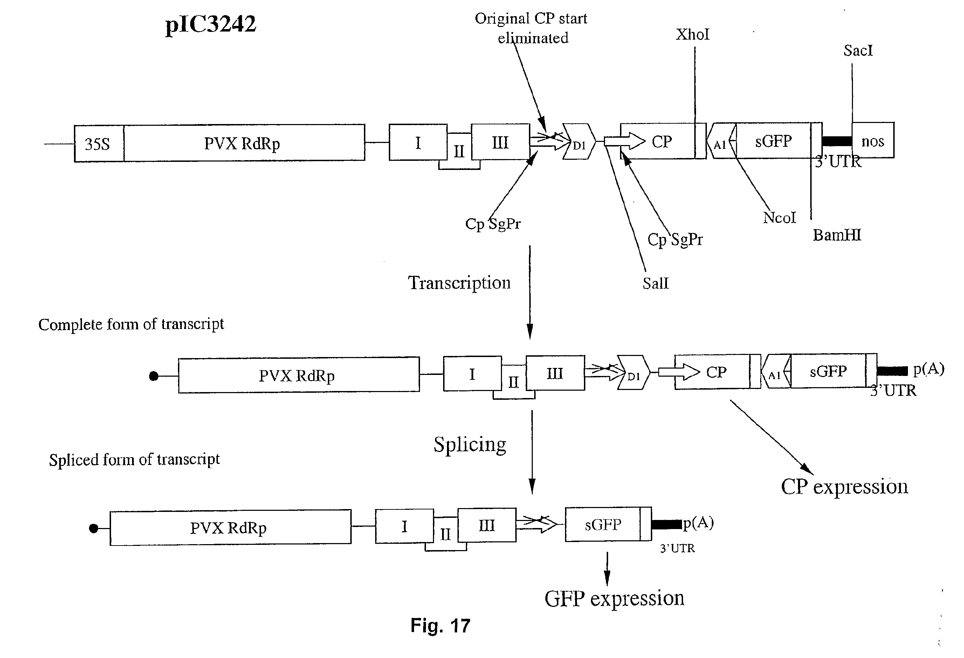

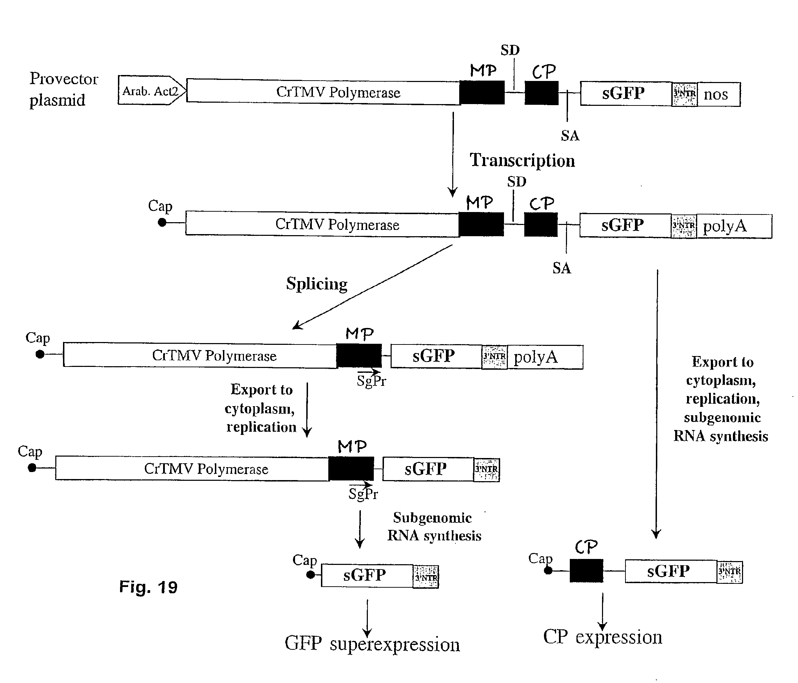

In another preferred embodiment, a plant cell is provided with the cDNA of a precursor vector (Figs. 17 and 19). After transcription which produces the precursor vector, processing in the cell by splicing endows the cell with an RNA virus-based replicon from which a sequence of interest can be amplified or expressed. Since splicing is slow and/or does not happen in all the precursor vector molecules in the cell, unspliced precursor molecules will remain in the cell. These remaining unspliced precursor vectors are replicons as well provided they are capable of autonomous replication. They are used for the expression of (a) function(s) necessary for amplification or expression of said sequence of interest, e.g. a function for spreading of the replicon(s) to neighbor cells.

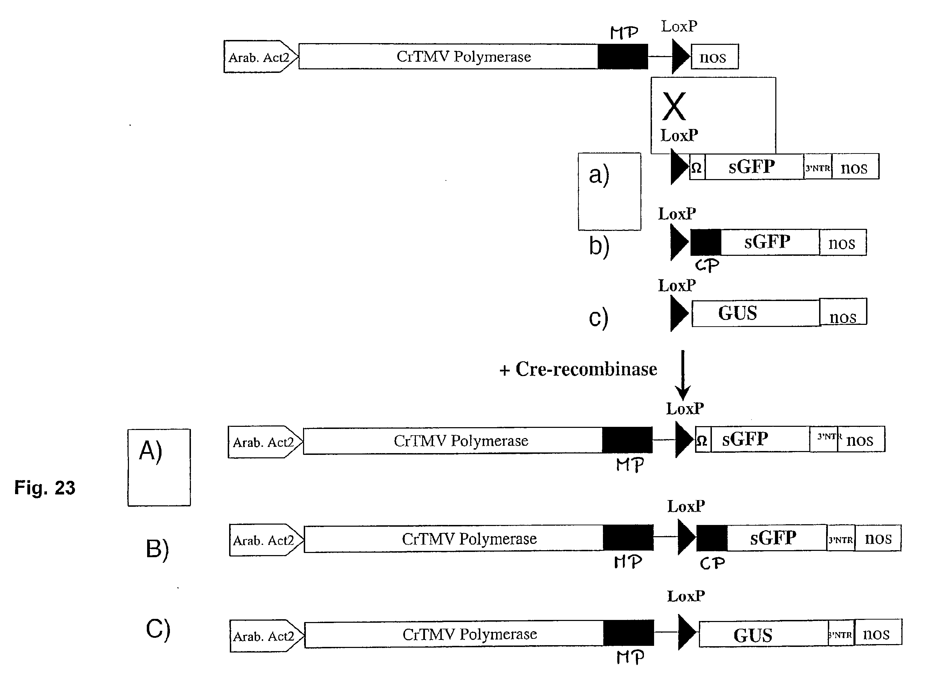

In another preferred embodiment, a set of replicons of the type AB₁, AB₂, ... , ABₙ or of the type B₁A, B₂A, ... , BₙA are generated in a cell by site-specific recombination of a primary precursor vector (A) with a set of at least two secondary precursor vectors (B₁, B₂, ... , Bₙ), wherein n is an integer of ≥2. In the embodiment shown in Fig. 23, precursor vectors (secondary vectors) containing a sequence to be expressed or amplified are recombined with another precursor vector (primary precursor vector) to form replicons of the type AB₁, AB₂ and AB₃, from which these sequences can be amplified or expressed. At least three precursor vectors are needed to endow the cell with at least two replicons. Preferably, functions for spreading the replicons are expressed from one or more of said replicons.

For performing the above processes, precursor vectors may directly be used for transforming or transfecting a plant or plant cell. This is particularly true for DNA virus-based replicons. For RNA virus-based replicons, DNA vectors used for transformation or transfection require transcription for producing the precursor vectors inside the cell.

The processes described here may be used for generating high amounts of one or more nucleic acid sequences in a plant, plant tissue, plant cell or cell culture in an environmentally safe way. Notably, said processes may be used for generating high amounts of said at least one or said at least two replicons. Said replicon(s) may be purified or enriched from the plant material. Said replicon(s) may be vectors or viral material that may be used, optionally after enrichment or purification, for transforming or transfecting a further plant, plant tissue, plant cells or cell culture. In this embodiment, the subject processes may be biologically safe processes of producing infectious viral material or vectors for transforming or transfecting plants on a large scale like on an agricultural or farming scale. Said infectious viral material or said vectors may be packaged or coated viral material. The protein material necessary for packaging said viral material may be expressed from said replicon(s).

### Detailed description of Pro-vector system

Viruses belonging to different taxonomic groups can be used for the construction of virus-based vectors according to the principles of the pro-vector system. This is true for both RNA- and DNA-containing viruses, examples for which are given in WO 02/088369.

Mostly, vectors of plant viral origin are used as plasmids capable of autonomous replication in plants (replicons). However the principles necessary for engineering such plasmids using non-viral elements are known. For example, many putative origins of replication from plant cells have been described (Berlani *et al.,* 1988, *Plant Mol. Biol.,* 11, 161-162; Hernandes *et al.,* 1988, *Plant Mol. Biol.,* 10, 413-422; Berlani *et al.,* 1988, *Plant Mol. Biol.,* 11, 173-182; Eckdahl *et al.,* 1989, *Plant Mol. Biol.,* 12, 507-516). It has been shown that the autonomously replicating sequences (ARS elements) from genomes of higher plants have structural and sequence features in common with ARS elements from yeast and higher animals (Eckdahl *et al.,* 1989, *Plant Mol. Biol.,* 12, 507-516). Plant ARS elements are capable of conferring autonomous replicating ability to plasmids in *Saccharomyces cerevisiae.* Studies of maize nuclear DNA sequences capable of promoting autonomous replication of plasmids in yeast showed that they represent two families of highly repeated sequences within the maize genome. Those sequences have a characteristic genomic hybridization pattern. Typically there was only one copy of an ARS-homologous sequence on each 12-15 kb of genomic fragment (Berlani *et al*., 1988, *Plant Mol. Biol.,* 11:161-162). Another source of replicons of plant origin are plant ribosomal DNA spacer elements that can stimulate the amplification and expression of heterologous genes in plants (Borisjuk et al., 2000, *Nature Biotech.,* 18, 1303-1306).

Therefore, a replicon or precursor vector contemplated here is not necessarily derived from a plant virus. Plant DNA viruses provide an easy way of engineering replicons (vectors) that could be especially useful for targeted DNA transformation, but vectors made entirely or partially of elements from plant RNA viruses or even non-plant viruses are possible. Plant virus-based replicons are evidently advantageous. Such replicons, in addition to replication, may provide additional useful functions e.g. for cell to cell and long distance movement. Further, they can frequently be removed more easily from the plant cell *aposteriori* by using known methods of virus eradication from infected plants.

In the simplest case, one type of precursor vector (pro-vector) (A) is delivered into the plant cell where, upon its processing in said cell, it yields at least one or at least two structurally related and functionally distinct replicons (F and G) which are able to provide amplification and/or expression of the sequences of interest. More than one precursor vector can be introduced into the plant cell [A(+C,D,E...)]. Optionally, said plant cell can be transgenic and contain another additional component (B) required for the processing of the precursor vector and/or expression, replication, cell-to-cell or systemic movement.

In one embodiment, we describe a pro-vector (precursor vector) system that is based on the splicing of the pro-vector RNA in the plant nucleus. This system comprises a DNA molecule of the pro-vector containing parts of plant virus genome cDNA, gene(s) of interest and splicing-sites functional in the plant cell (see for example Figures 17 or 19). After transcription in the transfected plant cell, the primary RNA product (pro-vector or precursor vector) can be processed by splicing. Due to the design of the pro-vector (precursor vector), the gene of interest (e.g. GFP) substitutes one of the viral genes (e.g. CP) in this spliced form. This allows the gene of interest (GFP) to be expressed via the normal viral pathway instead of substituted viral protein. However, since splicing cannot proceed with 100% efficiency, a fraction of the primary transcript remains unspliced and is exported from the nucleus as is the spliced form. As a result, two types of self-replicating viral vector RNAs (replicons) appear in the cytoplasm of the transfected plant cell. This leads to the expression of both GFP and CP from replicons generated using one precursor vector. It must be mentioned that, in the exemplified case, the level of GFP expression is likely much higher than that of CP. It was shown for tobamoviruses that the amount of viral protein produced during infection depends on the distance between gene encoding the protein and 3'-terminus of the virus. Since GFP is expressed from the spliced form of RNA, the corresponding subgenomic RNA is significantly shorter than the RNA from which CP is expressed (Figure 19; sGFP stands for a synthetic or engineered GFP).

As an exemplification of this embodiment we constructed two pro-vectors (precursor vectors) based on two well-known plant viruses: potato virus X (PVX) and crucifer infecting tobamovirus (CrTMV). In both cases, the CP gene of the viruses was flanked with donor (upstream) and acceptor (downstream) splicing sites (SA and SD, respectively). GFP was cloned downstream of the acceptor site to be expressed only in the spliced transcript. The physiological roles of CP in PVX and CrTMV are different. In the case of PVX, CP is required for cell-to-cell movement of the virus. In CrTMV, CP participates mainly in long-distance spread of the virus (systemic infection) and is not crucial for infection of neighbor cells. These examples provide two different patterns of reporter gene (GFP) expression. In the case of the PVX-based system, viral spread is stalled in primary transfected cells until the required amount of CP is expressed from a less efficient replicon that was formed from unspliced RNA. This leads to super-production of much more rapidly synthesized GFP in the same cell. Finally, the necessary amount of CP accumulates and both replicons penetrate neighbor cells where the process repeats. On the contrary, in the case of the CrTMV-based pro-vector, viral spread is not limited to cell-to-cell movement. Both forms of the vector act independently, which leads to faster growth of the infected area.

Although specific examples describing RNA modification as a mechanism for generating replicons are based on RNA splicing, other RNA modification mechanisms may be used as well. These include inter alia modifications as RNA ligation or RNA recombination. Ligation of different RNA molecules by enzymes such as RNA ligase allows to produce a plurality of different RNA replicons within a cell based on the internal enzymatic activity of plant cells or based on the expression of well known ligases such as T4 RNA ligase (Nishigaki *et al.,* 1998, *Mol. Divers., 4,* 187-190) or mitochondrial RNA ligase from *Leishmania* (Blanc *et al.,* 1999, J. *Biol. Chem.,* 274, 24289-24296). RNA-RNA recombination is a well researched phenomenon. It readily occurs in a plant host between viral RNA molecules with a certain degree of homology (Allison *et al.,* 1990, *Proc. Natl. Acad. Sci.* 87, 1820-1824; Rao *et al.,* 1990, J. *Gen. Virol.,* 71, 1403-1407; US 5,877,401).

In another embodiment, the precursor vectors are processed by site-specific DNA recombination producing a replicon or partially different replicons or assembling one viral replicon *in vivo.* In this case, molecular rearrangements proceed on the DNA level. Several molecules (pro-vectors) are shuffled by means of recombination to produce one or several vector molecules (replicons). The first DNA-component of the system may contain a plant promoter (Arabidopsis Actin 2) fused to the main part of CrTMV genome - the polymerase gene plus the MP gene followed by a specific recombination site. Secondary components are plasmid DNAs comprising the same recombination site followed by a gene(s) of interest (any reporter gene) or viral CP gene. The 3'-UTR of CrTMV should be cloned downstream of the gene of interest. The last component of the system may be Cre-recombinase or integrase expressing DNA. These enzymes promote reorganization of pro-vector components into active vector molecules (replicons). It must be stressed that none of the pro-vector components is infectious alone, which is important in terms of the biological safety of the system.

After providing a plant cell with all the components of the described multi-component system, recombination occurs and one or several replicons can be formed (see Figures 23 A,B,C). These rearranged DNA molecules can be transcribed (since they carry Arabidopsis Actin 2 promoter) in the nucleus and exported to the cytoplasm. Finally, like in the case involving RNA splicing, several replicating vector RNAs appear in the cytoplasm of the transfected cell. In this embodiment, we describe the system where all of these vectors contain a functional MP gene and a gene positioned downstream. This allows each vector RNA to express both a functional MP gene and a gene positioned downstream and to penetrate into neighbor cells. One has to state that other combinations are also possible.

Two different approaches of recombinase delivery are exemplified. The recombinase may be delivered into the cell in a process of co-bombardment or by *Agrobacterium-*mediated transient expression together with other DNA-components of the system. As another approach, a transgenic plant expressing cre-recombinase has been obtained. This reduces the number of components the cell has to be provided with and as a result raises the overall efficiency of the system. Additionally, this further improves the safety of the process as it cannot occur outside of a plant that was genetically manipulated to support the process.

During the cloning of pro-vector components, a LoxP recombination site was cloned upstream of the gene(s) of interest. A LoxP site contains two small inverted repeats spaced by several nucleotides and it forms stem-and-loop structure in RNA. The stem contains only 4 GC pairs so it is not very stable. However, this stem can reduce the efficiency of translation of a downstream gene. To solve this problem, any translational enhancer can be cloned between LoxP and the gene of interest. In the examples with Cre recombinase we used an omega leader of TMV U1. However any other translation-enhancing sequence can be used (e.g. IRESₘₚ75 from CrTMV or TMV U1) as well as plant IRESes. IRESₘₚ75 elements may preferably be used as translational enhancers as in the examples with integrase of phage PhiC31. In this case, in a set of provectors the LoxP recombination sites were replaced by att-sites (attachment-sites) from the *Streptomyces* phage PhiC31 (Thorpe & Smith, 1998, *Proc. Natl. Acad. Sci.,* 95, 5505-5510; Groth *et al.,* 2000, *Proc. Natl. Acad. Sci.,* 97, 5995-6000) which are the target sequences for the recombination enzyme integrase. Two different att-sites were cloned into the provectors: Whereas attP was inserted in the vectors of the type polymerase-MP-LoxP, an attB-recombination site was cloned into the provectors, which carry the gene of interest followed by a 3'NTR sequence and a nos-terminator. Similar to the Cre system, the att-recombination-sites limit the efficiency of translation of a gene, which is located downstream. Hence, also in this system translational enhancer sequences were cloned between the attB-sites and the genes of interest. It was shown that IRESₘₚ75 sequences have comparable or even better effect as translational enhancers in comparison with the omega leader of TMV U1.

Suitable recombinases/recombination site systems include *inter alia* the Cre-Lox system from bacteriophage P1 (Austin *et al.,* 1981, *Cell,* 25, 729-736), the Flp-Frt system from *Saccharomyces cerevisiae* (Broach *et al.,* 1982, *Cell,* 29, 227-234), the R-Rs system from *Zygosaccharomyces rouxii* (Araki *et al.,* 1985, *J. Mol. Biol.,* 182, 191-203), the integrase from the *Streptomyces* phage PhiC31 (Thorpe & Smith, 1998, *Proc. Natl. Acad. Sci.,* 95, 5505-5510; Groth *et al.,* 2000, *Proc. Natl. Acad. Sci.,* 97, 5995-6000), and resolvases. In addition, other methods of DNA rearrangement are contemplated. Other DNA modification enzyme systems can all be used to generate related but functionally distinct replicons inside of a wild-type or a genetically engineered plant cell: restriction endonuclease, transposase, general or specific recombinase, etc.

Different methods may be used for providing a plant cell with precursor vectors. DNA may be transformed into plant cells by a Ti-plasmid vector carried by *Agrobacterium* (US 5,591,616; US 4,940,838; US 5,464,763) or particle or microprojectile bombardment (US 05100792; EP 00444882B1; EP 00434616B1). Other plant transformation methods can also be used like microinjection (WO 09209696; WO 09400583A1; EP 175966B1), electroporation (EP00564595B1; EP00290395B1; WO 08706614A1) or PEG-mediated transformation of protoplasts etc. The choice of the transformation method depends on the plant species to be transformed. For example, microprojectile bombardment is generally preferred for monocot transformation, while for dicots, *Agrobacterium*-mediated transformation gives better results in general. *Agrobacterium*-mediated delivery of precursor vectors is preferred.

Construction of plant viral vectors for the expression of non-viral genes in plants has been described in several papers (Dawson *et al.,* 1989, *Virology,* 172, 285-293; Brisson *et al.,* 1986, *Methods in Enzymology,* 118, 659; MacFarlane & Popovich, 2000, *Virology,* 267, 29-35; Gopinath *et al.,* 2000, *Virology,* 267, 159-173; *Voinnet et al.,* 1999, *Proc. Natl. Acad. Sci. USA,* 96, 14147-14152) and can be easily performed by those skilled in the art.

The current processes have a number of advantages compared to existing viral vector-based strategies to express foreign genes in plants.

Most importantly, the process can be used for the expression of more than one nucleic acid sequence of interest and can thus be used for the expression of multiple genes of a biochemical pathway or cascade. In this regard, the processes described here are the only available method that can be effectively used for the expression of genes for the purpose of gene function determination or for the purpose of biochemical production.

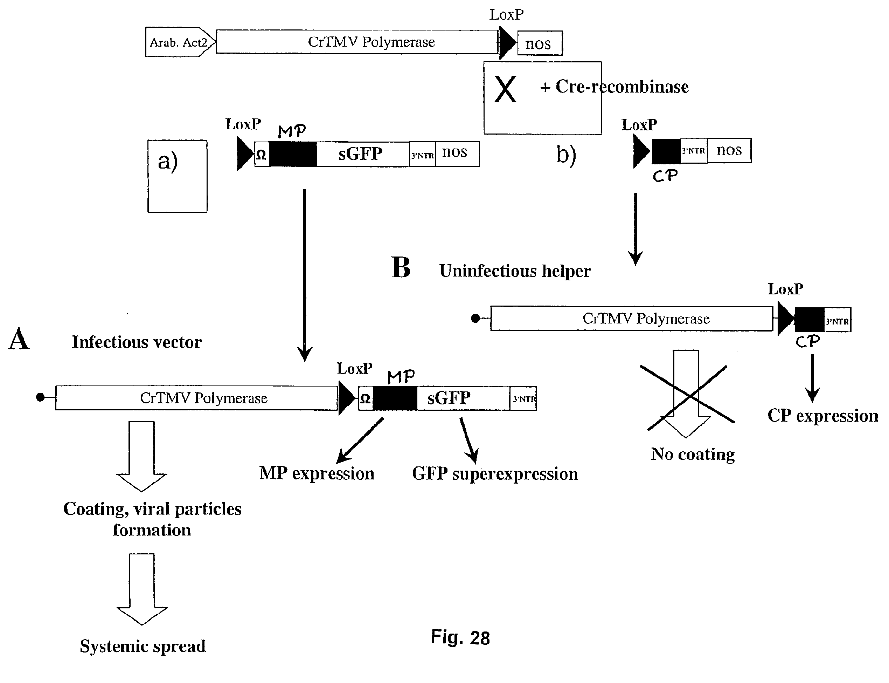

The invention also opens up a wide range of opportunities for any kind of biological cascade construction. One example is presented in Fig. 28. This system utilizes recombination of 3 precursor vector components into 2 replicons. First, replicon A expresses MP and a gene of interest (GFP in the example). Due to the expression of the movement protein (MP), this vector is able to move from cell-to-cell in the inoculated leaf. However it cannot spread systemically in the infected plant and cannot be transmitted to other plants due to the absence of the coat protein (CP). In other words, this replicon is infectious only if it is artificially delivered into a plant cell. The second replicon B expresses only CP. Note that it is not able to form viral particles since its RNA lacks the origin of virus assembly (positioned inside the MP gene). However it does express CP in significant amounts.

The proposed process is inherently more safe as it is operable only in the presence of all the precursor vectors. If both of the replicons described above are present in the same cell, they complement each other and both components are able to move to neighbor cells. However only vector A can be coated with CP to form viral particles and therefore only this component will be exported from the infected leaf into the whole plant. If such viral particles penetrate uninfected leaves, they deliver only the infectious component A, but not B. This leads to the systemic spread of infection in the whole plant but the virus cannot infect other plants because viral particles can be formed only in primary inoculated leaves and these particles contain only one replicon component, which is not enough for systemic infection of another plant. This system represents a unique example of highly efficient expression of a transgene in the whole plant via a noncontagious viral vector.

Additionally, the process of assembly of one viral replicon from at least two replicon precursors through site-specific DNA recombination guaranties a higher level of safety in comparison with convenient viral vector being used for infecting plant cells. Said process of viral replicon assembly from at least two components requires the presence of said two components and a site-specific recombinase to be present in the same plant cell. Said recombinase can be delivered in cis together with one of said components. Preferably, the recombinase can be delivered separately. More preferably, the host plant can be engineered to expresses said recombinase. In the latter case, assembly of functional vector from provector elements will be restricted specifically to engineered host plant.

The pro-vector system is exemplified in reference examples 4 to 17.

### EXAMPLES

### EXAMPLE 1

### Construction of vectors carrying different types of GFP fusions with the tobacco pectin methylesterase (PME) gene for transient expression and stable nuclear transformation of plants

A red-shifted mutant of GFP (GFPst65T) was chosen for fusion with PME to determine the location of GFP *in vivo.* The following four constructs were created: 1) full length PME-GFP; 2) GFP-mature PME; 3) GFP- full length PME and 4) mature PME -GFP. The first construct was designed for targeting the protein of interest (GFP) to the cell wall. Constructs 2-4 were used as negative controls.

Plasmid pUC8 containing a full sequence of an unprocessed PME gene with untranslated 5' and 3'-regions was used for PCR amplification with the following primers:
a) 5'-TGACCCATGGTGGATTCCGGCAAGAACGTT-3', corresponding to the N-terminal part of the PME coding sequence and containing a Ncol site.
b) 5'-TTTTGGATCCACGGAGACCAAGAGAAAAAG-3', corresponding the C-terminal part of the PME coding sequence and containing a BamHl site.
   This pair of primers was designed for generating PME without a translation termination signal.
c) 5'-TGACGGATCCTTGGATTCCGGCAAGAACGTTAAT-3', corresponding to the N-terminal part of the coding sequence of full length PME and containing a BamHl site.
d) 5'-CCCCTCTAGATCAGAGACCAAGAGAAAAAGGGAA-3', corresponding to the C-terminal part of full length PME and containing a Xbal site.
   This pair of primers was designed for creating PME without the first methionine residue but with a translation termination signal.
e) 5'-TTTTCCATGGTGAGGCCCGACGTGGTTGTGGC-3' corresponding to the N-terminal part of the mature PME gene containing a translation start codon in the Ncol site.
   This primer was designed for creating a mature PME gene with a translation start but without a translation termination signal.
f) 5'-ATAAGGATCCGTGAGGCCCGACGTGGTTGTGGC-3', corresponding to the N-terminal part of the mature PME gene without translation termination signal within the BamHl site. This primer was designed for creating mature PME gene without start codon but with a translation termination signal.

Different types of PCR products were cloned into pGEM-T vector (Promega) under the control of the T7 promoter, resulting in plasmids plC2406 {primers a) and b)}; plC2396 {primers b) and c)}; pIC2425 {primers a) and f)} and pIC2415 {primers b) and f)}. Then the GFP gene was modified for N- or C-terminal translational fusion with PME. Two modifications of the GFP gene were cloned into the pGEM-T vector under the control of the T7 promoter. GFP with a translation start codon and without a translation termination signal was obtained using primers g) and h) (pIC2441). GFP without a translation start codon but with a translation termination signal was obtained using primers I) and j) (pIC2431).

Used GFP primers:
g) 5'-TTTTCCATGGTGAGCAAGGGCGAGGAGCTG-3' corresponding to the N-terminal part of the GFP gene and containing a translation start codon within the Ncol site.
h) 5'-TTTTGGATCCTATTCTTGCGGGCCCTCGCTTGTACAGCTCGTCCATGCC-3' corresponding to the C-terminal end of the GFP gene and containing BamHl restriction site.
i) 5'- TTTTGGATCCATAAGAACGGGGCCCAGAGTGAGCAAGGGCGAGGAGCTGT-3', corresponding to the N-terminal part of the GFP gene and containing a BamHl site.
j) 5'-TTTTTCTAGATTACTTGTACAGCTCGTCCA-3', corresponding to the C-terminal part of the GFP gene and containing a translation termination signal within Xbal site.

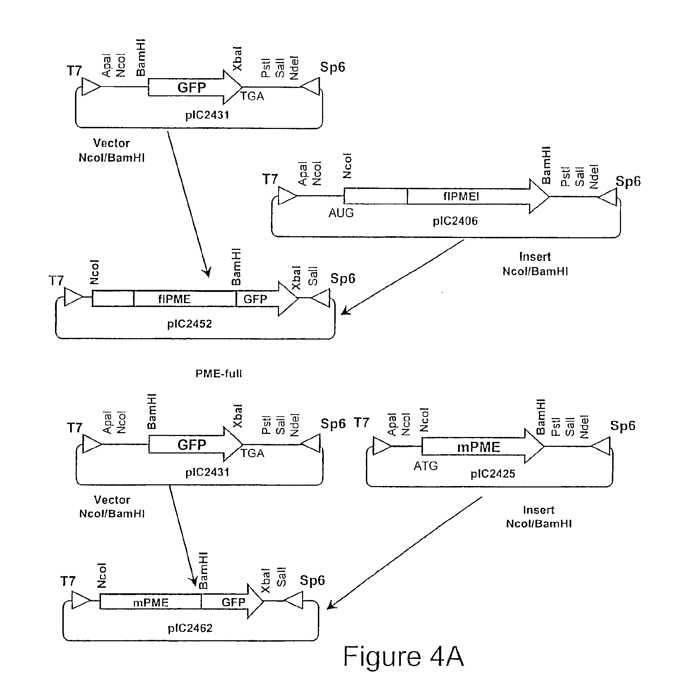

For PME - GFP fusions, the Ncol/BamHl fragment of plC2431 containing GFP without translation start codon was ligated with the large Ncol/BamHl fragments of plC2406 and plC2425, yielding constructs plC2452 and plC2462, respectively. The general scheme of the cloning procedures in shown in Fig. 4A.

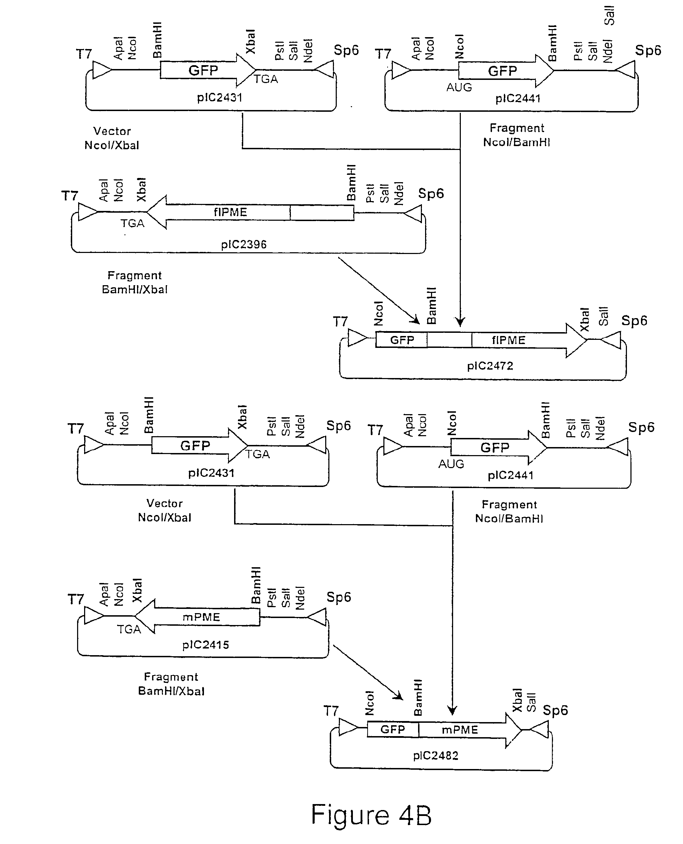

For GFP-PME fusions, the small Ncol/BamHl fragment of plC2441 was ligated with the large Ncol/BamH1 fragment of plC2396 or with the large Nco1/BamH1 fragment of plC2411 resulting in plasmids plC2472 and plC2482, respectively. The general scheme of the cloning procedures in shown in Fig. 4B.

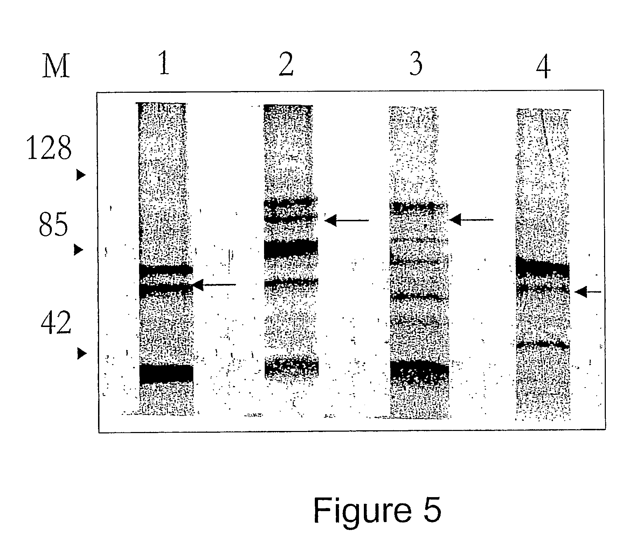

In order to check the correctness of the PME-GFP fusions, the constructs pIC 2452, 2462, 2472, and 2482 were used for *in vitro* translation in rabbit reticulocyte lysate (RRL). The results presented in Fig. 5 show the intactness of different PME-GFP fusion products.

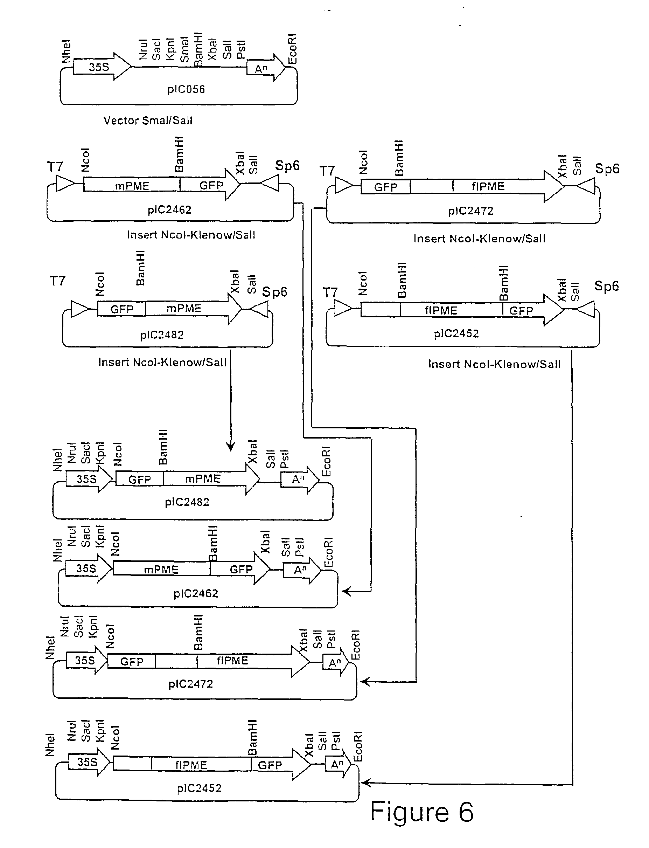

For transient expression experiments, the large Ncol-Klenow/Sall fragments of plC 2452, 2462, 2472 and 2482 were ligated with the Smal/Sall large fragment of plC056 expression cassete between the 35S promoter and the nos terminator sequences, yielding constructs carrying p35S:full length(fl) PME-GFP, p35S:mature(m) PME-GFP, p35S:GFP-fIPME and p35S:GFP-mPME fusions. The cloning scheme is shown in Fig. 6.

### EXAMPLE 2

### Transient expression of the PME-GFP fusion in Nicotiana benthamiana leaves

### Plasmid DNA preparation

Plasmids carrying p35S:fIPME-GFP, p35S:mPME-GFP, p35S:GFP-fIPME, and p35S:GFP-mPME fusions were transformed into *E.coli* strain DH10B, maxi preps were grown in LB medium and DNA was purified using the Qiagen kit.

### Microprojectile bombardment

Microprojectile bombardment was performed utilizing the Biolistic PDS-1000/He Particle Delivery System (Bio-Rad). The cells were bombarded at 900-1100 psi, at 15 mm distance from a macrocarrier launch point to the stopping screen and 60 mm distance from the stopping screen to a target tissue. The distance between the rupture disk and the launch point of the macrocarrier was 12 mm. The cells were bombarded after 4 hours of osmotic pretreatment.

A DNA-gold coating according to the original Bio-Rad's protocol (Sanford *et al.,* 1993, In: Methods in Enzymology, ed. R.Wu, 217, 483-509) was done as follows: 25 µl of gold powder (0.6, 1.0 mm) in 50% glycerol (60 mg/ml) was mixed with 5 µl of plasmid DNA at 0.2 µg/µl, 25 µl CaCl₂ (2.5 M) and 10 µl of 0.1 M spermidine. The mixture was vortexed for 2 min followed by incubation for 30 min at room temperature, centrifugation (2000 rpm, 1 min), washing by 70% and 99.5% ethanol. Finally, the pellet was resuspended in 30 µl of 99.5% ethanol (6 µl/shot). A new DNA-gold coating procedure (PEG/Mg) was performed as follows: 25 µl of gold suspension (60 mg/ml in 50% glycerol) was mixed with 5 µl of plasmid DNA in an Eppendorf tube and supplemented subsequently by 30 µl of 40% PEG in 1.0 M MgCl₂. The mixture was vortexed for 2 min and than incubated for 30 min at room temperature without mixing. After centrifugation (2000 rpm, 1 min) the pellet was washed twice with 1 ml of 70% ethanol, once by 1 ml of 99.5% ethanol and dispersed finally in 30 µl of 99.5% ethanol. Aliquots (6 µl) of DNA-gold suspension in ethanol were loaded onto macrocarrier disks and allowed to dry up for 5-10 min.

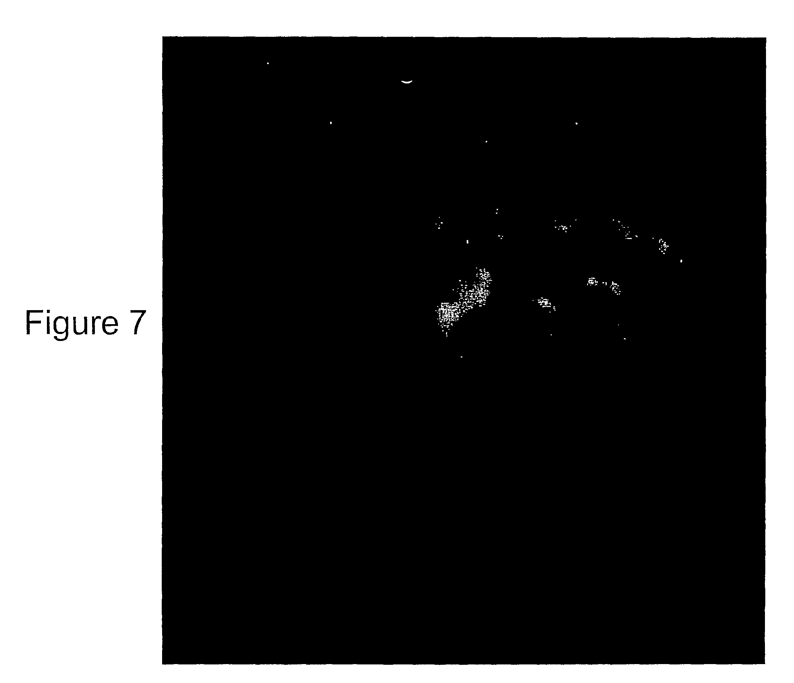

The leaves of *Nicotiana benthamiana* were bombarded by these gold particles coated with different plasmid DNA. The results of the experiments presented in Fig. 7 show that the fIPME-GFP fusion product is targeted to the cell wall.

### EXAMPLE 3

### Agrobacterium-mediated transformation of Arabidopsis thaliana

### Construct design

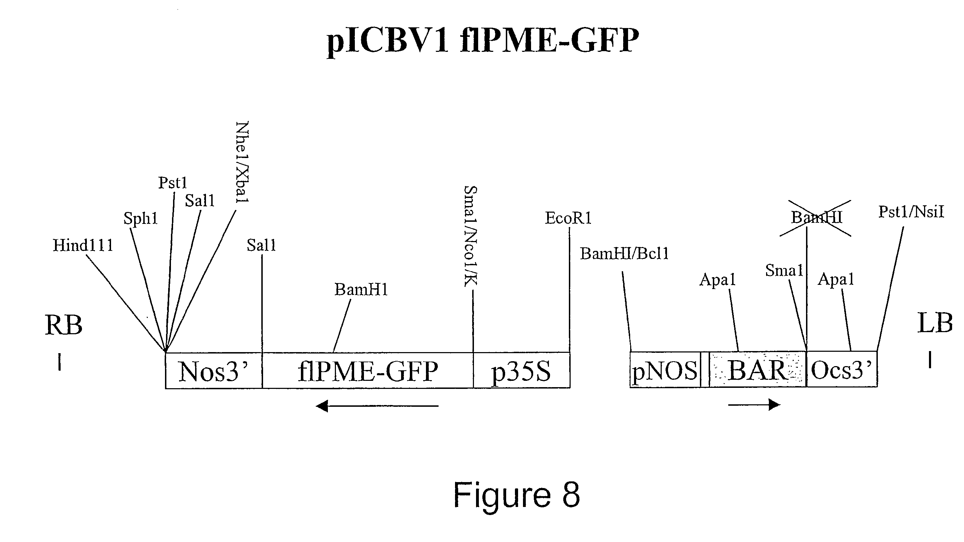

The large Nhe1-EcoR1 fragments of plasmids p35S:flPME-GFP, p35S:mPME-GFP, p35S: GFP-flPME and p35S:GFP- mPME described in Example 1 werte ligated with the large Xba1-EcoR1 fragment of binary vector pICBVI . Resulting plasmids pICBV flPME-GFP, piCBV1 mPME-GFP, pICBV1 GFP- flPME and plCBV1 GFP- mPME contained the p35S:flPME-GFP, p35S:mPME-GFP, p35S:GFP- fIPME and p35S:GFP- mPME expression cassettes in T-DNA region with the BAR gene as selectable marker. The T-DNA region of one construct, pICBV flPME-GFP is shown in Fig. 8.

### In planta transformation of Arabidopsis thaliana

The plasmids (carbenicillin resistant) were immobilized into *Agrobacterium tumefaciens* (strain GV 2260) by electroporation. The bacterial cells were grown in 300ml 2YT media with antibiotics, collected by centrifugation and resuspended in 5% sucrose to OD₆₀₀=0.8.

*A. thaliana* plants were grown until flowering. Then flowering bolts of *Arabidopsis* plants were dipped in *Agrobacterium* solution under vacuum applied for a few seconds. Transformed plants were kept in a dark place for 24 hours at high humidity and then transferred into the greenhouse. The seeds were collected 3-4 weeks later, sowed in soil and sprayed with 100mg/L phosphinothricin, 0.01 % Silvet. The treatment was repeated 2-3 times depending on the efficiency of selection and the frequency of late germination events. Transgenic *Arabidopsis* plants were used for studying the GFP localization within plant tissues.

### Selection for transformants

Two to four days after the bombardment, the filter paper with cells was transferred to the plate with CIM supplemented with the appropriate selection agent (10-15 µg/ml PPT). Every seven days, the material was transferred to fresh selection media. The plates were kept in the dark and after approximately 6 weeks the plant material was transferred to Petri plates with Morphogenesis Inducing Medium (MIM) (see Appendix) supplemented with the appropriate selection agent (10-15 µg/ml PPT). The plates were incubated at high light intensity, 16 hours day length.

### EXAMPLE 4

### Cell wall targeting of GFP using a translational fusion with the NtTLRP gene

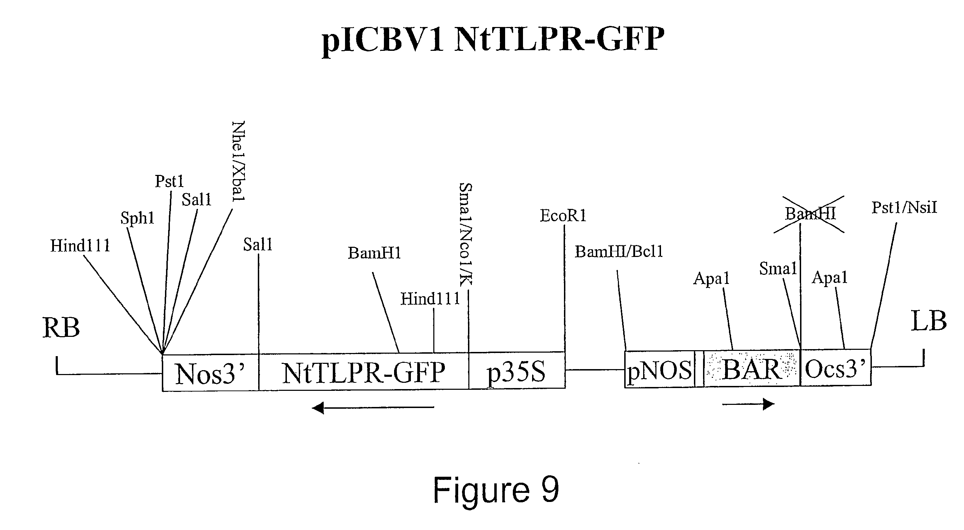

The binary vector-based translational fusion of GFP with the tobacco cell wall protein TLPR (Domingo *et al.,* 1999, *Plant J.,* 20, 563-570; Accession number Y19032) was performed in the same way as described for PME-GFP fusion, except for different primers.
a) 5'-TGACCCATGGGTTCTAAGGCATTTCTGTTTCTTG-3', corresponding to the N-terminal part of the NtTLPR coding sequence and containing a Ncol site.
b) 5'-TTTTGGATCCTTGTGAAGGCTGAACTTCAGTAAG-3', corresponding to the C-terminal part of the NtTLPR coding sequence and containing a BamHI site. The cloning strategy was similar to that of fIPME-GFP fusion described in Examples 1 and 3. The final construct containing the p35S:NtTLPR-GFP expression cassette within the T-DNA borders is shown in Fig. 9.

In order to introduce a protein cleavage site which can be cut precisely at the predetermined place in front of any amino acid residue, the *Arabidopsis* ubiquitin gene (UBQ11, US 5773705) was introduced between the NtTLPR and the GFP genes. The primers used for UBQ11 amplification were:
c) 5'-TTTTGGATCCAGATCTTCGTAAAGACTTTGACCG-3', corresponding to the N-terminal part of the UBQ11 coding sequence and containing a BamHl site;
d) 5'-TTTTGGATCCTTGTGAAGGCTGAACTTCAGTAAG-3', corresponding to the C-terminal part of the NtTLPR coding sequence and containing a BamHl site.

The BamH1 treated and gel-purified PCR fragment of the IBQ11 gene was ligated with the BamH1-digested and alkaline phosphatase treated vector pICBV1 NtTLPR-GFP. The orientation of cloned fragment was checked by PCR using different combinations of UBQ1 and GFP primers. The T-DNA region of the resulting plasmid is shown in Figure 8. Transient expression experiments and stable transformation of plants were performed as described in Examples 2 and 3.

### EXAMPLE 5

### Expression of NtTLPR-GFP fusion from crTMV vector

The Nco1-Sal1 fragment of NtTLPR-GFP fusion was cloned into a crTMV vector (Fig. 11) and used for transfection of *Nicotiana benthamiana, Nicotiana tabacum* and *Brassica* plants. Details of crTMV-based vector construction and reporter gene expression via an IRES element are given in reference examples 1 to 3 and in WO 02/29068.

### EXAMPLE 6

### Use of Ca²⁺-pectate binding site as polypeptide capable of binding to a cell wall component

In this example. a Ca²⁺-pectate binding site of apoplastic isoperoxidase from zucchini (APRX) (Carpin *et al.,* 2001, *The Plant Cell,* 13, 511-520) is used. It was shown that this site binds *in vivo* as well *in vitro* to pectin chains via cross-links formed by Ca²⁺-ions. This site has a of motif of four clustered arginines that expose their positive charges at the surface of the peroxidase. Ca²⁺-chelating agents like EGTA or EDTA are able to suppress the ionic interaction to the pectin chains because of their stronger affinity to Ca²⁺-ions. The peptide of 27 amino acid residues with α-helical structure was fused to the C-terminus of calreticulin targeting signal-GFP gene to create expression vector plCH8600-C (Fig. 14) and the 3' component (pICH9666-C, fig.14) of the viral "pro-vector system" (PCT/EP02/03476). The peptide contains three arginines of the Ca²⁺-pectate binding site which are mainly responsible for the binding of the APRX-protein to pectin.

The GFP-protein was isolated from agroinoculated *N. benthamiana* plants in three different ways: a) from crude extracts; b) from intercellular fluid (IF); c) from plant tissue squashed and washed in the presence of Ca²⁺-containing buffer.

In the case of a), the GFP-protein was bound to the cell wall fraction of crude extracts in the presence of Ca²⁺. After centrifugation, the supernatant was removed and the bound GFP-protein could be dissolved in an EGTA-buffer. In the case of b), GFP was eluted from the apoplast using EGTA-containing buffer. The same buffer was used to extract GFP from the squashed plant tissue. Further SDS-PAGE and Western blot-analysis confirmed comparative yields and purification efficiencies for all three different purification procedures.

### a) Cloning of protein purification expression vectors plCH8600-C and pICH9666-C

The GFP-sequence was amplified by PCR from construct plCH5290 (35Sprom-sGFP-NOS term) to fuse the Ca²⁺-pectate binding site in frame to the C-terminal end of the GFP-gene. The sequence of the forward primer (5'-TTTTCC ATG GTG AGC AAG GGC GAG GAG CTGT-3') introduced a Nco I-site at the 5'-end of the GFP-sequence whereas the reverse primer (5'-TTTT GGA TCC TAT TCT TGC GGG CCC TCG CTT GTA CAG CTC GTC CAT GCC-3') included an additional 24 bp spacer after the last codon of the GFP-sequence followed by a BamH I-site. This PCR-product was subcloned into construct plCH5290 as Nco I/BamH I-fragment generating construct plCH8155 (35Sprom-sGFP spacer wth 8 amino acid residues without stop codon-NOS transcription termination signal). The Ca²⁺-pectate biding site was introduced into construct plCH8155 by an adapter using the following oligos: 5'-cgggatccat tgttaaccgt cttggttctc gtgaaggaa ctttctttag acaatttcgt g-3' and 5'-tgctctagat tacctaatgt ttcccatctt aatcatagaa acacgaaatt gtctaaagaa agttccttc-3'. Protruding ends of this adaptor correspond to BamHl- and Xba l-digested sites, which were used for subcloning of the Ca²⁺-pectate binding domain into construct plCH8155. The final construct plCH8600 was Cla1 - Nco1 digested and used for cloning the calreticulin signal peptide (CSP) as Cla1 - Nco1 fragment (Borisjuk et al., 1999, *Nat. Biotechnol.,* 17, 466-469). The GFP-fusion cassette was also subcloned into the 3'-provector plCH9422 (not shown) as Nco I/Xba I-fragment yielding the 3'-provector plCH9666. The plCH9666 was Cla1-Nco1 digested, gel-purified and ligated with Cla1-Nco1 fragment of calreticulin signal peptide. The final construct, plCH9666-C is shown in Fig. 14.

### b) Expression of GFP-fusion protein in N. benthamina leaves after agroinfiltration of construct plCH8600-C and plCH9666-C

Agroinfiltration of tobacco plants was performed according to a modified protocol described by Yang and colleagues (Yang *et al.,* 2000, *Plant J.,* 22, 543-551). *Agrobacterium tumefaciens* strain GV31 01 transformed with appropriate constructs was grown in LB-medium supplemented with Rifampicin 50 mg/L, carbencillin 50 mg/l and 100 µM (micromolar) acetosyringone at 28 °C. Agrobacterium cells of an overnight culture (5 ml) were collected by centrifugation (10 min, 4500 g) and resuspended in 10 mM MES (pH 5.5) buffer supplemented with 10 mM MgSO₄ and 100 µM acetosyringone. Bacterial suspension was adjusted to a final OD₆₀₀ of 0.8. In case of delivery of several constructs, *Agrobacterium* suspensions of different constructs were mixed before infiltration. Agroinfiltration was conducted on near fully expanded leaves that were still attached to the intact plant. The bacterial suspension was infiltrated with a 5 ml syringe. By infiltrating 100 µl of bacterial suspension into each spot (typically 3-4 cm² in infiltrated area) 8 to 16 spots separated by veins could be arranged on a single tobacco leaf. Plants were further grown under greenhouse conditions at 22 °C and 16 h light.

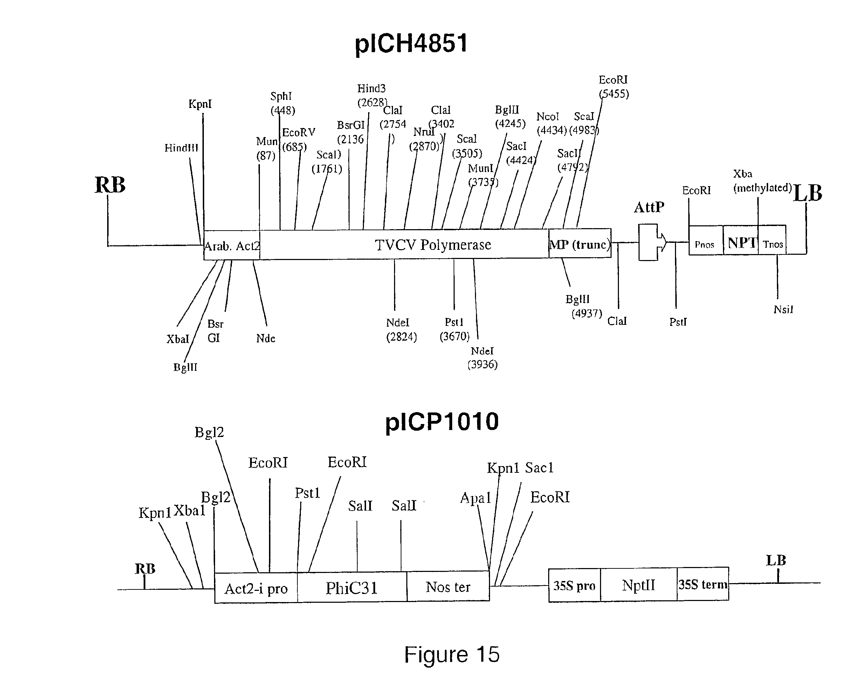

Construct pICH9666-C was co-infiltrated with the corresponding 5' pro-vector pICH4851 and the integrase construct pICP1010 (see Fig.15). As the result of integrase-mediated recombination between attP and attB sites, a viral vector expressing GFP at high level was assembled. Instead of plCP1010, any other vector capable of expressing the PhiC31 recombinase may be used.

The purification of the GFP fusion from infiltrated plant material was performed by using three different approaches:

### 1) Purification by binding to cell wall matrix:

Leaf material expressing the GFP-fusion construct was ground with an extraction buffer, containing 2 mM CaCl₂, 20 mM HEPES pH 7.0, 0.1 % Triton-x 100 and 5 mM β-Mercaptoethanol. Samples were centrifuged at 9500 g for 5 min at 4 °C after incubation on ice for 60 min. The supernatant was removed carefully and the pellet was resuspended in 20 mM HEPES pH 7.0 containing 2 mM EGTA and 0.1 % Tween 20 to dissolve the GFP-fusion protein. The GFP-content was analysed in supernatants of wash- and elution buffers by Western blot-analysis.

### 2) Purification by elution from apoplast:

Leaf material was infiltrated with 50 mM Tris-HCl, pH 7.5, 2mM CaCl₂, 2mM EDTA. The intercellular fluid (IF) containing secreted proteins, but not GFP, was removed by low-speed centrifugation (2000-3000 g). The procedure was repeated with the second buffer (50 mM Tris-HCI, pH 7.5, 2mM EDTA). The GFP, eluted by the second extraction buffer, was used for Western blot analysis. Different versions of extracting apoplast-targeted proteins can be found in several publications (Firek et al., 1993, *Plant Mol. Biol.,* 23, 861-870; Voss et al. 1995, *Molecular Breeding,* 1, 39-50; De Wilde et al., 1996, *Plant Science,* 114, 233-24; Kinai et al., 1995, *Plant* Cell, 7, 677-688; Liu et al., 1 996, *Plant Science,* 121, 123-131). The procedure described here, works well for isolation of protein fusion with a Ca²⁺-pectate binding site.

### 3) Purification by extraction from squashed plant tissue

The plant tissue was squashed between the rollers of sap extractor (Erich Pollahne, Hannover, Germany) three or seven (in case of viral provector system) days after agroinfiltration in presence of 2 mM CaCl₂, 20 mM HEPES pH 7.0, 0.1 % Triton-x 100 and 5 mM β-Mercaptoethanol. The remaining plant tissue was rinsed once with the same buffer and fusion protein was eluted using 20 mM HEPES pH 7.0 containing 2 mM EGTA and 0.1 % Tween 20. The GFP-content was analysed in supernatants of wash- and elution buffers by Western blot-analysis.

### EXAMPLE 7

### Use of Ca²⁺-pectate binding site for producing a cytotoxic protein

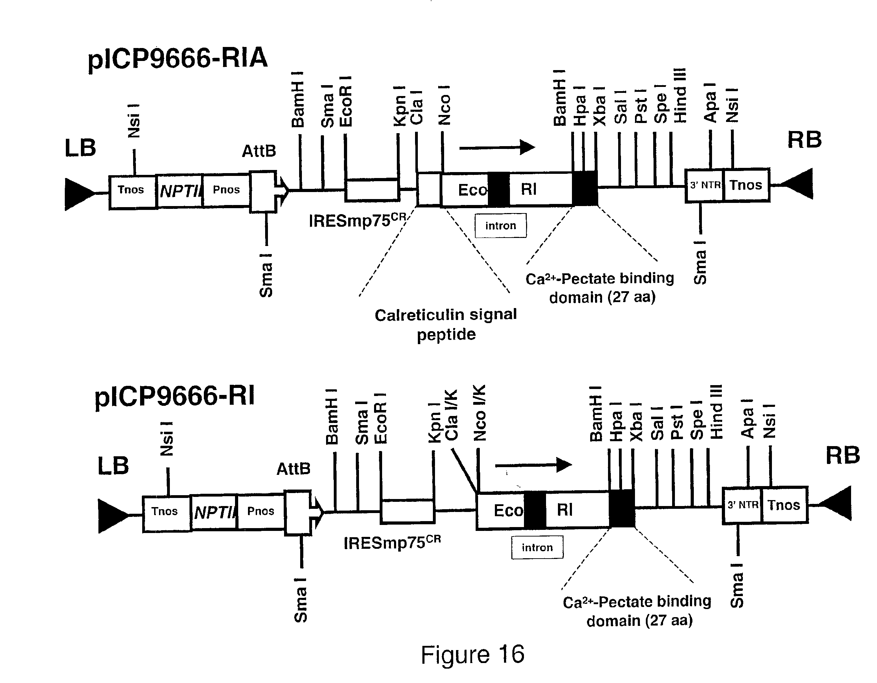

A synthetic gene encoding the EcoRl endonuclease (genomic sequence accession No. J01675), flanked by Ncol (5' end) and BamHl (3' end) restriction sites and containing an inserted plant splicesome-recognised intron after 721 bp of EcoRl cDNA. The following intron sequence (shown in bold) together with flanking EcoRl endonuclease sequences (in italics) was used: *5'-tatactcaag* **gttcgtaaag aacttttta ttttatcagt gtagtttgag cagttgtgac atattgtagt tctttaatac tcatgaaatt gtttttaatt tttaatatgg agtatag** *gagatggga* -3'. The 940 bp Ncol-BamHl fragment of synthetic sequence was ligated with the large Ncol-Xbal fragment of plCH9666-C (Fig. 14) and small BamHl-Xbal fragment containing Ca²⁺-pectate binding site yielding plasmid plCH9666-RIA (Fig. 16). Religation of large Clal-Ncol Klenow- treated fragment of pICH9666-RIA yielded pICH9666-RI, where the EcoRl endonuclease lacks apoplast-targeting signal peptide (Fig. 16).

The agrodelivery of pICH9666-RIA or pICH9666-RI together with the 5' pro-vector plCH4851 and the integrase construct pICP1010 was performed as described in example 6. As the result of integrase-mediated site-specific recombination, an amplification vector was assembled that produced the EcoRl enzyme either targeted into apoplast (for pICH9666-RIA) or with cytosolic location (for piCH9666-RI).

The cytotoxic effect of EcoRI in case of cytosolic localisation of EcoRI (construct pICH9666-RI) was clearly evident three days later, and five days after agroinfiltration the leaf tissue was badly damaged. On the contrary, apoplast targeting of the enzyme allowed to preserve agroinfiltrated plant leaves for much longer period of time (up to two weeks). The isolation of enzyme from plant tissue was performed as described in example 6 (1. "Purification by binding to cell wall matrix") from plant material three and five days after agroinfiltration. The enzyme concentration in activity units was measured by adding 1 µL of different x10 - x10⁴ dilutions to 20 µL of EcoRl reaction buffer containing pBS(KS+) with cloned 1.2 kb EcoRl DNA fragment. Commercially available EcoRl enzyme was used for determining the concentration. The comparative yield was approximately fifty times higher in case of apoplast targeting of the enzyme.

### REFERENCE EXAMPLES

The following reference examples 1 to 3 demonstrate the construction of viral vectors and expression of the GUS reporter gene via an IRES element. Further details can be found in WO 02/29068.

### REFERENCE EXAMPLE 1

### Construction of a tobamovirus vector infecting cruciferous plants

Virions of a known tobamovirus called crucifer tobamovirus (crTMV) which is able to infect systemically crucifer plants were isolated from *Olearacia officinalis L.* with mosaic symptoms. Results of crTMV host-range examination are presented in Table1.

### Plasmid constructions

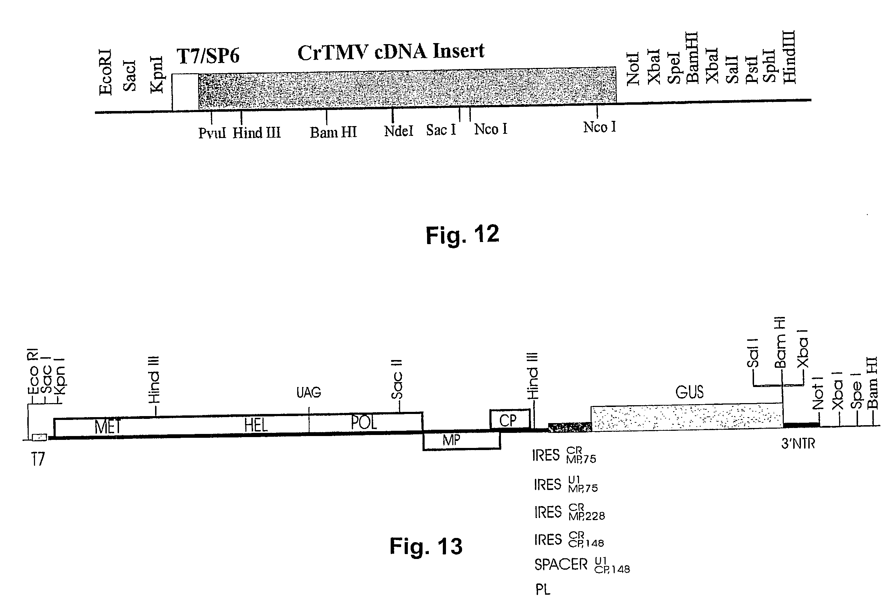

CrTMV cDNA was characterized by dideoxynucleotide sequencing (Dorokhov *et al.,* 1994 FEBS Letters 350, 5-8). Full length T7 RNA polymerase promoter-based infectious crTMV cDNA clones were obtained by RT-PCR from crTMV RNA using oligonucleotides crTMV1-Kpn 5' -gcat***ggtacc***ccttaatacgactcactataGTTTTAGTTTTATTGCAACAACAACAA (upstream), wherein the italic bold letters are a sequence of a Kpn I site, the underlined lowercase letters are nucleotide sequence of the T7 RNA polymerase promoter, the uppercase letters are from the 5'-termini of crTMV cDNA; and crTMV2 5'-gcat***gcggccgc***TGGGCCCCTACCCGGGGTTAGGG (downstream), wherein the italic bold letters are sequence of Notl site, the uppercase letters are from 3'-termini of crTMV cDNA and cloning into pUC19 between Kpnl and Bam Hl restriction sites (Fig. 12).

Full length SP6 RNA polymerase promoter-based infectious crTMV cDNA clones were obtained by RT-PCR from crTMV RNA by using oligonucleotides crTMV1-SP6 5'-gcat***ggtacc***atttaggtgacactatagaactcGTTTT AGTTTT A TTGCAACAACAACAA (upstream), wherein the italic bold letters are a sequence of a Kpn I site, the underlined lowercase letters are a nucleotide sequence of the T7 RNA polymerase promoter, the uppercase letters are from the 5'-termini of f crTMV cDNA; and crTMV2 5'-gcat***gcggccgc***TGGGCCCCTACCCGGGGTTAGGG (downstream), wherein the italic bold letters are a sequence of a Not I site, the uppercase letters are from 3'-termini of crTMV cDNA and cloning into pUC19 between Kpnl and Bam Hl restriction sites (Fig. 12).

The full-length crTMV cDNA clones were characterized by dideoxynucleotide sequencing. The ability of crTMV infectious transcripts to infect systemically *Nicotiana* and crucifer species was confirmed by infection tests on respectively *Nicotiana tabacum* var. Samsun and *Arabidopsis thaliana.*

### REFERENCE EXAMPLE 2

### Construction of tobamoviral vectors for expression of GUS genes in Nicotiana and crucifer plants via viral IRESs

Series of IRES-mediated expression vectors T7/crTMV/GUS were constructed as follows. First, Hind III and Xba I sites were inserted in the end of the CP gene of Sac II/Not I fragment of T7/crTMV vector (Fig. 12) by a polymerase chain reaction (PCR) and two pairs of specific primers. Second, IRES_{MP.75}^{CR}-GUS, IRES_{MP,75}^{UI}-GUS, IRES_{MP,228}^{CR}-GUS, IRES_{CP,148}^{CR-}GUS, IRES_{CP,148}^{UI}-GUS, PL-GUS cDNA described in Skulachev *et al.* (1999, Virology 263, 139-154) were inserted into Hind III and Xba I containing Sac II/Not I fragment of T7/crTMV vector to obtain Sac I-IRES_{MP,75}^{CR}-GUS-Not I, Sac II-IRES_{MP,75}^{UI}-GUS-Not I, Sac II-IRES_{MP,228}^{CR}-GUS-Not I, Sac II-IRES_{CP,148}^{CR}-GUS-Not I, Sac II-IRES_{CP,148}^{UI}-GUS-Not I, Sac II-PL-GUS-Not I cDNA, respectively. Third, Sac II-Not I cDNA fragment of T7/crTMV vector was replaced by Sac I-IRES_{MP,75}^{CR}-GUS-Not I or Sac II-IRES_{MP,75}^{UI}-GUS-Not I or Sac II-IRES_{MP,228}^{CR}-GUS-Not I or Sac II-IRES_{CP,148}^{CR}-GUS-Not I or Sac II-IRES_{CP,148}^{UI}-GUS-Not I or Sac II-PL-GUS-Not I cDNA to obtain respectively, vectorT7/crTMV/IRES_{MP,75}^{CR}-GUS (Fig. 13), vector T7/crTMV/IRES_{MP,75}^{UI-}GUS (Fig. 13), vector T7/crTMV/IRES_{MP,228}^{CR}-GUS (Fig. 13), vector T7/crTMV/IRES_{CP,148}^{CR}-GUS (Fig. 13), vector T7/crTMV/IRES_{CP,148}^{UI}-GUS (Fig. 13) and vectorT7/crTMV/PL-GUS (Fig. 13).

### REFERENCE EXAMPLE 3

### Expression of GUS gene in transfected Nicotiana and crucifer plants via viral IRESs

This example demonstrates the tobamovirus IRES-mediated expression of the GUS gene in *Nicotiana benthamiana* and *Arabidopsis thaliana* plants infected crTMV-based vectors: T7/crTMV/IRES_{MP,75}^{CR}-GUS (Fig. 13), vector T7/crTMV/IRES_{MP,75}^{UI}-GUS (Fig. 13), vector T7/crTMV/IRES_{MP,228}^{CR}-GUS (Fig. 13, vector T7/crTMV/IRES_{CP,148}^{CR}-GUS (Fig. 13), vector T7/crTMV/IRES_{CP,148}^{UI}-GUS (Fig. 13) and vectorT7/crTMV/PL-GUS (Fig. 13).

### In vitro transcription:

The plasmids T7lcrTMV/IRES_{MP,75}^{CR}-GUS (Fig. 13), vector T7/crTMV/IRES_{MP,75}^{UI}-GUS (Fig. 13), vector T7/CrTMV/IRES_{MP,228}^{CR}GUS (Fig. 13), vector T7/crTMV/IRES_{CP,14B}^{CR}-GUS (Fig. 13), vector T7/crTMV/IRES_{CP,148}^{UI}-GUS (Fig. 13) and vectorT7/crTMV/PL-GUS (Fig. 13) were linearized by Not I. The recombinant plasmids were transcribed *in vitro* as described by Dawson *et al*. (1986 Proc. Natl. Acad. Sci. USA 83, 1832-1836). Agarose gel electrophoresis of RNA transcripts confirmed that they were intact. The RNA concentration was quantified by agarose gel electrophoresis and spectrophotometry.

### GUS detection

Inoculated leaves were collected 10-14 days after transfection with capped full-length transcripts. IRES activity was monitored by histochemical detection of GUS expression as described earlier (Jefferson, 1987, Plant Molecular Biology Reporter 5, 387-405). Samples were infiltrated using the colorimetric GUS substrate, but the method (De Block and Debrouwer, 1992, Plant J. 2, 261-266) was modified to limit the diffusion of the intermediate products of the reaction: 0.115 M phosphate buffer, pH 7.0 containing 5-bromo-4-chloro-3-indolyl-β-D-glucuronide (X-Gluc) 600 µg/ml; 3 mM potassium ferricyanide; 10 mM EDTA. After incubation overnight at 37°C, the leaves were destained in 70% ethanol and examined by light microscopy.

### REFERENCE EXAMPLE 4

### Construction of spliceable PVX-based provector.

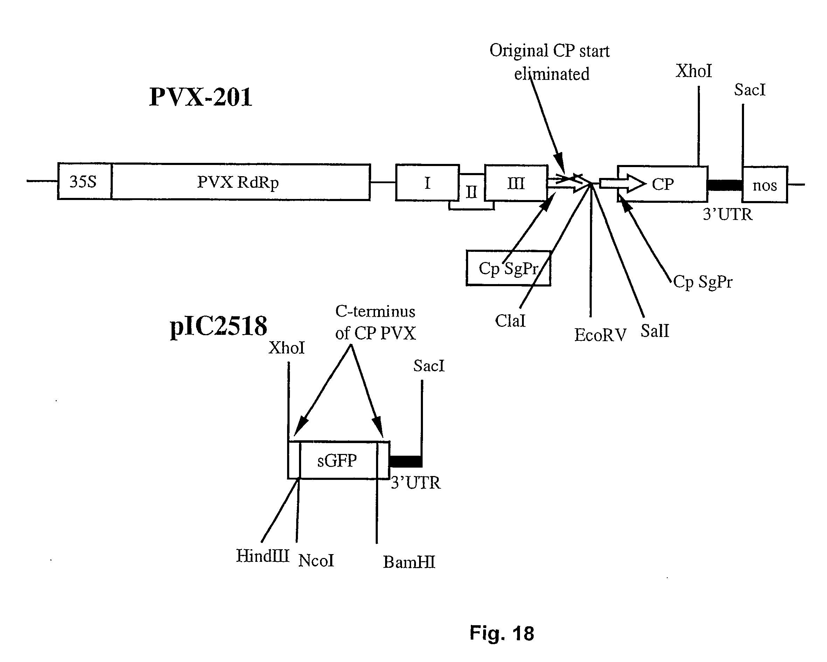

Two 35-PVX based provectors have been constructed. Plasmid PVX-201 was used as a basic construct for cloning (Chapman S *et al,* 1992, Plant J 1992 Jul;2(4):549-57). This plasmid contains the full-length cDNA of the potato virus X (PVX) genome (Gene Bank Accession Numbers NC 001455; AF172259) fused to the 35S promoter and the nos terminator. The CP subgenomic promoter region is duplicated and a few cloning sites are inserted between the duplicated regions in PVX-201 (see Fig. 18).

At the first step an intermediate construct plC2518 was obtained. This plasmid contains consequently: the C-terminus of CP (62 bp) from the Xhol site to the terminator with a Hindlll site introduced right after the termination codon, enhanced GFP (sGFP) gene (starting codon included into Ncol site) and the 3'-terminus of PVX virus including the C-terminus of CP (62nt), 3'-UTR and s poly (A) sequence followed by a Sacl site (Fig. 18).

Two sets of oligonucleotides were synthesized to clone 2 different pairs of donor/acceptor splicing sites:
D1+ AACGGTCGGTAACGGTCGGTAAA
D1- TCGACTTTACCGACCGTTACCGACCGTT
A1+ AGCTAACCTAGCAGGTTATATGCAGGTTATATGCAGGTC
A1- TTGGATCGTCCAATATACGTCCAATATACGTCCAGGTAC

### 2. D2+ CGAAAGGTAAG

D2- TCGACTTACCTTT
A2+ AGCTAACCTATTGCAGGTTGC
A2- CATGGCAACCTGCAATAGGTT

After annealing to each other, corresponding oligonucleotides form the following double stranded DNA fragments:
D1+/D1- fragment D1
   5' CGAACGGTCGGTAACGGTCGGTAAAG 3'
   3' TTGCCAGCCATTGCCAGCCATTTCAGCT 5'
A1+/A1- fragment A1
   5'AGCTAACCTAGCAGGTTATATGCAGGTTATATGCAGGTC 3'
   3' TTGGATCGTCCAATATACGTCCAATATACGTCCAGGTAC 5'
D2+/D2- fragment D2
   5' CGAAAGGTAAG 3'
   3' TTTCCATTCAGCT 5'
A2+/A2- fragment A2
   5' AGCTAACCTATTGCAGGTTGC 3'
   3' TTGGATAACGTCCAACGGTAC 5'
The 5'-protruding ends of the fragments are adhesive to Clal/Sall restricted DNA in case of D1 and D2. Fragments A1 and A2 can be ligated into HindIII-Ncol sites.

Plasmid PVX-201 described above (Fig. 18) was digested with Clal and Sall. Then the D1 or D2 fragment was ligated into the digested vector, yielding construct pIC3033 (in case of D1) of plC3053 (in case of D2).

Plasmid plC2518 (Fig. 18) was digested Hindlll and Ncol. Ligation of fragment A1 or A2 produced construct pIC3041 (A1) or plC3068 (A2), respectively.

Two variants of a spliceable PVX provector plasmid were obtained by cloning of the Xhol-Sacl fragment from pIC3041 into pIC3033 and of the XhoI-SacI fragment from pIC3068 into pIC3053. The resulting plasmids were named pIC3242 (splice sites D1+A1) and pIC3258 (splice sites D2+A2) (Fig. 18)

### REFERENCE EXAMPLE 5

### Microprojectile bombardment

Microprojectile bombardment was performed with the Biolistic PDS-1000/He Particle Delivery System (Bio-Rad). Separate *N. benthamiana* leaves were bombarded at 900 psi with 15 mm distance from a macrocarrier launch point to the stopping screen and 60 mm distance from the stopping screen to a target tissue. The distance between the rupture disk and a launch point of the macrocarrier was 12 mm. The cells were bombarded after 4 hours of osmotic pretreatment.

The DNA-gold coating procedure (PEG/Mg) was performed as follows: 25 µl of gold suspension (60 mg/ml in 50% glycerol) was mixed with 10 µl of plasmid DNA (up to 1 µg/ul) in an Eppendorf tube and supplemented subsequently by 10 µl of 40% PEG in 1.0 M MgCl₂. The mixture was vortexed for 2 min and than incubated for 30 min at room temperature without mixing. After centrifugation (2000 rpm, 1 min), the pellet was washed twice with 1 ml of 70% ethanol, once by 1 ml of 99.5% ethanol and finally it was dispersed in 30 µl of 99.5% ethanol. Aliquots (6 µl) of DNA-gold suspension in ethanol were loaded onto macrocarrier disks and allowed to dry up for 5-10 min.

### Plasmid DNA preparation

Plasmids were transformed into *E.coli* strains DH10B and JM109, maxi preps were grown in LB medium and DNA was purified using the Qiagen kit.

### REFERENCE EXAMPLE 6

### Mechanical inoculation of plants with provector plasmid DNA

Fully developed leaves of five to seven weeks old *Nicotiana benthamiana* plants were inoculated with plasmid DNA by mechanical wounding. For this purpose, 10 -50 µg of DNA was mixed with 3x GKP-buffer (50 mM glycine, 30 mM K₂HPO₄, 3% celite, 3% benthonite) and scratched gently on the upper side of the leaves.

### REFERENCE EXAMPLE 7

### Expression of a reporter gene in plant leaves by converted (spliced) PVX-based provector

Fully developed *N. benthamiana* leaves were bombarded with plasmids plC3242 and plC3258. It was expected that two different RNA transcripts can be synthesized in plant cell from each of these plasmids - complete form and spliced form (see Fig. 17).

The presence of the second transcript can be detected by the GFP fluorescence in a cell transfected with the provector.

Strong GFP fluorescence has been observed in numerous leave cells bombarded with plC3242 (48 hours after bombardment). No GFP expression was detected in the case of plC3258 - so this construct may serve as a negative control in this experiment. This difference occurred due to the different donor/acceptor sites used in the constructs (see reference example 4). In the case of plC3258, a 9-nt sequences that represents splice-site consensus of *Arabidopsis thaliana* was used. In the case of plC3242, donor and acceptor sites were designed as described in Nussaume *et al. Mol.gen.genet,* 1995, 249:91-101 where they were tested and proved to be active in plants.

According to several investigations (Fedorkin *et al., J Gen Virol* 2001; 82(Pt 2):449-58, Cruz *et al., Plant Cell* 1998; 10(4):495-510), the CP of PVX is required for viral cell-to-cell transport. In case of the construct plC3242, the CP gene must be spliced out of RNA. This means that the spliced form of the transcript (Fig. 18) cannot express the CP and provide cell-to-cell movement of the virus. However, in several experiments with plC3242 not only single cells but also multi-cell.loci (10-1000 cells) expressing GFP where detected. This indicates that the splicing of the provector transcript does not occur with 100% efficiency. A fraction of the full length RNA remains unspliced and therefore CP can be expressed from this form of the transcript, while the GFP gene remains silent in this RNA (since no GFP expression was detected in case of plC3258).

### REFERENCE EXAMPLE 8

### Generating transgenic N. benthamiana plants expressing Cre recombinase.

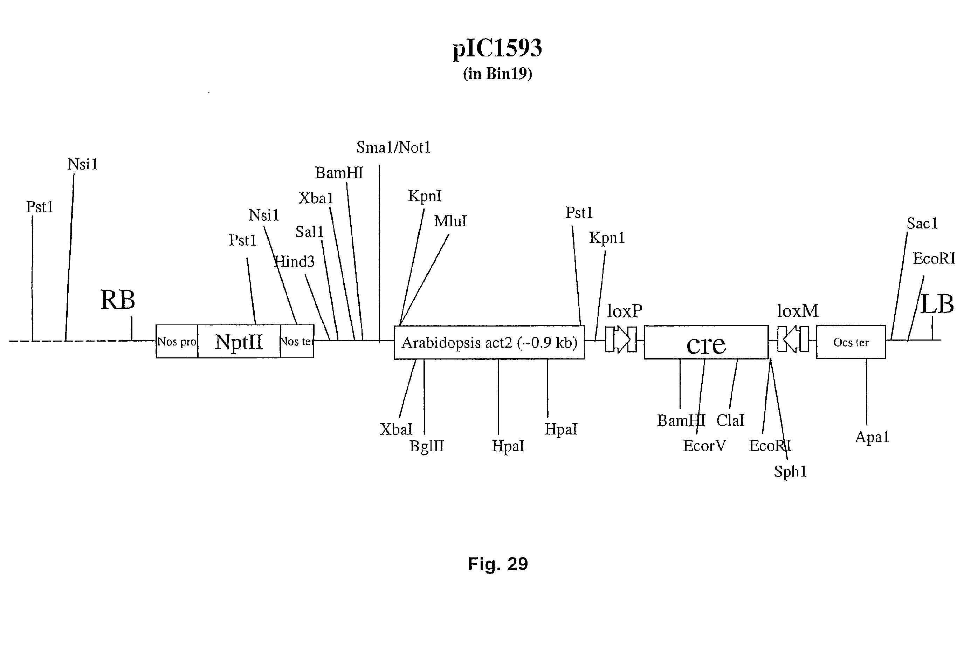

The actin2 promoter-LoxP-Cre Orf-Nos terminator fragment from pIC1321 was subcloned as a Not1 blunt-SacI fragment into the Smal and SacI sites of the binary vector pBIN19, resulting in construct pIC1593 (Fig. 29).

The plasmid pIC1593 was introduced in *Agrobacterium* strain Agl1 by electroporation and transformed agrobacteria were used to transform *N. benthamiana.* DNA extracted from 10 transformants was used to test for the presence of the transgene by PCR. All plants were found positive when PCR was performed with primers for the kanamycin transformation marker or for the Cre gene.

### REFERENCE EXAMPLE 9

### Generation of functional vectors from provectors using site-specific recombination

### a) General description of the system

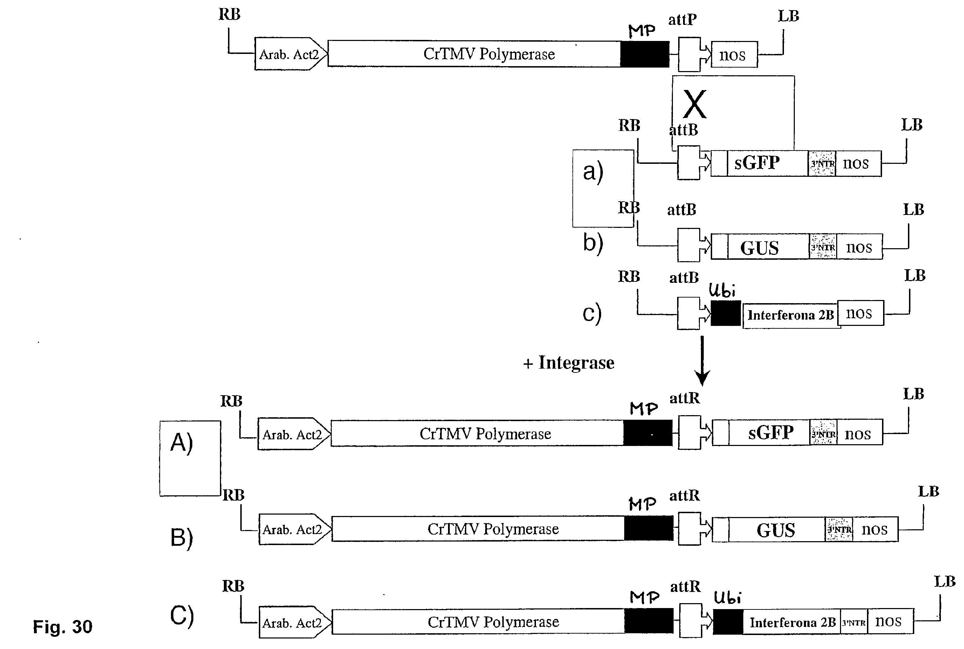

The system described consists of two core types of CrTMV based vectors which carry LoxP-recombination sites (Fig. 23):
i) Vector type: Polymerase-MP-LoxP: The vector encodes the RdRP of CrTMV and the movement protein (MP), which allows the virus to move from cell to cell, but not to move systemically. The viral transcription is controlled by an *Arabidopsis-Actin* 2 promoter.
ii) Vector type: LoxP-gene of interest-3'NTR-nos-terminator (see Fig. 23 a-c): This class of vectors encode a reporter gene (sGFP or GUS) and regulatory elements (nos: nopalin-synthase terminator; 3' NTR: nontranslated region, pseudoknots and tRNA-like structure of CrTMV). The expression of the coat protein (CP; see Fig. 23 b) allows the vector to spread systemically in the host plant.
iii) Vector type: Polymerase-MP-attP: See i), with attP-recombination site
iiii) Vector type: attB-gene of interest-3'NTR-nos-terminator (see Fig. 30 a-c): See ii), with attB-recombination site

After recombination catalyzed by Cre-recombinase or integase (via coinfection of a Cre or integrase encoding viral construct) functional units (replions) are formed which are able to express a reporter gene efficiently and are capable of moving from cell-to-cell (Fig. 23 A-C, 30 A-C) or systemically (Fig. 23 B).

### b) Plasmid construction

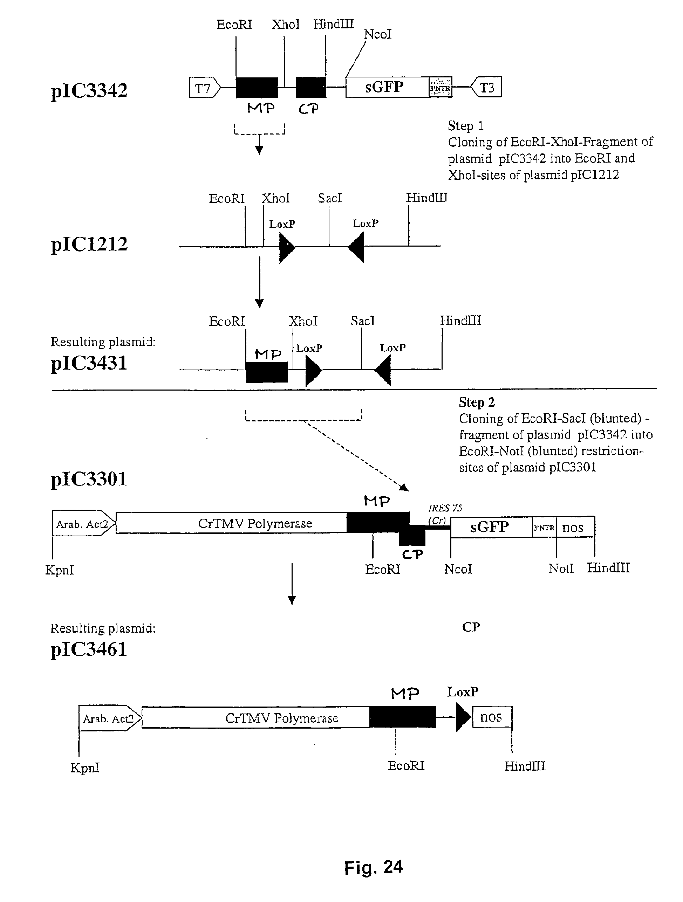

### A. Cloning of vector type Polymerase-MP-LoxP (Fig. 24)

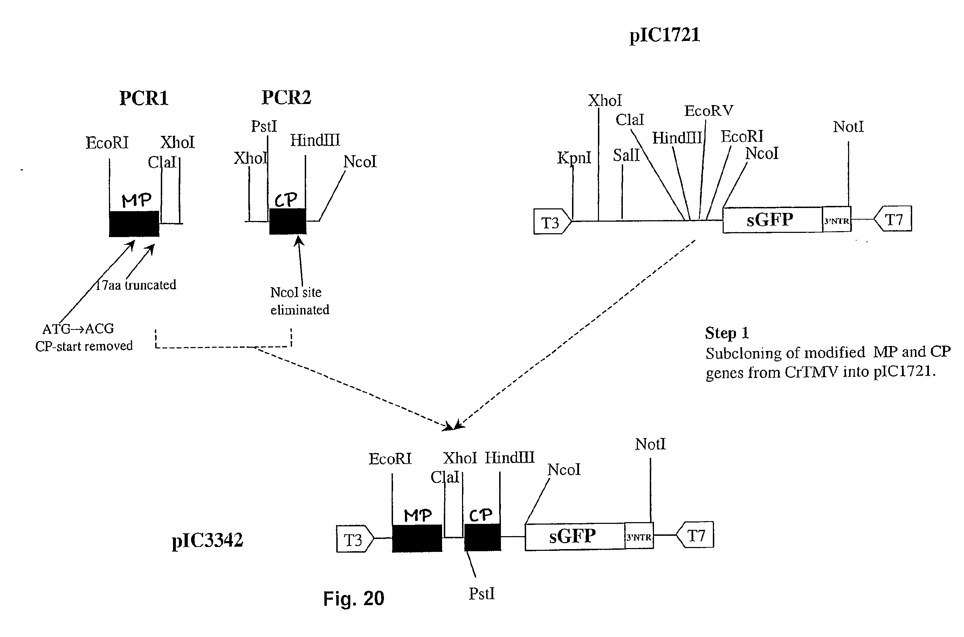

An EcoRI-Xhol-fragment of MP was taken from plasmid pIC3342 (Fig. 20) and was cloned into plasmid pIC1212 which carries two LoxP-sites in opposite orientations. The MP gene of plasmid pIC3342 contains a terminator codon which was introduced 25 AA before the natural stop. In the resulting product pIC3431, a fragment containing part of the MP gene is located next to a LoxP-site. Both elements are isolated via EcoRI-SacI restriction. After blunting the Sacl restriction site, the fragment was cloned into the vector-containing part of plasmid pIC3301, which contains a single EcoRI restriction site in the MP gene. NotI (blunted) was chosen as a second restriction site. In the resulting ligation product (pIC3461) the CP-gene, the IRES-sequence, the gene for sGFP and the 3' nontranslated region of pIC3301 are replaced by LoxP (Fig. 24).

### B. Cloning of LoxP-reportergene-3'NTR-nos-terminator vectors

### a) Construct LoxP-sGFP-3'NTR-nos (Fig. 23a)

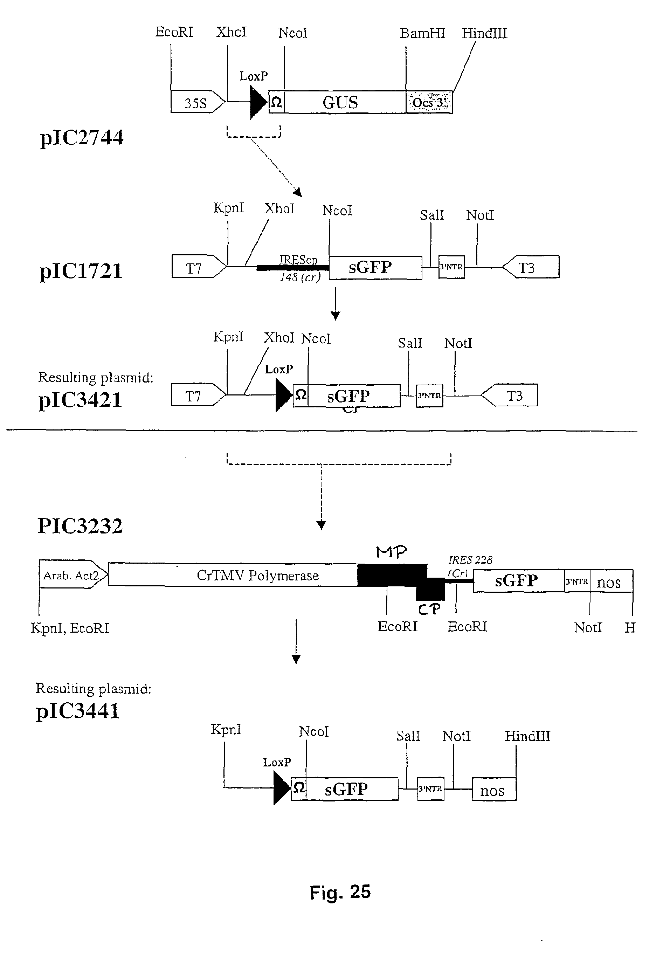

The Xhol-Ncol-fragment which carries a LoxP-site next to an Ω-leader-sequence was taken from vector pIC2744. In order to place the sequences adjacent to a reporter gene, the fragment was cloned into plasmid pIC1721, which contains the appropriate restriction sites next to a gene for sGFP and a 3'NTR-sequence. Replacement of an IRES-sequence by the fragment let to the resulting plasmid pIC3421. In order to add a nos-terminator-sequence, plasmid pIC3421 was cut by Kpnl and Notl. The fragment was introduced into the vector-containing part of plasmid plC 3232 and the final construct pIC3441 could be obtained (see Figures 25 and 23a).

### b) Construct LoxP-CP-sGFP-3'NTR-nos (Fig. 23 b)

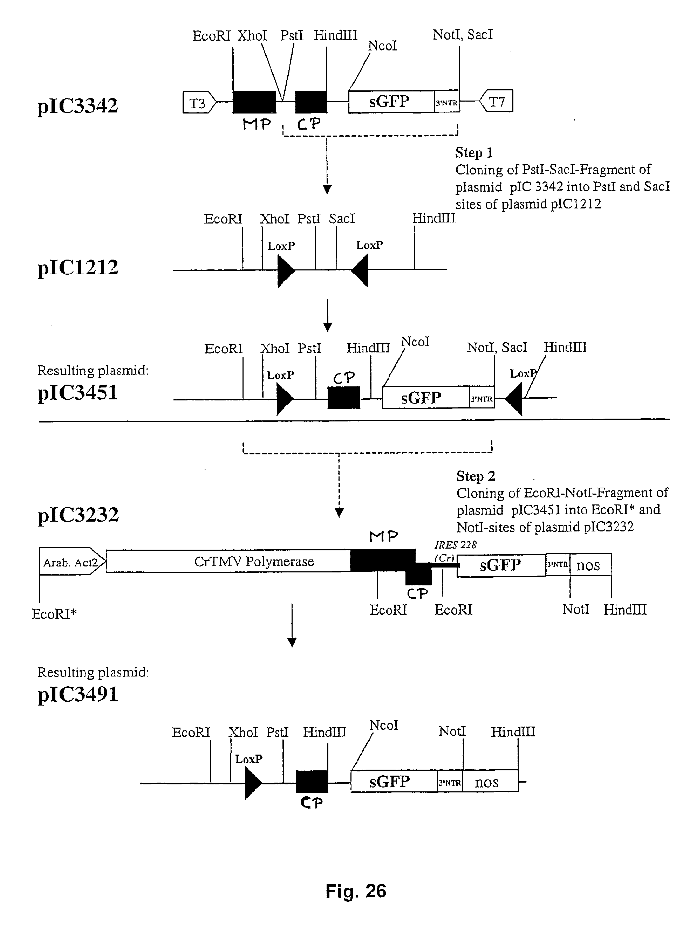

Described above plasmids plC3342 and plC1212 were used as starting vectors. The Pstl-Sacl fragment of plC3342 that contains the genes for CP and sGFP was cloned into plC1212. As a result, a LoxP-site is located next to CP and sGFP in the product pIC3451. In order to add a nos-terminator sequence, a similar approach as in case a) was used: An EcoRl-Notl-fragment of plC3451 was introduced into the vector-containing part of plC3232 resulting in plasmid plC3491 (see Fig. 26).

### c) Construct LoxP-CP-GUS-3'NTR-nos (Fig. 23 c)

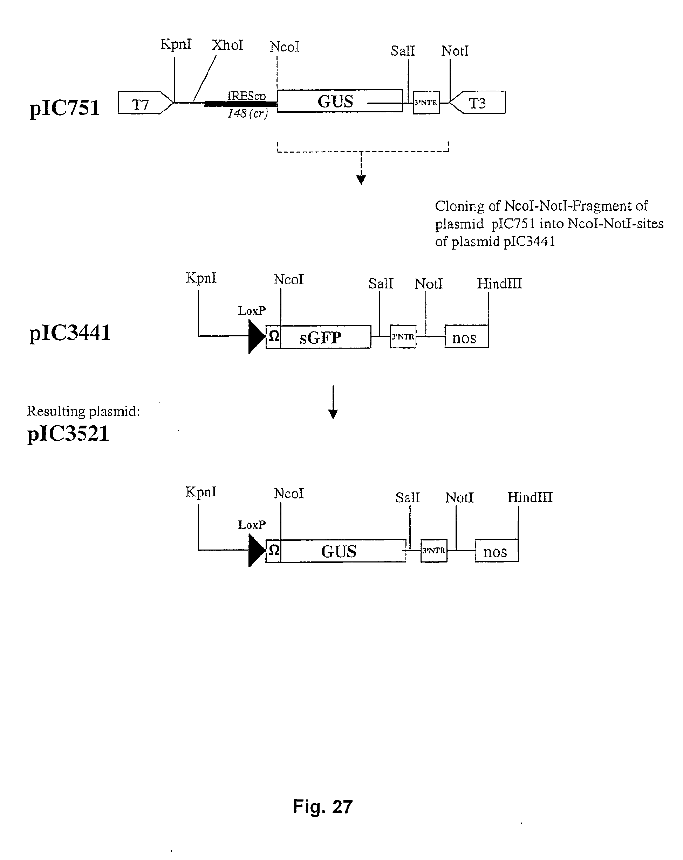

Plasmid plC751 is analogous to plC1721 and carries a GUS reporter gene instead of sGFP. By cutting with Ncol and Notl the GUS-Gene and the 3'-untranslated region can be directly cloned into plC3441 hence obtaining the final construct plC3521 (Fig. 27).

### REFERENCE EXAMPLE 10

### Expression of a single reporter gene in plant leaves by converted (recombined) CrTMV-based provector

Separate *N. benthamiana* leaves were particle-bombarded with a mixture of three plasmids: plC3441 (LoxP-GFP, Fig. 25), plC3461 (Actin2 promoter-CrTMV RdRp-MP-LoxP, Fig. 24) and plC2721 (Cre-Recombinase under control of hbt promoter). GFP fluorescence was detected in several multi-cellular loci 38 hours after bombardment. A strong increase of fluorescence and of the size of the infected area were observed during the following days (bombarded leaves where incubated at 25°C on wet filter paper). No GFP fluorescence was detected in control leaves bombarded with plC3441 together with pIC2721 without plC3461.

### REFERENCE EXAMPLE 11

### Expression of several genes in plant leaves by converted (recombined) CrTMV-based provector

Separate *N. benthamiana* leaves were particle-bombarded with a mixture of several plasmids:
a) plC3441 (LoxP-GFP, Fig. 25), plC3461 (Actin2 promoter-CrTMV RdRp-MP-LoxP, Fig. 24) and plC2721 (Cre-Recombinase under control of hbt promoter), plC3521 (LoxP-GUS, Fig. 27).
b) plC3441 (LoxP-GFP, Fig. 25), plC3461 (Actin2 promoter-CrTMV RdRp-MP-LoxP, Fig. 24) and plC2721 (Cre-Recombinase under control of hbt promoter), plC3491 (LoxP-CP, Fig. 26).

Both reporter genes GFP and GUS were strongly expressed in bombarded leaves in case a). Viral particle formation and systemic movement of the virus are take place in case b).

### REFERENCE EXAMPLE 12

### Construction of spliceable CrTMV-based provector

Spliceable provector based on CrTMV virus has certain advantages comparing to PVX-based one. Described in Fig. 19 system allows one to create reporter gene expressing system with the virus, which is fully functional in the infected leaf. Contrary to PVX, coat protein of CrTMV is not required for viral cell-to- cell movement so splicing does no influence on the viral spread in the infected leaf.

### A. Cloning of intermediate construct pIC3312

A PCR fragment was obtained using cloned cDNA of CrTMV and the following primers:
MPERI+ (corresponds to middle-region of MP including EcoRI site - position 5455 in CrTMV genome): GTGGTTGACGAATTCGTC
MP- (Complementary to C terminus of MP 17 aa upstream of the natural termination codon introducing artificial terminator with downstream Clal and Xhol sites. Also this primer contains point mutation of CP ATG into ACG which removes natural start of CP gene without amino acid change in the MP): GGTCTCGAGTTATCGATTATTCGGG-TTTGTAATGTTGTAAGACGTTTTCTTCTTTC

Another PCR fragment was obtained using the same template and the following primers:
CP+ (Corresponds to beginning of CP gene with Xhol and Pstl sites introduced upstream of ATG): TAACTCGAGACCTGCAGCATGTCTTACAACATTACAAACC-CGAATCAG
CP- (Complementary to C terminus of CP introducing single nucleotide substitution to eliminate Ncol site in CP gene. Also this primer introduce Hindlll and Ncol restriction sites downstream of CP gene): CTACTCCATGGTCAAGCTTAAGTAGC-AGCAGCAGTAGTCCACGGCACC

First, the PCR fragment was digested with EcoRI and Xhol enzymes. Second, Xhol and Ncol digested fragments were ligated together into vector plC1721 (Fig. 20) by EcoRl and Ncol sites yielding plasmid pIC3151 (Fig. 20). Then pIC3151 was digested Kpnl - EcoRV, the Kpnl site was blunted with T4 DNA polymerase and the cut plasmid was selfligated to eliminate the sites between Kpnl and EcoRV. The resulting plasmid was named plC 3312 (see Fig. 20).

### B. Cloning of spliceable provector pIC3393 and control construction pIC3401

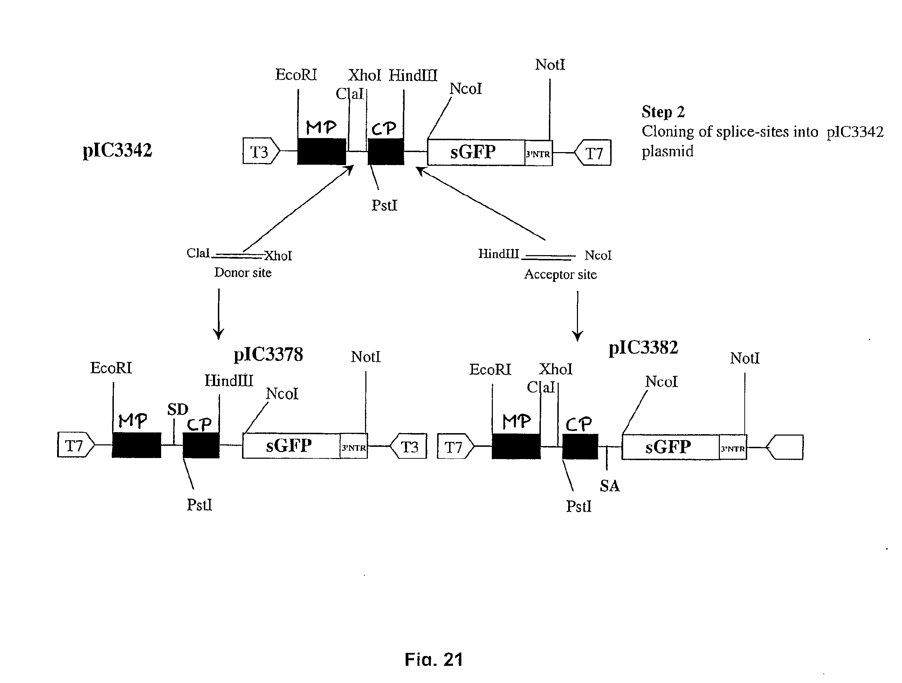

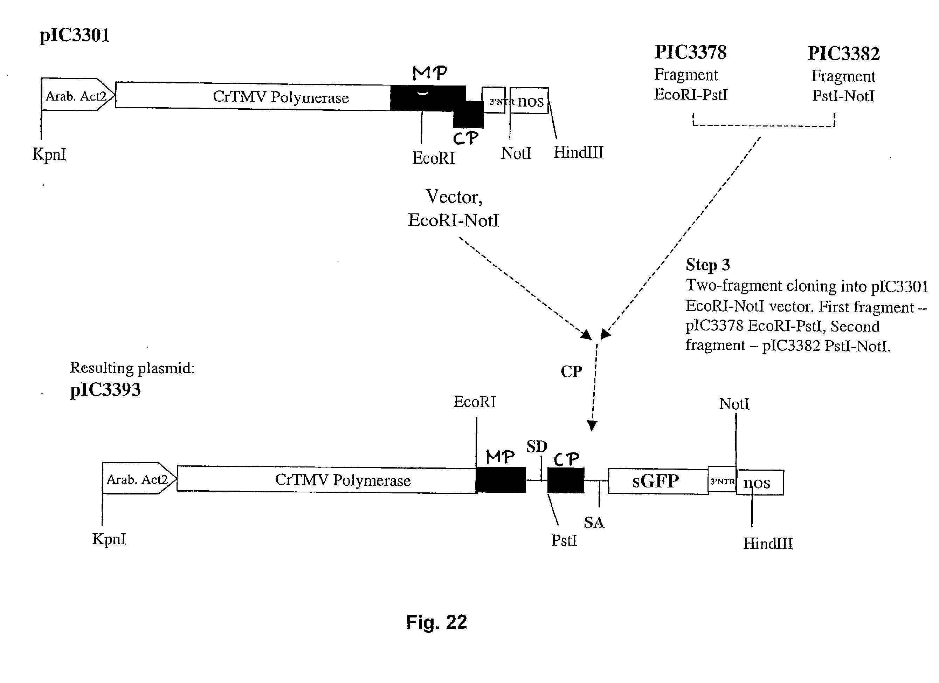

The dsDNA fragments described in reference example 4 containing donor and acceptor splice sites were cloned into plC3312 using Clal and Xhol sites in the case of donor and Hindlll and Ncol in case of acceptor splice sites (see Fig. 21). The obtained plasmids were named plC3378 (donor site) and plC3382 (acceptor site).
To obtain a negative control construct, plC3401 EcoRl-Notl fragment from plC3382 was cloned into plC3301 (Fig. 22).
The final construct plC3393 was obtained in two-fragment cloning using the EcoRl-Pstl fragment from plC3378 with the Pstl-Notl fragment from plC3382 and plC3301 digested EcoRl and Notl as a vector (Fig. 22).

### REFERENCE EXAMPLE 13

### Expression of reporter gene in plant leaves by converted CrTMV-based spliceable provector

Separate *N. benthamiana* leaves were particle-bombarded with plasmids plC3393 and plC3401. GFP fluorescence was detected in several multi-cellular loci 48 hours after bombardment in case of plC3393. No GFP fluorescence appeared in leaves bombarded with control construct plC3401.

### REFERENCE EXAMPLE 14

### Expression of one gene of interest from viral vector assembled from two CrTMV-based provectors through site specific att/integrase-based recombination system

### a) General description of the system

The system described consists of two core types of CrTMV based vectors which carry attB- or attP-recombination sites (Fig. 30):
i) Vector type: Polymerase-MP-attP: The vector encodes the RdRp of CrTMV and the movement protein (MP), which allows the virus cell to cell, but not systemic movement. The viral transcription is controlled by an *Arabidopsis-Actin* 2 promoter.
ii) Vector type: attB-gene of interest-3'NTR-nos-terminator (see Fig. 30 a-b): This class of vectors encodes a reporter gene (sGFP or GUS) and regulatory elements (nos: nopalin-synthase terminator; 3' NTR: nontranslated region, pseudoknots and tRNA-like structure of CrTMV).
iii) Vector type: attB-ubiquitin-gene of interest-3'NTR-nos-terminator (see Fig. 30 c): In order to obtain a defined protein which can be isolated from plants, an ubiquitin-cleavage-signal-sequence was introduced between the attB-recombination-site and the downstream located gene of interest (e.g. GFP, Interferona2B, Insulin or Somatotropin). By using this system, any amino acid except prolin can be chosen as the first amino acid of the synthesized protein.

After recombination catalyzed by the integrase-enzyme (via coinfection of a integrase-encoding viral construct), functional units (replicons) are formed which are able to express the gene of interest efficiently and are capable of cell-to-cell movement (Fig. 23 A-C).

### b) Plasmid construction

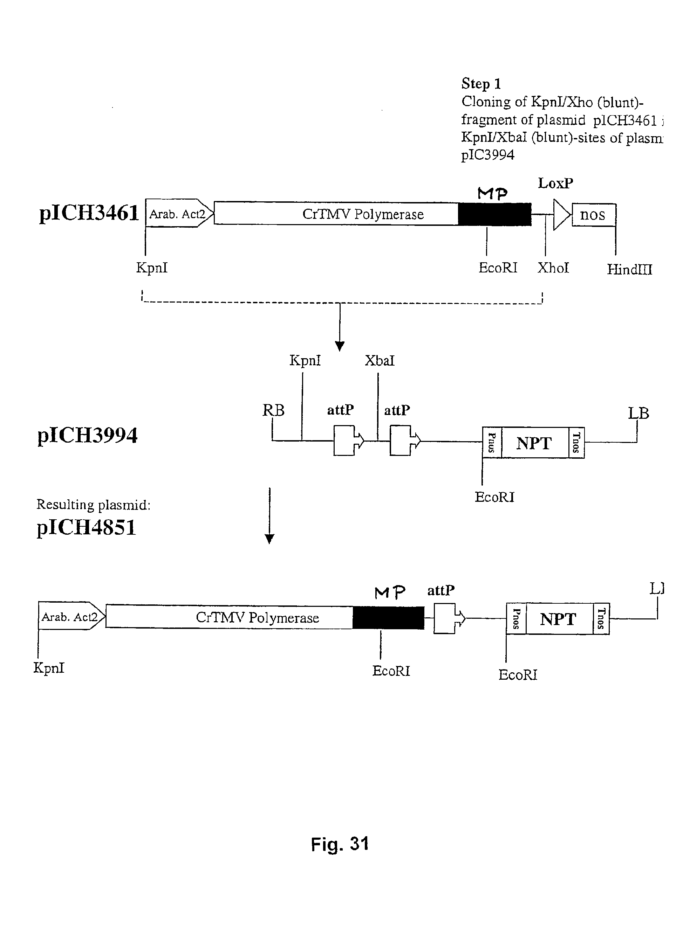

### A) Cloning of vector type: polymerase-MP-attP (Fig. 31)

An KpnI-XhoI-fragment fragment was taken from plasmid pIC3461 (Fig. 31). After blunting the XhoI-restriction-site, the fragment was cloned into the binary vector pIC3994 which carries two attB-sites in direct orientations, left- and right-T-DNA-borders (LB and RB) and a Kanamycin-expression-cassette as a plant transformation marker. Like the analogous clone from the Cre/Lox-system, the resulting plasmid pICH4851 carries an MP gene with a terminator codon which was introduced 25 AA before the natural stop.

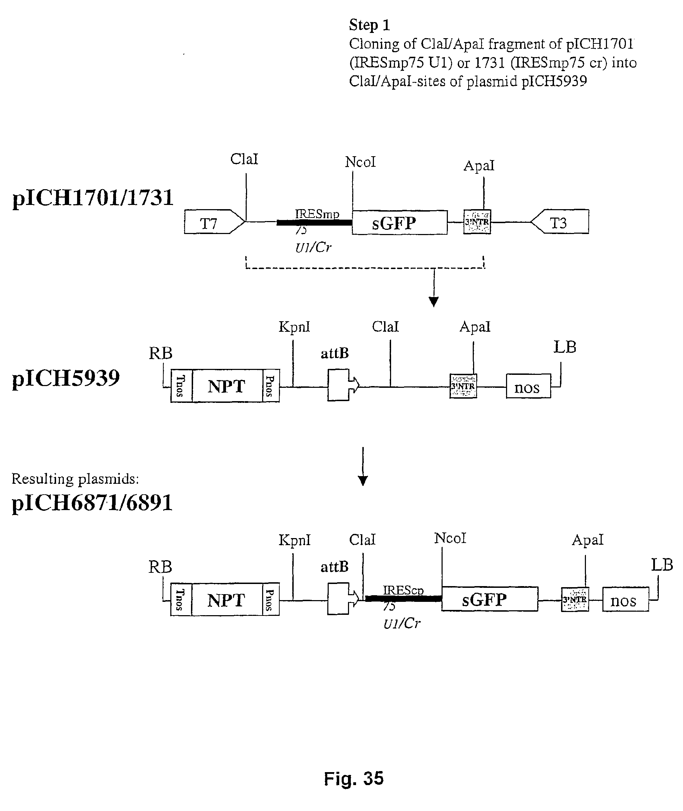

### B. Cloning of the vector-type: attB-gene of interest-3'NTR-nos-terminator (Figs. 27-35)

### a) Construct attB-sGFP-3'NTR-nos

### Primer combination A)

- attB Xho (+):: ATCACTCGAGCTCGAAGCCGCGGTGCGGGT: Complementary to the 5'-part of attB and carrying an Xhol-restriction site at the 5'-terminus
- attB Ω (-):: GGTAATTGTTGTAAAAATACGATGGGTGAAGGTGGAGTACG: The 3'-part of the primer corresponds to the 3'-region of the attB-sequence, the 5'-part contains 20 nucleotides with complementary to the 5'-part of the Ω-element.

Primer combination A was used on an attB-containing template in order to create a PCR-product which consists of the complete attB-sequence, 20 Nt of the Ω-leader sequence and an Xhol-restriction-site at the 5'end.

### Primer combination B)

- attB Ω (+):: CGTACTCCACCTCACCCATCGTATTTTTACAACAATTACC:
The 3'-part of the primer corresponds to the 5'-region of the Ω-sequence, the 5'-part contains 20 nucleotides with complementary to the 5'-part of the attB-element.
- Ω Ncol (-):: CATGCCATGGGTAATTGTAAATAGTAATTGTAATGTT
Complementary to the 3'-part of Ω and carrying an Ncol-restriction site at the 5'-terminus

Primer combination B was used on an Ω-sequence-containing template in order to create a PCR-product which contains the complete sequence of the Ω-leader, 20 Nt of the attB-recombination site and a Ncol-restriction-site at the 3'end.

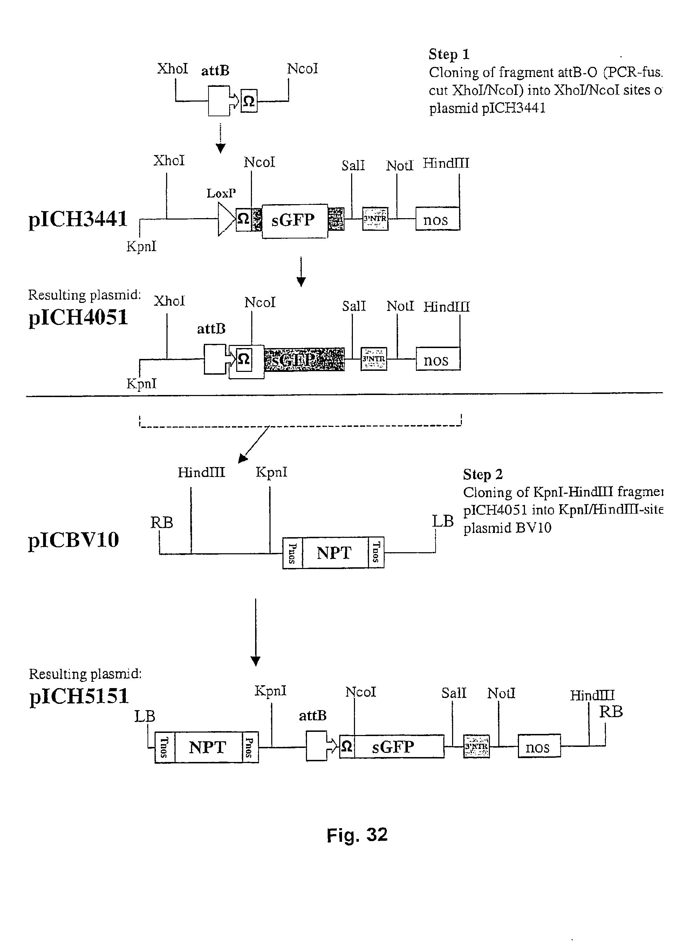

After obtaining PCR products from primer combination A) and B), the isolated oligonucleotides were used together as templates for a PCR-reaction by using the primers attB Xhol (+) and Ω Ncol (-). As a final PCR-product, a fusion of the attB-recombination- and Ω-leader-sequence could be synthesized. This fragment contains Xhol- and a Ncol-restriction sites at its termini. The PCR-product was isolated, digested and cloned into the Xhol- and Ncol-sites of plasmid pICH3441 in order to replace the LoxP-Ω-fusion. The intermediate plasmid was treated with KpnI and HindIII. By inserting this fragment into the corresponding sites of the binary vector BV10, a attB-sGFP-3'NTR-nos vector could be obtained which is transformable into *Agrobacterium* (pICH5151, see Fig. 32).

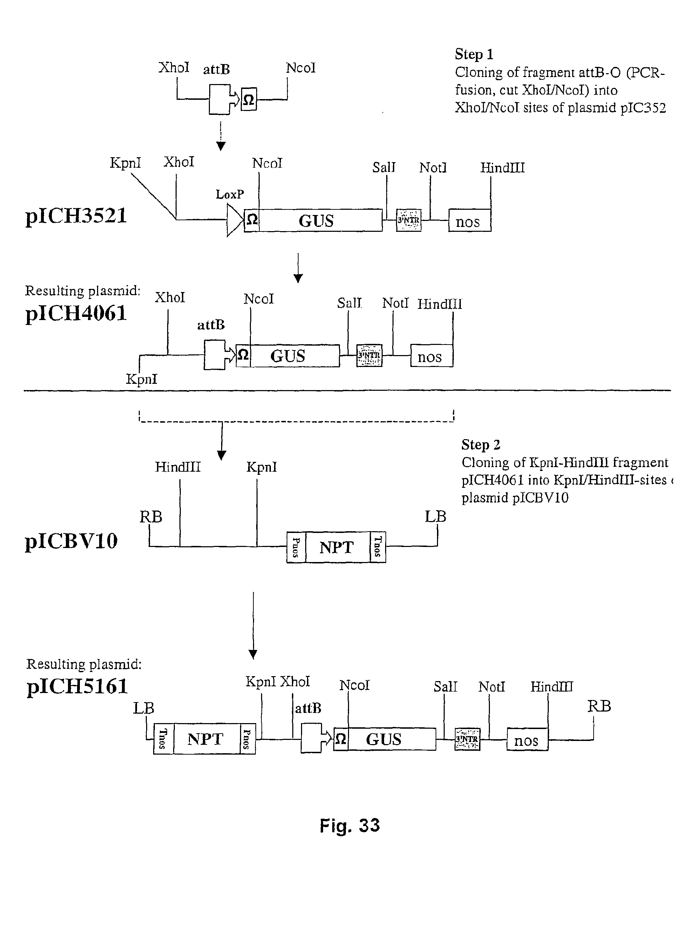

A similar approach was used for cloning an analogous provector with the reporter gene GUS. The above described PCR-fragment was cloned into the Xhol- and Ncol-sites of the GUS-containing plasmid plCH3521 resulting in plasmid plCH4061. Finally, a Kpnl-Hindlll-fragment of plasmid plasmid plCH4061 was ligated into the binary-vector BV10, resulting in the final vector plCH5161 which is capable of stable Agrobacterium-transformation (plCH5161, see Fig. 33).

### C) Construction of a attB-Ubiquitin-interferon-containing 3'-provector

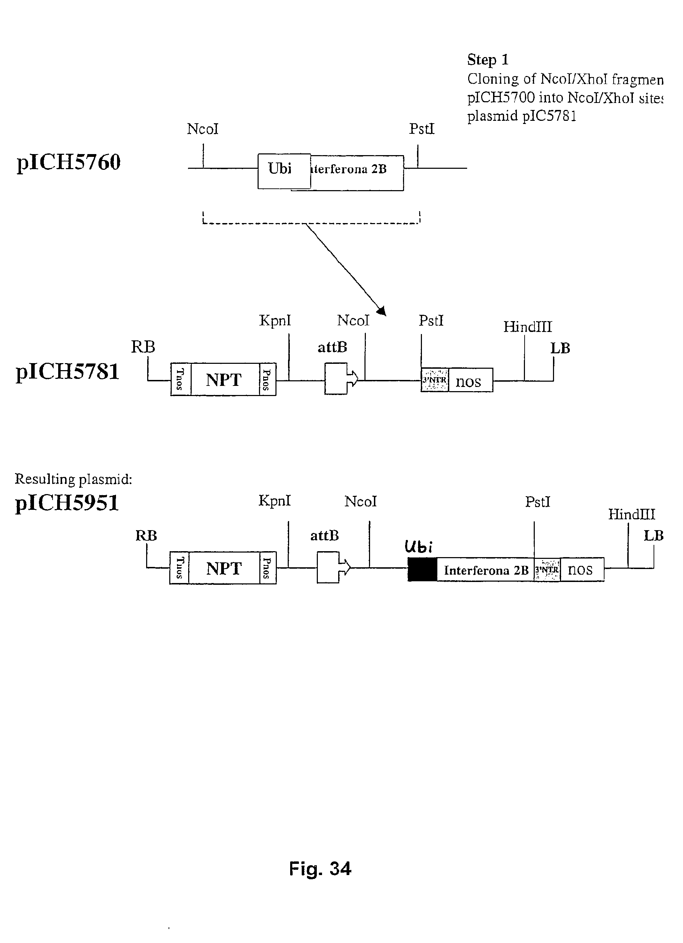

In order to clone a fusion of a ubiquitin-sequence and interferon α2B into an attB-3'-provector, both sequences were synthesized as a fusion and cloned into pUC19 (obtaining plasmid pUC5760). The vector was digested with Ncol and Pstl and the resulting fragment was ligated into the corresponding restriction-sites of plasmid pICH5781. The resulting plasmid plCH5951 contains an attB-recombination site, the ubiquitin-interferon fusion, an 3' nontranslated region, a nos promoter and a Kanamycin-expression-cassette. All said sequences are flanked by T-DNA borders (LB and RB, see Fig. 34).

D) In order to replace the Ω-sequence by different IRES-elements which can serve as translational enhancers, a ClaI/ApaI-fragment was taken from plasmid pIC1701 (that contains IRESₘₚ75(U1)) or pICH1731 (contains IRESₘₚ75(cr)), respectively. The fragments were cloned into the plasmid pICH5939 (similar to plasmid pICH5781, but without Ncol-restriction-site with ATG-sequence as transcription initiation-codon). In the final binary vectors (pICH6871, pICH6891), the attB-recombination-site is located adjacent to an IRES-sequence of 75 bp (6871: U1-origin, 6891: CrTMV-origin), a sGFP-reporter gene and the regulatory elements 3'NTR and nos.

### E) Cloning of vector type Polymerase-MP-LoxP into binary vectors

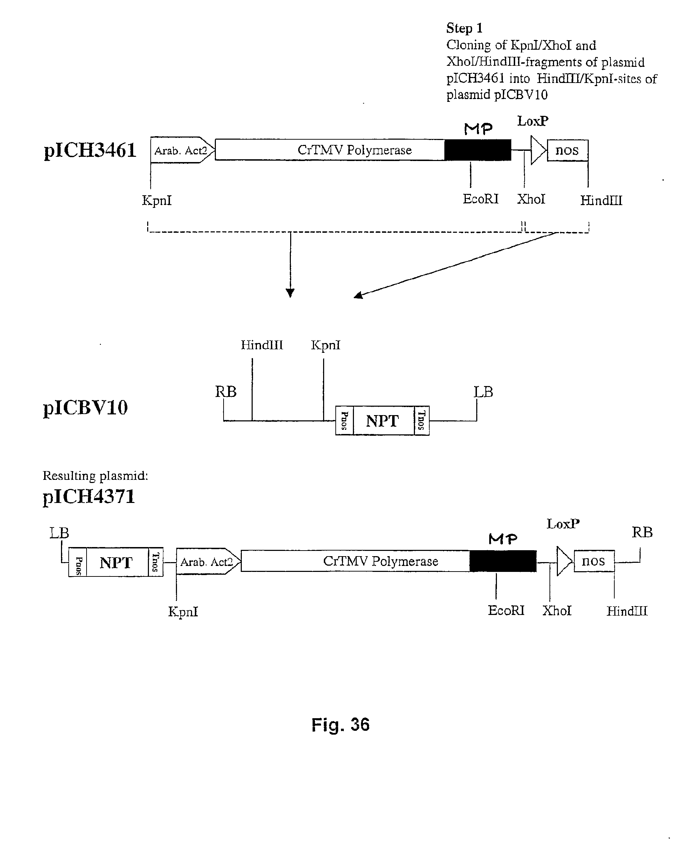

In order to clone the polymerase-MP carrying LoxP-provector into a binary vector, a Kpnl/Xhol and a Xhol/HindIII-fragment were ligated together into the vector pICBV10 which was digested with HindIII and Kpnl. The resulting plasmid plCH4371 can be stably transformed into *Agrobacterium* (Fig. 36).

### F) Cloning of vector type LoxP-gene of interest-3'NTR-nos-terminator into binary vector

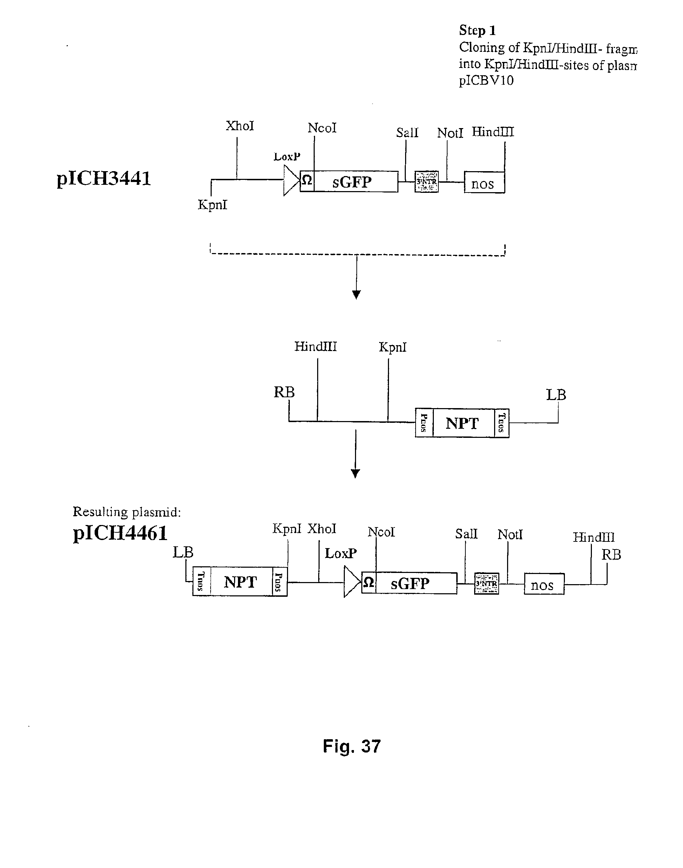

An analogous cloning strategy like in E) was used to clone the LoxP-reporter gene provector into a binary vector. Plasmid plCH3441 was digested with the enzymes Kpnl and HindIII. The resulting fragment was inserted in the corresponding sides of pICBV10 resulting in the binary plasmid plCH4461 (see Fig. 37).

### REFERENCE EXAMPLE 15

### Delivery of viral vector constructs by infiltration of Agrobacterium tumefaciens suspension into plant leaves of N. benthamiana and N. tabacum

All provectors of the Cre/Lox and the integrase/att-system have been cloned into binary vectors which can be transformed stably into *Agrobacteria.* Hence, DNA-delivery methods alternative to the described techniques are available.

A very simple delivery method is the infiltration of *Agrobacteria* suspensions into intact leaves. This so called agroinfiltration was initially developed to analyze foreign gene expression and gene silencing in plants (Kaplia *et al.,* 1997, Plant Science, 122, 101-108; Schöb *et al.,* 1997, Mol. Gen. Genet., 256, 581-588). Agroinfiltration of transgenic tobacco plants was performed according to a modified protocol described by Yang *et al.,* 2000, The Plant Journal, 22(6), 543-551. *Agrobacterium tumefaciens* strain GV3101 was transformed with individual constructs (pICH4851, pICH5151, pICP1010, Fig. 15) was grown in LB-medium supplemented with Rifampicin 50 mg/l, carbencilin 50 mg/l and 100 µM acetosyringone at 28 °C. *Agrobacterium* cells of an overnight culture (5 ml) were collected by centrifugation (10 min, 4500 g) and resuspended in 10 mM MES (pH 5.5) buffer supplemented with 10 mM MgSO₄ and 100 µM acetosyringone. The bacterial suspension was adjusted to a final OD₆₀₀ of 0.8. In case of delivery of several constructs, Agrobacteria suspensions of different constructs were mixed in equal volumes before infiltration.

Agroinfiltration was conducted on near fully expanded leaves that were still attached to the intact plant. A bacterial suspension was infiltrated using a 5 ml syringe. By infiltrating 100 µl of bacterial suspension into each spot (typically 3-4 cm⁻² in infiltrated area), 8 to 16 spots separated by veins could be arranged in a single tobacco leaf. After infiltration, plants were further grown under greenhouse conditions at 22 °C and 16 h light.

Leaves of *N. benthamiana* and *N. tabacum* that were infiltrated with the constructs show expanding sectors of strong GFP-expression 8-12 days after infiltration. The expression could be detected under UV-light directly on the infiltrated leaves. No GFP expression could be observed in case of controls (provector-combination without the integrase-clone).

### REFERENCE EXAMPLE 16

### Delivery of viral provectors by infiltration of an Agrobacterium tumefaciens suspension into plant leaves of transgenic tobacco plants expressing the Cre recombinase (plCH1754)

Leaves of transgenic tobacco plants (transformed construct: plCH1754, species *Nicotiana tabacum)* infiltrated with construct plCH4371 and plCH4461 showed 16 days after infiltration growing sectors of strong GFP-expression which could be observed without microscope under UV-light on intact plants. No GFP-expression was visible on wild type leaves infiltrated with the same Agrobacteria suspension mix.

### REFERENCE EXAMPLE 17

### The use of IRES sequences of viral origin (IRESₘₚ75 from CrTMV or U1) as translational enhancer sequences

The presence of an att- or LoxP- recombination-site between promoter and a downstream located gene limits the efficiency of translation. Therefore, in most cases, it is necessary to use translational enhancing elements to reach an appropriative level of reporter gene-expression in the provector-system. Cloning of the viral IRES sequences IRESₘₚ75(U1) or IRESₘₚ75(cr) respectively between the attB-recombination site and GFP clearly increases the expression of the reportergene. Whereas plants, which have been infiltrated with GFP-containing provectors that carry no translational enhancer show nearly no detectable GFP-expression after 10 days, the use of said IRES-sequences led to expression of GFP, which can be detected under UV-light without using a microscope after 5 days. The expression level of gene of interest provided by IRESₘₚ75-based translational enhancer activity is comparable or even higher than such provided by "omega" translational enhancer.

## Claims

1. A process of producing a protein or polypeptide of interest in a plant or in plant cells, comprising:
(i) transforming or transfecting a plant or plant cells with a viral vector, said viral vector containing a nucleotide sequence having a coding region encoding a fusion protein comprising the protein or polypeptide of interest, a signal peptide functional for targeting said fusion protein to the apoplast, and a polypeptide capable of binding the fusion protein to a cell wall component,
(ii) enriching cell wall components having expressed and bound fusion protein, and
(iii) separating the protein or polypeptide of interest or a protein comprising the protein or polypeptide of interest from said cell wall components.

2. The process of claim 1, wherein said plant is transfected for transient expression of said fusion protein.

3. The process of one of claims 1 to 2, whereby a plant is grown up to a desired growth state before carrying out step (i).

4. The process according to one of claims 1 to 3, whereby said enriching of cell wall components of step (ii) comprises mechanical, physical or chemical separation of cell contents.

5. The process according to one of claims 1 to 4, wherein the polypeptide capable of binding the fusion protein to a cell wall component binds to a plant cell wall polysaccharide.

6. The process according to claim 5, wherein the polypeptide capable of binding the fusion protein to a cell wall component binds to β-1,4-glycan.

7. The process according to claim 5, wherein the polypeptide capable of binding the fusion protein to a cell wall component binds to cellulose.

8. The process according to claim 5, wherein the polypeptide capable of binding the fusion protein to a cell wall component binds to hemicellulose.

9. The process according to claim 5, wherein the polypeptide capable of binding the fusion protein to a cell wall component binds to pectin.

10. The process of one of claims 1 to 9, wherein said fusion protein comprises a pectin methylesterase or a functional fragment thereof which functions as said signal peptide and as said polypeptide capable of binding said fusion protein to a cell wall component.

11. The process of one of claims 1 to 10, wherein said fusion protein comprises the cystein-rich domain of the tobacco NtTLPR protein or a functional fragment thereof capable of cross-linking said fusion protein to a cell wall component.

12. The process of one of claims 1 to 11 further comprising cleaving said fusion protein for obtaining said protein or polypeptide of interest free of said signal peptide and/or said polypeptide capable of binding said fusion protein to a cell wall component.

13. The process of claim 12, wherein said fusion protein comprises a cleavage sequence allowing cleavage of the fusion protein.

14. The process of claim 13, wherein the cleavage sequence is recognized by a site-specific protease.

15. The process of claim 14, wherein the cleavage sequence comprises the C-terminal portion of ubiquitin.

16. The process of one of claims 1 to 15, wherein said fusion protein contains said signal peptide at its N-terminal end followed by said polypeptide capable of binding the fusion protein to a cell wall component followed by said protein or polypeptide of interest.

17. The process of one of claims 1 to 15, wherein said fusion protein contains said signal peptide at its N-terminal end followed by said protein or polypeptide of interest which is followed by said polypeptide capable of binding the fusion protein to a cell wall component.

18. The process of one of claims 1 to 15, wherein said fusion protein contains said signal peptide at its N-terminal end and said protein or polypeptide of interest is flanked on both sides by a polypeptide capable of binding the fusion protein to a cell wall component.

19. The process of one of claims 1 to 18, wherein said nucleotide sequence for transforming or transfecting a plant or plant cells is DNA.

20. The process of one of claims 1 to 18, wherein said nucleotide sequence for transforming or transfecting a plant or plant cells is RNA.

21. The process according to one of claims 1 to 20, wherein said protein or polypeptide of interest contains one or more affinity peptide tags.

22. The process of claim 21, wherein said affinity peptide tag is selected from the group consisting of an oleosin protein or part thereof, an intein or part thereof, an additional cell wall binding domain, a starch binding domain, a streptavidin epitope-tag sequence, glutathione-S-transferase (GST) affinity tag, a 6xHis affinity tag, a calmodulin binding peptide (CBP) affinity tag.

23. The process of one of claims 1 to 22, whereby said nucleotide sequence is amplified and/or expressed in said plant by a process comprising: providing the plant or cells thereof with at least one precursor vector designed for undergoing processing in cells of said plant, whereby due to said processing said plant cell is endowed with at least one replicon which provides for said amplification and/or expression.

24. The process of claim 23, wherein the plant or cells thereof are provided with at least two precursor vectors.

25. The process of claim 23, wherein due to said processing said plant cell is endowed with at least two replicons which
(a) are structurally related to each other owing to said processing;
(b) are functionally distinct from each other; and
(c) provide for said amplification and/or expression.

## Patentansprüche

1. Verfahren zur Herstellung eines Proteins oder Polypeptids von Interesse in einer Pflanze oder in Pflanzenzellen, umfassend:
(i) Transformieren oder Transfektieren einer Pflanze oder von Pflanzenzellen mit einem Virusvektor, wobei der Virusvektor eine Nucleotid-Sequenz, die eine ein Fusionsprotein kodierende Region aufweist, das das Protein oder Polypeptid von Interesse umfasst, ein zum Einschleusen des Fusionsproteins in das Apoplast funktionelles Signalpeptid, und ein Polypeptid, das zum Binden des Fusionsproteins an eine Zellwand-Komponente fähig ist, enthält,
(ii) Anreichern von Zellwand-Komponenten, die exprimiertes und gebundenes Fusionsprotein aufweisen, und
(iii) Trennen des Proteins oder Polypeptids von Interesse oder eines Proteins, das das Protein oder Polypeptid von Interesse umfasst, von den Zellwand-Komponenten.

2. Verfahren nach Anspruch 1, worin die Pflanze für eine transiente Expression des Fusionsproteins transfektiert wird.

3. Verfahren nach einem der Ansprüche 1 bis 2, wobei eine Pflanze vor Ausführung der Stufe (i) bis zu einem gewünschten Wachstumsstadium wachsen gelassen wird.

4. Verfahren nach einem der Ansprüche 1 bis 3, wobei die Anreicherung der Zellwand-Komponenten der Stufe (ii) eine mechanische, physikalische oder chemische Trennung von Zellinhalten umfasst.

5. Verfahren nach einem der Ansprüche 1 bis 4, worin das zum Binden des Fusionsproteins an eine Zellwand-Komponente fähige Polypeptid an ein Pflanzenzellwand-Polysaccharid bindet.

6. Verfahren nach Anspruch 5, worin das zum Binden des Fusionsproteins an eine Zellwand-Komponente fähige Polypeptid an β-1,4-Glycan bindet.

7. Verfahren nach Anspruch 5, worin das zum Binden des Fusionsproteins an eine Zellwand-Komponente fähige Polypeptid an Cellulose bindet.

8. Verfahren nach Anspruch 5, worin das zum Binden des Fusionsproteins an eine Zellwand-Komponente fähige Polypeptid an Hemocellulose bindet.

9. Verfahren nach Anspruch 5, worin das zum Binden des Fusionsproteins an eine Zellwand-Komponente fähige Polypeptid an Pektin bindet.

10. Verfahren nach einem der Ansprüche 1 bis 9, worin das Fusionsprotein eine Pektin-Methylesterase oder ein funktionelles Fragment davon umfasst, das als Signalpeptid und als Polypeptid zum Binden des Fusionsproteins an eine Zellwand-Komponente fungiert.

11. Verfahren nach einem der Ansprüche 1 bis 10, worin das Fusionsprotein die Cysteinreiche Domäne von Tabak-NtTLPR-Protein oder einem funktionellen Fragment davon umfasst, das zum Vernetzen des Fusionsproteins an eine Zellwand-Komponente fähig ist.

12. Verfahren nach einem der Ansprüche 1 bis 11, das außerdem umfasst das Spalten des Fusionsproteins zum Erhalt des vom Signalpeptid und/oder dem zum Binden des Fusionsproteins an eine Zellwand-Komponente fähigen Polypeptid freien Proteins oder Polypeptids von Interesse.

13. Verfahren nach Anspruch 12, worin das Fusionsprotein eine Spaltsequenz umfasst, die die Spaltung des Fusionsproteins ermöglicht.

14. Verfahren nach Anspruch 13, worin die Spaltsequenz durch eine positionsspezifische Protease erkannt wird.

15. Verfahren nach Anspruch 14, worin die Spaltsequenz den C-terminalen Teil von Ubiquitin umfasst.

16. Verfahren nach einem der Ansprüche 1 bis 15, worin das Fusionsprotein das Signalpeptid an seinem N-Terminus enthält, gefolgt vom zum Binden des Fusionsproteins an eine Zellwand-Komponente fähigen Polypeptid, gefolgt von dem Protein oder Polypeptid von Interesse.

17. Verfahren nach einem der Ansprüche 1 bis 15, worin das Fusionsprotein das Signalpeptid an seinem N-Terminus enthält, gefolgt vom Protein oder Polypeptid von Interesse, an das das zum Binden des Fusionsproteins an eine Zellwand-Komponente fähige Polypeptid folgt.

18. Verfahren nach einem der Ansprüche 1 bis 15, worin das Fusionsprotein das Signalpeptid an seinem N-Terminus enthält, und das Protein oder Polypeptid von Interesse an beiden Seiten von einem Polypeptid flankiert wird, das zum Binden des Fusionsproteins an eine Zellwand-Komponente fähig ist.

19. Verfahren nach einem der Ansprüche 1 bis 18, worin die Nucleotid-Sequenz zum Transformieren oder Transfektieren einer Pflanze oder von Pflanzenzellen DNA ist.

20. Verfahren nach einem der Ansprüche 1 bis 18, worin die Nucleotid-Sequenz zum Transformieren oder Transfektieren einer Pflanze oder von Pflanzenzellen RNA ist.

21. Verfahren nach einem der Ansprüche 1 bis 20, worin das Protein oder Polypeptid von Interesse ein oder mehrere Affinitätspeptid-Tags enthält.

22. Verfahren nach Anspruch 21, worin das Affinitätspeptid-Tag ausgewählt ist aus der Gruppe bestehend aus einem Oleosinprotein oder einem Teil davon, einem Intein oder einem Teil davon, einer zusätzlichen Zellwand-Bindungsdomäne, einer Stärke-bindenden Domäne, einer Streptavidin-Epitop-Tag-Sequenz, Glutathion-S-Transferase(GST)-Affinitäts-Tag, einem 5xHis-Affinitäts-Tag, einem Calmodulin-Bindungspeptid(CBP)-Affinitäts-Tag.

23. Verfahren nach einem der Ansprüche 1 bis 22, wobei die Nucleotid-Sequenz in der Pflanze amplifiziert und/oder exprimiert wird durch ein Verfahren, umfassend: Versehen der Pflanze oder Zellen davon mit mindestens einem Precursor-Vektor, der ausgestaltet ist, um einem Processing in Zellen dieser Pflanze zu unterliegen, wobei aufgrund des Processing die Pflanzenzelle mit mindestens einem Replicon ausgestattet wird, das für die Amplifikation und/oder Expression sorgt.

24. Verfahren nach Anspruch 23, worin die Pflanze oder Zellen davon mit mindestens zwei Precursor-Vektoren versehen sind.

25. Verfahren nach Anspruch 23, worin aufgrund des Processing die Pflanzenzelle mit mindestens zwei Replicons ausgestattet wird, die
(a) aufgrund des Processing strukturell mit einander verwandt sind;
(b) von einander funktionell verschieden sind; und
(c) für die Amplifikation und/oder Expression sorgen.

## Revendications

1. Procédé pour produire une protéine ou un polypeptide à laquelle/auquel on s'intéresse dans une plante ou dans des cellules végétales, comprenant :
(i) la transformation ou la transfection d'une plante ou de cellules végétales avec un vecteur viral, ledit vecteur viral contenant une séquence de nucléotides ayant une région codante codant une protéine de fusion comprenant la protéine ou le polypeptide à laquelle/auquel on s'intéresse, un peptide signal fonctionnel pour cibler ladite protéine de fusion vers l'apoplaste, et un polypeptide capable de lier la protéine de fusion à un composant de paroi cellulaire,
(ii) l'enrichissement des composants de paroi cellulaire ayant une protéine de fusion exprimée et liée, et
(iii) la séparation de la protéine ou du polypeptide à laquelle/auquel on s'intéresse ou d'une protéine comprenant la protéine ou le polypeptide à laquelle/auquel on s'intéresse d'avec lesdits composants de paroi cellulaire.

2. Procédé selon la revendication 1, dans lequel ladite plante est transfectée pour l'expression transitoire de ladite protéine de fusion.

3. Procédé selon l'une des revendications 1 et 2, une plante étant amenée à croître jusqu'à un état de croissance souhaitée avant la mise en oeuvre de l'étape (i).

4. Procédé selon l'une des revendications 1 à 3, ledit enrichissement de composants de paroi cellulaire de l'étape (ii) comprenant une séparation mécanique, physique ou chimique des contenus cellulaires.

5. Procédé selon l'une quelconque des revendications 1 à 4, dans lequel le polypeptide capable de lier la protéine de fusion à un composant de paroi cellulaire se lie à un polysaccharide de paroi cellulaire végétale.

6. Procédé selon la revendication 5, dans lequel le polypeptide capable de lier la protéine de fusion à un composant de paroi cellulaire se lie au β-1,4-glycane.

7. Procédé selon la revendication 5, dans lequel le polypeptide capable de lier la protéine de fusion à un composant de paroi cellulaire se lie à la cellulose.

8. Procédé selon la revendication 5, dans lequel le polypeptide capable de lier la protéine de fusion à un composant de paroi cellulaire se lie à l'hémicellulose.

9. Procédé selon la revendication 5, dans lequel le polypeptide capable de lier la protéine de fusion à un composant de paroi cellulaire se lie à la pectine.

10. Procédé selon l'une des revendications 1 à 9, dans lequel ladite protéine de fusion comprend une pectine méthylestérase ou un fragment fonctionnel de celle-ci qui fonctionne comme ledit peptide signal et comme ledit polypeptide capable de lier ladite protéine de fusion à un composant de paroi cellulaire.

11. Procédé selon l'une des revendications 1 à 10, dans lequel ladite protéine de fusion comprend le domaine riche en cystéine de la protéine NtTLPR du tabac ou un fragment fonctionnel de celui-ci capable de lier par réticulation ladite protéine de fusion à un composant de paroi cellulaire.

12. Procédé selon l'une des revendications 1 à 11, comprenant en outre la coupure de ladite protéine de fusion pour obtenir ladite protéine ou ledit polypeptide à laquelle/auquel on s'intéresse exempt(e) dudit peptide signal et/ou dudit polypeptide capable de lier ladite protéine de fusion à un composant de paroi cellulaire.

13. Procédé selon la revendication 12, dans lequel ladite protéine de fusion comprend une séquence de coupure permettant la coupure de la protéine de fusion.

14. Procédé selon la revendication 13, dans lequel la séquence de coupure est reconnue par une protéase à spécificité de site.

15. Procédé selon la revendication 14, dans lequel la séquence de coupure comprend la partie C-terminale de l'ubiquitine.

16. Procédé selon l'une des revendications 1 à 15, dans lequel ladite protéine de fusion contient ledit peptide signal à son extrémité N-terminale, suivi dudit polypeptide capable de lier la protéine de fusion à un composant de paroi cellulaire, suivi de ladite protéine ou dudit polypeptide à laquelle/auquel on s'intéresse.

17. Procédé selon l'une des revendications 1 à 15, dans lequel ladite protéine de fusion contient ledit peptide signal à son extrémité N-terminale, suivi de ladite protéine ou dudit polypeptide à laquelle/auquel on s'intéresse qui est suivi dudit polypeptide capable de lier la protéine de fusion à un composant de paroi cellulaire.

18. Procédé selon l'une des revendications 1 à 15, dans lequel ladite protéine de fusion contient ledit peptide signal à son extrémité N-terminale et ladite protéine ou ledit polypeptide à laquelle/auquel on s'intéresse est flanqué, sur les deux côtés, par un polypeptide capable de lier la protéine de fusion à un composant de paroi cellulaire.

19. Procédé selon l'une des revendications 1 à 18, dans lequel ladite séquence de nucléotides pour transformer ou transfecter une plante ou des cellules végétales est un ADN.

20. Procédé selon l'une des revendications 1 à 18, dans lequel ladite séquence de nucléotides pour transformer ou transfecter une plante ou des cellules végétales est un ARN.

21. Procédé selon l'une des revendications 1 à 20, dans lequel ladite protéine ou ledit polypeptide à laquelle/auquel on s'intéresse contient une ou plusieurs étiquettes peptidiques d'affinité.

22. Procédé selon la revendication 21, dans lequel ladite étiquette peptidique d'affinité est choisie dans le groupe constitué par une protéine oléosine ou une partie de celle-ci, une intéine ou une partie de celle-ci, un domaine de liaison à la paroi cellulaire additionnel, un domaine de liaison à l'amidon, une séquence d'étiquette épitopique de streptavidine, une étiquette d'affinité pour la glutathion-S-transférase (GST), une étiquette d'affinité pour 6xHis, une étiquette d'affinité pour le peptide de liaison à la calmoduline (CBP).

23. Procédé selon l'une des revendications 1 à 22, ladite séquence de nucléotides étant amplifiée et/ou exprimée dans ladite plante par un procédé comprenant : l'opération consistant à pourvoir la plante ou des cellules de celle-ci d'au moins un vecteur précurseur conçu pour subir une maturation dans des cellules de ladite plante, grâce à quoi, du fait de ladite maturation, ladite cellule végétale est dotée d'au moins un réplicon qui permet ladite amplification et/ou expression.

24. Procédé selon la revendication 23, dans lequel la plante ou les cellules de celle-ci sont pourvues d'au moins deux vecteurs précurseurs.

25. Procédé selon la revendication 23, dans lequel, du fait de ladite maturation, ladite cellule végétale est dotée d'au moins deux réplicons qui
(a) sont structurellement apparentés l'un à l'autre du fait de ladite maturation ;
(b) sont fonctionnellement distincts l'un de l'autre ; et
(c) permettent ladite amplification et/ou expression.
